# EUROPEAN PATENT SPECIFICATION

(11) **EP 3 762 079 B1**
(45) Date of publication and mention of the grant of the patent: **28.05.2025**
(21) Application number: 19764205.1
(22) Date of filing: 07.03.2019
(51) Int. Cl.: A61M 16/06, A61M 16/08

(54) **PATIENT INTERFACE**
PATIENTENSCHNITTSTELLE
INTERFACE PATIENT

(30) Priority: 07.03.2018 US 201862639908 P; 01.06.2018 US 201862679675 P; 04.06.2018 US 201862680503 P; 16.11.2018 US 201862768765 P
(43) Date of publication of application: 13.01.2021
(73) Proprietor: Fisher & Paykel Healthcare Limited, East Tamaki Auckland 2013 (NZ)
(72) Inventor: LIM, Jae Yun, Auckland (NZ); PATEL, Roheet, Auckland (NZ); PRENTICE, Craig Robert, Auckland (NZ); RICHARDSON, Thomas Mark, Auckland (NZ); MITTERMEIER, Simon, Auckland (NZ); GANTERER, Monika, Auckland (NZ); ROSE, Hamish Joshua, Auckland (NZ); BETTERIDGE, Max Leon, Auckland (NZ); YOUNG, Jeremy Owen, Auckland (NZ); TRINIDADE, Nigel, Auckland (NZ); TOMLINSON, Mark Richard, Auckland (NZ); KAPELEVICH, Vitaly, Auckland (NZ); MARTIN, Campbell Neil Addison, Auckland (NZ); SPEAR, Tony William, Auckland (NZ); PEDERSEN, Matthew James, Auckland (NZ)
(74) Representative: Treeby, Philip David William
(86) International application number: PCT/NZ2019/050024
(87) International publication number: WO 2019/172783

(56) References cited:
- WO-A1-2016/032343
- WO-A1-2017/049356
- WO-A2-2015/057087
- US-A1- 2017 143 925
- US-A1- 2017 361 048
- US-B2- 8 596 273

## Description

### BACKGROUND

### Field

The present disclosure relates to respiratory therapy systems. In particular, the disclosure relates to interface assemblies for use in respiratory therapy and portions thereof.

### Description of Related Art

Respiratory masks are used to provide respiratory therapy to the airways of a person suffering from any of a number of respiratory illnesses or conditions. Such therapies may include but are not limited to continuous positive airway pressure (CPAP) therapy and non-invasive ventilation (NIV) therapy.

CPAP therapy can be used to treat obstructive sleep apnea (OSA), a condition in which a patient's airway intermittently collapses, during sleep, preventing the patient from breathing for a period of time. The cessation of breathing, or apnea, results in the patient awakening. Repetitive and frequent apneas may result in the patient rarely achieving a full and restorative night's sleep.

CPAP therapy involves the delivery of a supply of continuous positive air pressure to the airway of the patient via a respiratory mask. The continuous positive pressure acts as a splint within the patient's airway, which secures the airway in an open position such that the patient's breathing and sleep are not interrupted.

Respiratory masks typically comprise a patient interface and a headgear, wherein the patient interface is configured to deliver the supply of continuous positive air pressure to the patient's airway via a seal or cushion that forms an airtight seal in or around the patient's nose and/or mouth. Respiratory masks are available in a range of styles including full-face, nasal, direct nasal and oral masks, which create an airtight seal with the nose and/or mouth. The seal or cushion is held in place on the patient's face by the headgear. In order to maintain an airtight seal the headgear should provide support to the patient interface such that it is held in a stable position relative to the patient's face during use. Such respiratory masks may also be used to deliver NIV and other therapies.

WO 2015/057087 discloses headgear for a respiratory interface comprising multiple separate sections of material joined to form the headgear and different levels of resilient extensibility or stretchability in at least four different parts or straps of the headgear.

US 8,596,273 B2, US 2017/361048 A1 and US 2017/143925 A1 are also acknowledged.

### SUMMARY

The invention is defined in the appended claims. The systems, methods and devices described herein have innovative aspects, no single one of which is indispensable or solely responsible for their desirable attributes. Without limiting the scope of the claims, some of the advantageous features will now be summarized.

According to the present invention there is provided a respiratory mask comprising a cushion module according to claim 1. Preferred features are set out in the dependent claims.

In some configurations, a respiratory mask includes a frame configured to connect to headgear. The frame comprises a front wall having a vent and a gas inlet opening. The frame further comprises a collar extending away from the front wall. The collar surrounds the vent and the gas inlet opening. A cushion module comprises a housing and a cushion. The housing is made of a material more rigid than the cushion. The housing forms at least a portion of a breathing chamber of the respiratory mask. The housing defines a connection opening. A friction coupling is configured to selectively couple the connection opening of the cushion module to the collar of the frame such that a flow of gas can be delivered to the breathing chamber through the gas inlet opening of the frame and the connection opening of the cushion module. The friction coupling comprises an elastomeric friction member coupled to a portion of the housing that defines the connection opening.

In some configurations, the mask further comprises a divider wall within an interior of the collar. The divider wall having a first end and a second end, each connected to the collar. The divider wall separates the vent and the gas inlet opening.

In some configurations, a surface of the divider wall adjacent the vent extends substantially in a direction of gas flow through the vent.

In some configurations, the collar and the divider wall cooperate to define a socket configured to receive an elbow.

In some configurations, the mask further comprises an elbow received by the socket, wherein the elbow has a swivel connection with the frame.

In some configurations, the collar extends rearwardly from the front wall of the frame to a rearward edge. A rearward edge of the divider wall is positioned between the front wall of the frame and the rearward edge of the collar.

In some configurations, the frame forms at least a portion of the breathing chamber.

In some configurations, a space defined by an interior surface of the collar, an upper surface of the divider wall and an interior surface of a vent wall portion of the front wall forms at least a portion of the breathing chamber.

In some configurations, the collar comprises an internal surface and a separate engagement surface, such as an external engagement surface, wherein the engagement surface engages with the friction member.

In some configurations, the collar further comprises a rim.

In some configurations, the friction member comprises an internal engagement surface and a peripheral surface.

In some configurations, the connection opening is defined by a support wall, wherein the support wall is disposed, at least in part, within the friction member and the housing extends through the peripheral surface of the friction member.

In some configurations, the cushion module further comprises a support flange that extends along at least a part of a length of and supports the friction member.

In some configurations, the support flange is embedded within the friction member.

In some configurations, a portion of the housing disposed, at least in part, within the friction member comprises a plurality of apertures.

In some configurations, the friction member extends through each of the plurality of apertures.

In some configurations, at least a portion of the apertures are located between the support wall and the peripheral surface.

In some configurations, at least a portion of the apertures are located on the support flange.

In some configurations, a plurality of apertures are located at a junction between the support flange and an adjacent portion of the housing, and the elastomeric friction member extends through each of the apertures.

In some configurations, the friction member defines an abutment surface that contacts the front wall of the frame when the cushion module is coupled to the frame.

In some configurations, the friction member defines a first length, the support flange defines a second length, an unsupported portion of the friction member that extends beyond the support flange defines a third length, and the collar defines a fourth length.

In some configurations, the first length is equal to the sum of the second length and the third length.

In some configurations, the first length is greater than the sum of the second length and the third length.

In some configurations, the first length is less than the sum of the second length and the third length.

In some configurations, the friction member defines a first thickness, the support flange defines a second thickness, a portion of the friction member located outside of the support flange defines a third thickness, a portion of the friction member located inside of the support flange defines a fourth thickness, and the collar defines a fifth thickness.

In some configurations, the first thickness equals the sum of the second thickness, the third thickness, and the fourth thickness.

In some configurations, the third thickness and the fourth thickness are equal.

In some configurations, the second thickness is equal to each of the third thickness and the fourth thickness.

In some configurations, the fourth thickness is greater than or equal to the third thickness.

In some configurations, the fourth thickness is less than or equal to the third thickness.

In some configurations, the second thickness and the fifth thickness are equal.

In some configurations, the second thickness is greater than the fifth thickness.

In some configurations, the second thickness is less than the fifth thickness.

In some configurations, the second thickness is less than or equal to either one of the third thickness and the fourth thickness.

In some configurations, the second thickness is greater than or equal to either one of the third thickness and the fourth thickness.

In some configurations, the first thickness is greater than or equal to the fifth thickness.

In some configurations, the first thickness is less than or equal to the fifth thickness.

In some configurations, the fourth thickness varies along at least a part of the length of the friction member.

In some configurations, fourth thickness varies along at least a part of the length of the friction member from a minimum at or near a proximal end of the friction member to a maximum at or near a distal end of the friction member.

In some configurations, the fourth thickness varies along at least a part of the length of the friction member from a maximum at or near a proximal end of the friction member to a minimum at or near a distal end of the friction member.

In some configurations, the third thickness is zero.

In some configurations, a portion of the friction member that overlaps with the collar defines an overlap length.

In some configurations, the overlap length is greater than or equal to the third length.

In some configurations, the overlap length is greater than or equal to a sum of the second length and the third length.

In some configurations, the overlap length is less than the third length.

In some configurations, the overlap length is less than a sum of the second length and the third length.

In some configurations, the overlap length equals the fourth length.

In some configurations, the overlap length is less than the fourth length.

In some configurations, the overlap length is greater than the fourth length.

In some configurations, a portion of the friction member that does not overlap with the collar defines a non-overlap length and a portion of the front wall adjacent the support flange defines a first front wall thickness.

In some configurations, a sum of the first length and the first front wall thickness equals a sum of the overlap length and the non-overlap length.

In some configurations, a sum of the first length and the first front wall thickness is greater than a sum of the overlap length and the non-overlap length.

In some configurations, a sum of the first length and the first front wall thickness is less than a sum of the overlap length and the non-overlap length.

In some configurations, the support flange comprises a plurality of apertures and the friction member extends through each of the plurality of apertures. A distance between the portion of the front wall and a point on the plurality of apertures furthest from the portion of the front wall defines an aperture offset distance.

In some configurations, the non-overlap length is greater than or equal to a sum of the aperture offset distance and the first front wall thickness.

In some configurations, the non-overlap length is less than or equal to a sum of the aperture offset distance and the first front wall thickness.

In some configurations, a sum of the second length and the third length is greater than or equal to a sum of the overlap length and the aperture offset distance.

In some configurations, a sum of the second length and the third length is less than or equal to a sum of the overlap length and the aperture offset distance.

In some configurations, a sum of the overlap length and the aperture offset distance is less than or equal to first length.

In some configurations, a sum of the overlap length and the aperture offset distance is greater than or equal to first length.

In some configurations, a relative angle is defined between the collar and at least one of the support flange and a surface of the friction member that engages the collar in a direction of assembly of the cushion module to the frame.

In some configurations, the collar is angled relative to the direction of assembly.

In some configurations, at least one of the support flange and the surface of the friction member that engages the collar is angled relative to the direction of assembly.

In some configurations, a relative angle is defined between the collar and a surface of the friction member that engages the collar in a direction of assembly of the cushion module to the frame.

In some configurations, the collar is angled relative to the direction of assembly.

In some configurations, the surface of the friction member that engages the collar is angled relative to the direction of assembly.

In some configurations, the friction coupling is configured such that a force acting to compress the friction member is equal along a length of the collar.

In some configurations, the friction coupling is configured such that a force acting to compress the friction member is unequal along a length of the collar.

In some configurations, the force is larger at a location closer to the front wall relative to a location further from the front wall.

In some configurations, the force is smaller at a location closer to the front wall relative to a location further from the front wall.

In some configurations, a rearward-most extent of the friction member is at or forward of the front wall.

In some configurations, a rearward-most extent of the friction member is rearward of the front wall such that a portion of the friction member is located adjacent each of a forward surface and a rearward surface of the front wall.

In some configurations, the portion of the friction member adjacent the forward surface of the front wall has a surface that is aligned with a surface of the portion of the friction member adjacent the rearward surface of the front wall.

In some configurations, the connection opening is defined by a support wall. The support wall is disposed, at least in part, within the friction member such that the friction member is coupled to the cushion module.

In some configurations, the collar defines a collar length, the support wall defines a support wall thickness, and the friction member defines a total friction member length, a forward length forward of the support wall and a rearward length rearward of the support wall.

In some configurations, the total friction member length is equal to a sum of the forward length, the rearward length and the support wall thickness.

In some configurations, the total friction member length is greater than a sum of the forward length, the rearward length and the support wall thickness.

In some configurations, the total friction member length is less than a sum of the forward length, the rearward length and the support wall thickness.

In some configurations, the collar defines a collar thickness and the friction member defines a total thickness, a supported thickness along the support wall and an unsupported thickness inwardly of the support wall.

In some configurations, the total thickness equals a sum of the supported thickness and the unsupported thickness.

In some configurations, the total thickness equals twice the supported thickness.

In some configurations, the total thickness equals twice the unsupported thickness.

In some configurations, the supported thickness is equal to the unsupported thickness.

In some configurations, the supported thickness is greater than or equal to the unsupported thickness.

In some configurations, the supported thickness is less than or equal to the unsupported thickness.

In some configurations, the total thickness is greater than or equal to the collar thickness.

In some configurations, the total thickness is less than or equal to the collar thickness.

In some configurations, a portion of the friction member that overlaps with the collar defines an overlap length, a portion of the friction member that does not overlap with the collar defines a non-overlap length and a distance between a rearward end of the collar and the support wall along the direction of assembly defines a support wall offset distance.

In some configurations, the overlap length is greater than or equal to the support wall offset distance.

In some configurations, the overlap length is less than the support wall offset distance.

In some configurations, the overlap length is greater than or equal to the non-overlap length.

In some configurations, the overlap length is less than the non-overlap length.

In some configurations, the non-overlap length is greater than or equal to a sum of the support wall offset distance and the support wall thickness.

In some configurations, the non-overlap length is less than or equal to a sum of the support wall offset distance and the support wall thickness.

In some configurations, the overlap length equals the collar length.

In some configurations, the overlap length is less than the collar length.

In some configurations, the overlap length is greater than the collar length.

In some configurations, the total friction member length equals a sum of the overlap length and the non-overlap length.

In some configurations, the overlap length is less than the forward length.

In some configurations, the overlap length is greater than or equal to the forward length.

In some configurations, the overlap length is greater than or equal to a sum of the forward length, the rearward length and the support wall thickness.

In some configurations, the overlap length is less than or equal to a sum of the forward length, the rearward length and the support wall thickness.

In some configurations, the support wall thickness is less than or equal to the rearward length.

In some configurations, the support wall thickness is greater than the rearward length.

In some configurations, the housing comprises a wall portion external of the friction member and extending between the friction member and the cushion.

In some configurations, at least a surface of the friction coupling that engages the collar has a frosted or textured surface finish.

In some configurations, a respiratory mask comprises a frame configured to connect to headgear. The frame comprises a front wall at least partially defining a gas inlet opening. The frame further comprises a collar extending away from the front wall. The collar at least partially surrounds the gas inlet opening. A cushion module comprises a housing, a cushion and an elastomeric friction member. The cushion defines a face-contacting surface. The housing defines a connection opening. A portion of the housing defining the connection opening is embedded within the elastomeric friction member to couple the friction member to the housing. The housing is made of a material more rigid than the cushion and forms at least a portion of a breathing chamber of the respiratory mask. The friction member is configured to selectively connect the cushion module to the collar of the frame with a friction fit so that the flow of gas can be delivered to the breathing chamber through the gas inlet opening of the frame and the connection opening of the cushion module. The friction member engages an outer surface of the collar. An outer surface of the friction member is exposed when the cushion module is coupled to the frame.

In some configurations, the housing comprises a wall portion external of the friction member and extending between the friction member and the cushion.

In some configurations, the front wall of the frame further comprises a vent, and wherein the collar surrounds the vent in addition to the gas inlet.

In some configurations, a divider wall is located within an interior of the collar. The divider wall has a first end and a second end, each connected to the collar. The divider wall separates the vent and the gas inlet opening.

In some configurations, a surface of the divider wall adjacent the vent extends substantially in a direction of gas flow through the vent.

In some configurations, an elbow is supported by the frame and configured to deliver a flow of breathing gas through the gas inlet opening.

In some configurations, the collar and the divider wall cooperate to define a socket that receives the elbow.

In some configurations, the elbow has a swivel connection with the frame.

In some configurations, the collar extends rearwardly from the front wall of the frame to a rearward edge, wherein a rearward edge of the divider wall is positioned between the front wall of the frame and the rearward edge of the collar.

In some configurations, the housing of the cushion module further comprises a support flange, wherein the support flange at least partially defines the connection opening and extends within the friction member substantially in a direction of assembly of the cushion module to the frame.

In some configurations, the support flange comprises a plurality of apertures and wherein the friction member extends through each of the plurality of apertures.

In some configurations, a portion of the wall portion embedded within the elastomeric friction member comprises a plurality of apertures and wherein the elastomeric friction member extends through each of the apertures.

In some configurations, a portion of the wall portion embedded within the elastomeric friction member and the support flange each comprise a plurality of apertures and wherein the elastomeric friction member passes through each of the apertures.

In some configurations, a plurality of apertures are located at a junction between the support flange and a portion of the wall portion embedded within the elastomeric friction member, and the elastomeric friction member extends through each of the apertures.

In some configurations, the friction member defines an abutment surface that contacts the front wall of the frame when the cushion module is coupled to the frame.

In some configurations, the friction member defines a first length, the support flange defines a second length, an unsupported portion of the friction member that extends beyond the support flange defines a third length, and the collar defines a fourth length.

In some configurations, the first length is equal to the sum of the second length and the third length.

In some configurations, the first length is greater than the sum of the second length and the third length.

In some configurations, the first length is less than the sum of the second length and the third length.

In some configurations, the friction member defines a first thickness, the support flange defines a second thickness, a portion of the friction member located outside of the support flange defines a third thickness, a portion of the friction member located inside of the support flange defines a fourth thickness, and the collar defines a fifth thickness.

In some configurations, the first thickness equals the sum of the second thickness, the third thickness, and the fourth thickness.

In some configurations, the third thickness and the fourth thickness are equal.

In some configurations, the second thickness is equal to each of the third thickness and the fourth thickness.

In some configurations, the fourth thickness is greater than or equal to the third thickness.

In some configurations, the fourth thickness is less than or equal to the third thickness.

In some configurations, the second thickness and the fifth thickness are equal.

In some configurations, the second thickness is greater than the fifth thickness.

In some configurations, the second thickness is less than the fifth thickness.

In some configurations, the second thickness is less than or equal to either one of the third thickness and the fourth thickness.

In some configurations, the second thickness is greater than or equal to either one of the third thickness and the fourth thickness.

In some configurations, the first thickness is greater than or equal to the fifth thickness.

In some configurations, the first thickness is less than or equal to the fifth thickness.

In some configurations, the fourth thickness varies along at least a part of the length of the friction member.

In some configurations, the fourth thickness varies along at least a part of the length of the friction member from a minimum at or near a proximal end of the friction member to a maximum at or near a distal end of the friction member.

In some configurations, the fourth thickness varies along at least a part of the length of the friction member from a maximum at or near a proximal end of the friction member to a minimum at or near a distal end of the friction member.

In some configurations, the third thickness is zero.

In some configurations, a portion of the friction member that overlaps with the collar defines an overlap length.

In some configurations, the overlap length is greater than or equal to the third length.

In some configurations, the overlap length is greater than or equal to a sum of the second length and the third length.

In some configurations, the overlap length is less than the third length.

In some configurations, the overlap length is less than a sum of the second length and the third length.

In some configurations, the overlap length equals the fourth length.

In some configurations, the overlap length is less than the fourth length.

In some configurations, the overlap length is greater than the fourth length.

In some configurations, a portion of the friction member that does not overlap with the collar defines a non-overlap length and wherein a portion of the front wall adjacent the support flange defines a first front wall thickness.

In some configurations, a sum of the first length and the first front wall thickness equals a sum of the overlap length and the non-overlap length.

In some configurations, a sum of the first length and the first front wall thickness is greater than a sum of the overlap length and the non-overlap length.

In some configurations, a sum of the first length and the first front wall thickness is less than a sum of the overlap length and the non-overlap length.

In some configurations, the support flange comprises a plurality of apertures and the friction member extends through each of the plurality of apertures. A distance between the portion of the front wall and a point on the plurality of apertures furthest from the portion of the front wall defines an aperture offset distance.

In some configurations, the non-overlap length is greater than or equal to a sum of the aperture offset distance and the first front wall thickness.

In some configurations, the non-overlap length is less than or equal to a sum of the aperture offset distance and the first front wall thickness.

In some configurations, a sum of the second length and the third length is greater than or equal to a sum of the overlap length and the aperture offset distance.

In some configurations, a sum of the second length and the third length is less than or equal to a sum of the overlap length and the aperture offset distance.

In some configurations, a sum of the overlap length and the aperture offset distance is less than or equal to first length.

In some configurations, a sum of the overlap length and the aperture offset distance is greater than or equal to first length.

In some configurations, a relative angle is defined between the collar and at least one of the support flange and a surface of the friction member that engages the collar in a direction of assembly of the cushion module to the frame.

In some configurations, the collar is angled relative to the direction of assembly.

In some configurations, at least one of the support flange and the surface of the friction member that engages the collar is angled relative to the direction of assembly.

In some configurations, a relative angle is defined between the collar and a surface of the friction member that engages the collar in a direction of assembly of the cushion module to the frame.

In some configurations, the collar is angled relative to the direction of assembly.

In some configurations, the surface of the friction member that engages the collar is angled relative to the direction of assembly.

In some configurations, the friction coupling is configured such that a force acting to compress the friction member is equal along a length of the collar.

In some configurations, the friction coupling is configured such that a force acting to compress the friction member is unequal along a length of the collar.

In some configurations, the force is larger at a location closer to the front wall relative to a location further from the front wall.

In some configurations, the force is smaller at a location closer to the front wall relative to a location further from the front wall.

In some configurations, a rearward-most extent of the friction member is at or forward of the front wall.

In some configurations, a rearward-most extent of the friction member is rearward of the front wall such that a portion of the friction member is located adjacent each of a forward surface and a rearward surface of the front wall.

In some configurations, the portion of the friction member adjacent the forward surface of the front wall has a surface that is aligned with a surface of the portion of the friction member adjacent the rearward surface of the front wall.

In some configurations, the connection opening is defined by a support wall. The support wall is disposed, at least in part, within the friction member such that the friction member is coupled to the cushion module. The support wall extends in a direction substantially perpendicular to a direction of assembly of the cushion module to the frame.

In some configurations, the collar defines a collar length, the support wall defines a support wall thickness, and the friction member defines a total friction member length, a forward length forward of the support wall and a rearward length rearward of the support wall.

In some configurations, the total friction member length is equal to a sum of the forward length, the rearward length and the support wall thickness.

In some configurations, the total friction member length is greater than a sum of the forward length, the rearward length and the support wall thickness.

In some configurations, the total friction member length is less than a sum of the forward length, the rearward length and the support wall thickness.

In some configurations, the collar defines a collar thickness and the friction member defines a total thickness, a supported thickness along the support wall and an unsupported thickness inwardly of the support wall.

In some configurations, the total thickness equals a sum of the supported thickness and the unsupported thickness.

In some configurations, the total thickness equals twice the supported thickness.

In some configurations, the total thickness equals twice the unsupported thickness.

In some configurations, the supported thickness is equal to the unsupported thickness.

In some configurations, the supported thickness is greater than or equal to the unsupported thickness.

In some configurations, the supported thickness is less than or equal to the unsupported thickness.

In some configurations, the total thickness is greater than or equal to the collar thickness.

In some configurations, the total thickness is less than or equal to the collar thickness.

In some configurations, a portion of the friction member that overlaps with the collar defines an overlap length, a portion of the friction member that does not overlap with the collar defines a non-overlap length and a distance between a rearward end of the collar and the support wall along the direction of assembly defines a support wall offset distance.

In some configurations, the overlap length is greater than or equal to the support wall offset distance.

In some configurations, the overlap length is less than the support wall offset distance.

In some configurations, the overlap length is greater than or equal to the non-overlap length.

In some configurations, the overlap length is less than the non-overlap length.

In some configurations, the non-overlap length is greater than or equal to a sum of the support wall offset distance and the support wall thickness.

In some configurations, the non-overlap length is less than or equal to a sum of the support wall offset distance and the support wall thickness.

In some configurations, the overlap length equals the collar length.

In some configurations, the overlap length is less than the collar length.

In some configurations, the overlap length is greater than the collar length.

In some configurations, the total friction member length equals a sum of the overlap length and the non-overlap length.

In some configurations, the overlap length is less than the forward length.

In some configurations, the overlap length is greater than or equal to the forward length.

In some configurations, the overlap length is greater than or equal to a sum of the forward length, the rearward length and the support wall thickness.

In some configurations, the overlap length is less than or equal to a sum of the forward length, the rearward length and the support wall thickness.

In some configurations, the support wall thickness is less than or equal to the rearward length.

In some configurations, the support wall thickness is greater than the rearward length.

In some configurations, at least a surface of the friction coupling that engages the collar has a frosted or textured surface finish.

In some configurations, the frame further comprises a conduit connector that is unitarily formed with one or both of the front wall and the collar.

In some configurations, the housing of the cushion module defines an inlet recess positioned below the connection opening, and wherein the inlet recess is configured to accommodate a portion of the conduit connector.

In some configurations, the inlet recess is configured to accommodate a rearward-facing portion of the conduit connector.

In some configurations, the housing of the cushion module comprises a recess that surrounds the connection opening.

In some configurations, the embedded portion is displaced rearward from a main wall of the housing along an axis of the connection opening by a connecting portion.

In some configurations, the housing of the cushion module comprises a recess that extends around only a portion of the connection opening.

In some configurations, the housing of the cushion module comprises a recess that extends around only an upper portion of the connection opening.

In some configurations, the housing of the cushion module comprises a recess that extends around only a lower portion of the connection opening.

In some configurations, at least a portion of the friction member is located within the recess. In some configurations, the portion of the friction member defines a portion of the recess.

In some configurations, at least a portion of the friction member protrudes in front of the recess.

In some configurations, the recess defines a depth that is less than a total length of the friction member or a length of the friction member forward of the embedded portion of the housing.

In some configurations, the recess defines a depth that is greater than or equal to a total length of the friction member or a length of the friction member forward of the embedded portion of the housing.

In some configurations, the housing further comprises a support wall that supports the friction member and defines a portion or an entirety of the embedded portion, and the support wall is connected to the front wall by a connecting portion.

In some configurations, the housing further comprises a support wall that supports the friction member and defines a portion or an entirety of the embedded portion, and a radially-inward end of the support wall is connected to a support flange having a support flange length that is greater than a thickness of the support wall.

In some configurations, the support flange defines a longitudinal axis that is offset from an axis defined by a surface of the friction member that engages the collar.

In some configurations, an opening of the friction member defines a non-circular shape.

In some configurations, the opening of the friction member comprises a first axis and a second axis, the first axis extends in a lateral direction across a widest part of the opening, and the second axis extends in a vertical direction.

In some configurations, the friction member is symmetric about the second axis.

In some configurations, the friction member is asymmetric about the first axis.

In some configurations, the first axis is a major axis and the second axis is a minor axis. In some such configurations, the seal is configured to seal below the bridge of the nose of the user, such as on or below a tip of the nose of the user.

In some configurations, the first axis is a minor axis and the second axis is a major axis. In some such configurations, the seal is configured to seal on the bridge of the nose of the user.

In some configurations, a ratio of the first axis to the second axis is between about 1.2-1.4:1, is about 1.3:1, or is about 1.27:1.

In some configurations, a respiratory mask comprises a frame configured to connect to headgear. The frame comprises a front wall at least partially defining a gas inlet opening. The frame further comprises a collar extending away from the front wall. The collar at least partially surrounds the gas inlet opening. A cushion module comprises a housing, a cushion and an elastomeric friction member. The cushion defines a face-contacting surface. The housing defines a connection opening. The housing is made of a material more rigid than the cushion and forms at least a portion of a breathing chamber of the respiratory mask. The elastomeric friction member is configured to selectively connect the cushion module to the collar of the frame with a friction fit so that the flow of gas can be delivered to the breathing chamber through the gas inlet opening of the frame and the connection opening of the cushion module. The elastomeric friction member comprises an internal engagement surface and a peripheral surface. The internal engagement surface is configured to engage an outer surface of the collar when the cushion module is coupled to the frame. An embedded portion of the housing is disposed within the elastomeric friction member and the housing extends through the peripheral surface.

In some configurations, the embedded portion comprises a forward or rearward-extending flange.

In some configurations, the housing comprises a wall portion external of the friction member and extending between the friction member and the cushion.

In some configurations, the front wall of the frame further comprises a vent, and wherein the collar surrounds the vent in addition to the gas inlet.

In some configurations, the respiratory mask further comprises a divider wall within an interior of the collar. The divider wall has a first end and a second end, each connected to the collar. The divider wall separates the vent and the gas inlet opening.

In some configurations, a surface of the divider wall adjacent the vent extends substantially in a direction of gas flow through the vent.

In some configurations, the collar and the divider wall cooperate to define a socket configured to receive an elbow.

In some configurations, the respiratory mask further comprises an elbow received in the socket. The elbow has a swivel connection with the frame.

In some configurations, the collar extends rearwardly from the front wall of the frame to a rearward edge, wherein a rearward edge of the divider wall is positioned between the front wall of the frame and the rearward edge of the collar.

In some configurations, the housing of the cushion module further comprises a support flange, wherein the support flange at least partially defines the connection opening and extends within the friction member substantially in a direction of assembly of the cushion module to the frame.

In some configurations, the support flange comprises a plurality of apertures and wherein the friction member extends through each of the plurality of apertures.

In some configurations, a portion of the wall portion embedded within the elastomeric friction member comprises a plurality of apertures and wherein the elastomeric friction member extends through each of the apertures.

In some configurations, a portion of the wall portion embedded within the elastomeric friction member and the support flange each comprise a plurality of apertures and wherein the elastomeric friction member passes through each of the apertures.

In some configurations, a plurality of apertures are located at a junction between the support flange and a portion of the wall portion embedded within the elastomeric friction member, and the elastomeric friction member extends through each of the apertures.

In some configurations, the friction member defines an abutment surface that contacts the front wall of the frame when the cushion module is coupled to the frame.

In some configurations, the friction member defines a first length, the support flange defines a second length, an unsupported portion of the friction member that extends beyond the support flange defines a third length, and the collar defines a fourth length.

In some configurations, the first thickness equals the sum of the second thickness, the third thickness, and the fourth thickness.

In some configurations, the third thickness and the fourth thickness are equal.

In some configurations, the second thickness is equal to each of the third thickness and the fourth thickness.

In some configurations, the fourth thickness is greater than or equal to the third thickness.

In some configurations, the fourth thickness is less than or equal to the third thickness.

In some configurations, the second thickness and the fifth thickness are equal.

In some configurations, the second thickness is greater than the fifth thickness.

In some configurations, the second thickness is less than the fifth thickness.

In some configurations, the second thickness is less than or equal to either one of the third thickness and the fourth thickness.

In some configurations, the second thickness is greater than or equal to either one of the third thickness and the fourth thickness.

In some configurations, the first thickness is greater than or equal to the fifth thickness.

In some configurations, the first thickness is less than or equal to the fifth thickness.

In some configurations, the fourth thickness varies along at least a part of the length of the friction member.

In some configurations, the fourth thickness varies along at least a part of the length of the friction member from a minimum at or near a proximal end of the friction member to a maximum at or near a distal end of the friction member.

In some configurations, the at least a part of the length of the friction member from a maximum at or near a proximal end of the friction member to a minimum at or near a distal end of the friction member.

In some configurations, the third thickness is zero.

In some configurations, a portion of the friction member that overlaps with the collar defines an overlap length.

In some configurations, the overlap length is greater than or equal to the third length.

In some configurations, the overlap length is greater than or equal to a sum of the second length and the third length.

In some configurations, the overlap length is less than the third length.

In some configurations, the overlap length is less than a sum of the second length and the third length.

In some configurations, the overlap length equals the fourth length.

In some configurations, the overlap length is less than the fourth length.

In some configurations, the overlap length is greater than the fourth length.

In some configurations, a portion of the friction member that does not overlap with the collar defines a non-overlap length and a portion of the front wall adjacent the support flange defines a first front wall thickness.

In some configurations, a sum of the first length and the first front wall thickness equals a sum of the overlap length and the non-overlap length.

In some configurations, a sum of the first length and the first front wall thickness is greater than a sum of the overlap length and the non-overlap length.

In some configurations, a sum of the first length and the first front wall thickness is less than a sum of the overlap length and the non-overlap length.

In some configurations, the support flange comprises a plurality of apertures and the friction member extends through each of the plurality of apertures. A distance between the portion of the front wall and a point on the plurality of apertures furthest from the portion of the front wall defines an aperture offset distance.

In some configurations, the non-overlap length is greater than or equal to a sum of the aperture offset distance and the first front wall thickness.

In some configurations, the non-overlap length is less than or equal to a sum of the aperture offset distance and the first front wall thickness.

In some configurations, a sum of the second length and the third length is greater than or equal to a sum of the overlap length and the aperture offset distance.

In some configurations, a sum of the second length and the third length is less than or equal to a sum of the overlap length and the aperture offset distance.

In some configurations, a sum of the overlap length and the aperture offset distance is less than or equal to first length.

In some configurations, a sum of the overlap length and the aperture offset distance is greater than or equal to first length.

In some configurations, a relative angle is defined between the collar and at least one of the support flange and a surface of the friction member that engages the collar in a direction of assembly of the cushion module to the frame.

In some configurations, the collar is angled relative to the direction of assembly.

In some configurations, at least one of the support flange and the surface of the friction member that engages the collar is angled relative to the direction of assembly.

In some configurations, a relative angle is defined between the collar and a surface of the friction member that engages the collar in a direction of assembly of the cushion module to the frame.

In some configurations, the collar is angled relative to the direction of assembly.

In some configurations, the surface of the friction member that engages the collar is angled relative to the direction of assembly.

In some configurations, the friction coupling is configured such that a force acting to compress the friction member is equal along a length of the collar.

In some configurations, the friction coupling is configured such that a force acting to compress the friction member is unequal along a length of the collar.

In some configurations, the force is larger at a location closer to the front wall relative to a location further from the front wall.

In some configurations, the force is smaller at a location closer to the front wall relative to a location further from the front wall.

In some configurations, a rearward-most extent of the friction member is at or forward of the front wall.

In some configurations, a rearward-most extent of the friction member is rearward of the front wall such that a portion of the friction member is located adjacent each of a forward surface and a rearward surface of the front wall.

In some configurations, the portion of the friction member adjacent the forward surface of the front wall has a surface that is aligned with a surface of the portion of the friction member adjacent the rearward surface of the front wall.

In some configurations, the connection opening is defined by a support wall, wherein the support wall is disposed, at least in part, within the friction member such that the friction member is coupled to the cushion module.

In some configurations, the collar defines a collar length, the support wall defines a support wall thickness, and the friction member defines a total friction member length, a forward length forward of the support wall and a rearward length rearward of the support wall.

In some configurations, the total friction member length is equal to a sum of the forward length, the rearward length and the support wall thickness.

In some configurations, the total friction member length is greater than a sum of the forward length, the rearward length and the support wall thickness.

In some configurations, the total friction member length is less than a sum of the forward length, the rearward length and the support wall thickness.

In some configurations, the collar defines a collar thickness and the friction member defines a total thickness, a supported thickness along the support wall and an unsupported thickness inwardly of the support wall.

In some configurations, the total thickness equals a sum of the supported thickness and the unsupported thickness.

In some configurations, the total thickness equals twice the supported thickness.

In some configurations, the total thickness equals twice the unsupported thickness.

In some configurations, the supported thickness is equal to the unsupported thickness.

In some configurations, the supported thickness is greater than or equal to the unsupported thickness.

In some configurations, the supported thickness is less than or equal to the unsupported thickness.

In some configurations, the total thickness is greater than or equal to the collar thickness.

In some configurations, the total thickness is less than or equal to the collar thickness.

In some configurations, a portion of the friction member that overlaps with the collar defines an overlap length, a portion of the friction member that does not overlap with the collar defines a non-overlap length and a distance between a rearward end of the collar and the support wall along the direction of assembly defines a support wall offset distance.

In some configurations, the overlap length is greater than or equal to the support wall offset distance.

In some configurations, the overlap length is less than the support wall offset distance.

In some configurations, the overlap length is greater than or equal to the non-overlap length.

In some configurations, the overlap length is less than the non-overlap length.

In some configurations, the non-overlap length is greater than or equal to a sum of the support wall offset distance and the support wall thickness.

In some configurations, the non-overlap length is less than or equal to a sum of the support wall offset distance and the support wall thickness.

In some configurations, the overlap length equals the collar length.

In some configurations, the overlap length is less than the collar length.

In some configurations, the overlap length is greater than the collar length.

In some configurations, the total friction member length equals a sum of the overlap length and the non-overlap length.

In some configurations, the overlap length is less than the forward length.

In some configurations, the overlap length is greater than or equal to the forward length.

In some configurations, the overlap length is greater than or equal to a sum of the forward length, the rearward length and the support wall thickness.

In some configurations, the overlap length is less than or equal to a sum of the forward length, the rearward length and the support wall thickness.

In some configurations, the support wall thickness is less than or equal to the rearward length.

In some configurations, the support wall thickness is greater than the rearward length.

In some configurations, at least a surface of the friction coupling that engages the collar has a frosted or textured surface finish.

In some configurations, the internal engagement surface comprises a first engagement surface portion and a second engagement surface portion. A retention force between the internal engagement surface and the outer surface of the collar varies between the first engagement surface portion and the second engagement surface portion.

In some configurations, the first engagement surface portion and the second engagement surface portion define an angle relative to another.

In some configurations, at least one of the first engagement surface portion and the second engagement surface portion define an angle relative to the outer surface of the collar.

In some configurations, the second engagement surface portion does not contact the outer surface of the collar.

In some configurations, the retention force varies over a length of the first engagement surface.

In some configurations, the elastomeric friction member extends in a rearward direction from the housing and into the breathing chamber.

In some configurations, an entirety of the second engagement surface portion is located within the breathing chamber.

In some configurations, the elastomeric friction member also extends in a forward direction from the housing away from the breathing chamber.

In some configurations, the elastomeric friction member extends in the rearward direction further than it extends in the forward direction.

In some configurations, the outer surface of the collar has a first perimeter portion and a second perimeter portion.

In some configurations, the first perimeter portion has a first perimeter, the second perimeter portion has a second perimeter, and the first perimeter is greater than the second perimeter.

In some configurations, the first perimeter portion is more proximal to the front wall than the second perimeter portion.

In some configurations, the embedded portion is adjacent the first perimeter portion when the cushion module is coupled to the frame.

In some configurations, the embedded portion is adjacent the second perimeter portion when the cushion module is coupled to the frame.

In some configurations, one or both of the elastomeric friction member and the outer surface of the collar define a circular shape and the respiratory mask further comprises structures to indicate a correct assembly orientation, inhibit or prevent incorrect assembly, or guide the cushion module and the frame towards or into the correct assembly orientation.

In some configurations, the structures inhibit or prevent relative rotational movement of the cushion module and the frame when the cushion module is properly assembled to the frame.

In some configurations, the structures comprise one or more sets of cooperating protrusions and recesses.

In some configurations, the collar comprises at least one of the recesses and the elastomeric friction member comprises at least one of the protrusions.

In some configurations, the protrusion is configured to occupy an entirety of a corresponding recess.

In some configurations, the recess comprises an angled surface relative to a direction of assembly of the cushion module to the frame.

In some configurations, the recess comprises a straight surface that is aligned with the direction of assembly.

In some configurations, the angled surface and the straight surface cooperate to form a generally triangular shape.

In some configurations, the angled surface is configured to contact the corresponding protrusion to guide the cushion module towards or to the correct assembly orientation.

In some configurations, both of a first set and a second set of the cooperating protrusions and recesses are located within an upper half of the elastomeric friction member and the collar, respectively, and the angled surfaces are located further from a vertical centerline of the frame than the straight surfaces.

In some configurations, the embedded portion provides radial support to the elastomeric friction member.

In some configurations, the frame further comprises a conduit connector that is unitarily formed with one or both of the front wall and the collar.

In some configurations, the housing of the cushion module defines an inlet recess positioned below the connection opening, and wherein the inlet recess is configured to accommodate a portion of the conduit connector.

In some configurations, the inlet recess is configured to accommodate a rearward-facing portion of the conduit connector.

In some configurations, the housing of the cushion module comprises a recess that surrounds the connection opening.

In some configurations, the embedded portion is displaced rearward from a main wall of the housing along an axis of the connection opening by a connecting portion.

In some configurations, the housing of the cushion module comprises a recess that extends around only a portion of the connection opening.

In some configurations, the housing of the cushion module comprises a recess that extends around only an upper portion of the connection opening.

In some configurations, the housing of the cushion module comprises a recess that extends around only a lower portion of the connection opening.

In some configurations, at least a portion of the friction member is located within the recess. In some configurations, the portion of the friction member defines a portion of the recess.

In some configurations, at least a portion of the friction member protrudes in front of the recess.

In some configurations, the recess defines a depth that is less than a total length of the friction member or a length of the friction member forward of the embedded portion of the housing.

In some configurations, the recess defines a depth that is greater than or equal to a total length of the friction member or a length of the friction member forward of the embedded portion of the housing.

In some configurations, the housing further comprises a support wall that supports the friction member and defines a portion or an entirety of the embedded portion, and the support wall is connected to the front wall by a connecting portion.

In some configurations, the housing further comprises a support wall that supports the friction member and defines a portion or an entirety of the embedded portion, and a radially-inward end of the support wall is connected to a support flange having a support flange length that is greater than a thickness of the support wall.

In some configurations, the support flange defines a longitudinal axis that is offset from an axis defined by a surface of the friction member that engages the collar.

In some configurations, an opening of the friction member defines a non-circular shape.

In some configurations, the opening of the friction member comprises a first axis and a second axis, the first axis extends in a lateral direction across a widest part of the opening, and the second axis extends in a vertical direction.

In some configurations, the friction member is symmetric about the second axis.

In some configurations, the friction member is asymmetric about the first axis.

In some configurations, the first axis is a major axis and the second axis is a minor axis. In some such configurations, the seal is configured to seal below the bridge of the nose of the user.

In some configurations, the first axis is a minor axis and the second axis is a major axis. In some such configurations, the seal is configured to seal on the bridge of the nose of the user.

In some configurations, a ratio of the first axis to the second axis is between about 1.2-1.4:1, is about 1.3:1, or is about 1.27:1.

In some configurations, a respiratory mask includes a frame configured to connect to headgear. The frame comprises a front wall at least partially defining a gas inlet opening. The frame further comprises a collar extending away from the front wall. The collar at least partially surrounds the gas inlet opening. A cushion module comprises a housing, a cushion and an elastomeric friction member. The cushion defines a face-contacting surface. The housing defines a connection opening. A portion of the housing that defines the connection opening is embedded within the elastomeric friction member to couple the friction member to the housing. The friction member engages the collar when the cushion module is coupled to the frame.

In some configurations, the housing comprises a wall portion external of the friction member and extending between the friction member and the cushion.

In some configurations, the front wall of the frame further comprises a vent, and the collar surrounds the vent in addition to the gas inlet.

In some configurations, a divider wall is located within an interior of the collar. The divider wall has a first end and a second end, each connected to the collar. The divider wall separates the vent and the gas inlet opening.

In some configurations, a surface of the divider wall adjacent the vent extends substantially in a direction of gas flow through the vent.

In some configurations, an elbow is supported by the frame and configured to deliver a flow of breathing gas through the gas inlet opening.

In some configurations, the collar and the divider wall cooperate to define a socket that receives the elbow.

In some configurations, the elbow has a swivel connection with the frame.

In some configurations, the collar extends rearwardly from the front wall of the frame to a rearward edge, and a rearward edge of the divider wall is positioned between the front wall of the frame and the rearward edge of the collar.

In some configurations, the housing of the cushion module further comprises a support flange, and the support flange defines the connection opening and extends within the friction member substantially in a direction of assembly of the cushion module to the frame.

In some configurations, the support flange comprises a plurality of apertures and the friction member extends through each of the plurality of apertures.

In some configurations, the embedded portion of the housing comprises a plurality of apertures and wherein the elastomeric friction member extends through each of the apertures.

In some configurations, the embedded portion of the housing and the support flange each comprise a plurality of apertures and wherein the elastomeric friction member extends through each of the apertures.

In some configurations, a plurality of apertures are located at a junction between the support flange and a portion of the wall portion embedded within the elastomeric friction member, and the elastomeric friction member extends through each of the apertures.

In some configurations, the friction member defines an abutment surface that contacts the front wall of the frame when the cushion module is coupled to the frame.

In some configurations, the friction member defines a first length, the support flange defines a second length, an unsupported portion of the friction member that extends beyond the support flange defines a third length, and the collar defines a fourth length.

In some configurations, the first length is equal to the sum of the second length and the third length.

In some configurations, the first length is greater than the sum of the second length and the third length.

In some configurations, the first length is less than the sum of the second length and the third length.

In some configurations, the friction member defines a first thickness, the support flange defines a second thickness, a portion of the friction member located outside of the support flange defines a third thickness, a portion of the friction member located inside of the support flange defines a fourth thickness, and the collar defines a fifth thickness.

In some configurations, the first thickness equals the sum of the second thickness, the third thickness, and the fourth thickness.

In some configurations, the third thickness and the fourth thickness are equal.

In some configurations, the second thickness is equal to each of the third thickness and the fourth thickness.

In some configurations, the fourth thickness is greater than or equal to the third thickness.

In some configurations, the fourth thickness is less than or equal to the third thickness.

In some configurations, the second thickness and the fifth thickness are equal.

In some configurations, the second thickness is greater than the fifth thickness.

In some configurations, the second thickness is less than the fifth thickness.

In some configurations, the second thickness is less than or equal to either one of the third thickness and the fourth thickness.

In some configurations, the second thickness is greater than or equal to either one of the third thickness and the fourth thickness.

In some configurations, the first thickness is greater than or equal to the fifth thickness.

In some configurations, the first thickness is less than or equal to the fifth thickness.

In some configurations, the fourth thickness varies along at least a part of the length of the friction member.

In some configurations, the fourth thickness varies along at least a part of the length of the friction member from a minimum at or near a proximal end of the friction member to a maximum at or near a distal end of the friction member.

In some configurations, the fourth thickness varies along at least a part of the length of the friction member from a maximum at or near a proximal end of the friction member to a minimum at or near a distal end of the friction member.

In some configurations, the third thickness is zero.

In some configurations, a portion of the friction member that overlaps with the collar defines an overlap length.

In some configurations, the overlap length is greater than or equal to the third length.

In some configurations, the overlap length is greater than or equal to a sum of the second length and the third length.

In some configurations, the overlap length is less than the third length.

In some configurations, the overlap length is less than a sum of the second length and the third length.

In some configurations, the overlap length equals the fourth length.

In some configurations, the overlap length is less than the fourth length.

In some configurations, the overlap length is greater than the fourth length.

In some configurations, a portion of the friction member that does not overlap with the collar defines a non-overlap length and a portion of the front wall adjacent the support flange defines a first front wall thickness.

In some configurations, a sum of the first length and the first front wall thickness equals a sum of the overlap length and the non-overlap length.

In some configurations, a sum of the first length and the first front wall thickness is greater than a sum of the overlap length and the non-overlap length.

In some configurations, a sum of the first length and the first front wall thickness is less than a sum of the overlap length and the non-overlap length.

In some configurations, the support flange comprises a plurality of apertures and the friction member extends through each of the plurality of apertures. A distance between the portion of the front wall and a point on the plurality of apertures furthest from the portion of the front wall defines an aperture offset distance.

In some configurations, the non-overlap length is greater than or equal to a sum of the aperture offset distance and the first front wall thickness.

In some configurations, the non-overlap length is less than or equal to a sum of the aperture offset distance and the first front wall thickness.

In some configurations, a sum of the second length and the third length is greater than or equal to a sum of the overlap length and the aperture offset distance.

In some configurations, a sum of the second length and the third length is less than or equal to a sum of the overlap length and the aperture offset distance.

In some configurations, a sum of the overlap length and the aperture offset distance is less than or equal to first length.

In some configurations, a sum of the overlap length and the aperture offset distance is greater than or equal to first length.

In some configurations, a relative angle is defined between the collar and at least one of the support flange and a surface of the friction member that engages the collar in a direction of assembly of the cushion module to the frame.

In some configurations, the collar is angled relative to the direction of assembly.

In some configurations, at least one of the support flange and the surface of the friction member that engages the collar is angled relative to the direction of assembly.

In some configurations, a relative angle is defined between the collar and a surface of the friction member that engages the collar in a direction of assembly of the cushion module to the frame.

In some configurations, the collar is angled relative to the direction of assembly.

In some configurations, the surface of the friction member that engages the collar is angled relative to the direction of assembly.

In some configurations, the friction coupling is configured such that a force acting to compress the friction member is equal along a length of the collar.

In some configurations, the friction coupling is configured such that a force acting to compress the friction member is unequal along a length of the collar.

In some configurations, the force is larger at a location closer to the front wall relative to a location further from the front wall.

In some configurations, the force is smaller at a location closer to the front wall relative to a location further from the front wall.

In some configurations, a rearward-most extent of the friction member is at or forward of the front wall.

In some configurations, a rearward-most extent of the friction member is rearward of the front wall such that a portion of the friction member is located adjacent each of a forward surface and a rearward surface of the front wall.

In some configurations, the portion of the friction member adjacent the forward surface of the front wall has a surface that is aligned with a surface of the portion of the friction member adjacent the rearward surface of the front wall.

In some configurations, the connection opening is defined by a support wall, and the support wall is disposed, at least in part, within the friction member such that the friction member is coupled to the cushion module.

In some configurations, the collar defines a collar length, the support wall defines a support wall thickness, and the friction member defines a total friction member length, a forward length forward of the support wall and a rearward length rearward of the support wall.

In some configurations, the total friction member length is equal to a sum of the forward length, the rearward length and the support wall thickness.

In some configurations, the total friction member length is greater than a sum of the forward length, the rearward length and the support wall thickness.

In some configurations, the total friction member length is less than a sum of the forward length, the rearward length and the support wall thickness.

In some configurations, the collar defines a collar thickness and the friction member defines a total thickness, a supported thickness along the support wall and an unsupported thickness inwardly of the support wall.

In some configurations, the total thickness equals a sum of the supported thickness and the unsupported thickness.

In some configurations, the total thickness equals twice the supported thickness.

In some configurations, the total thickness equals twice the unsupported thickness.

In some configurations, the supported thickness is equal to the unsupported thickness.

In some configurations, the supported thickness is greater than or equal to the unsupported thickness.

In some configurations, the supported thickness is less than or equal to the unsupported thickness.

In some configurations, the total thickness is greater than or equal to the collar thickness.

In some configurations, the total thickness is less than or equal to the collar thickness.

In some configurations, a portion of the friction member that overlaps with the collar defines an overlap length, a portion of the friction member that does not overlap with the collar defines a non-overlap length and a distance between a rearward end of the collar and the support wall along the direction of assembly defines a support wall offset distance.

In some configurations, the overlap length is greater than or equal to the support wall offset distance.

In some configurations, the overlap length is less than the support wall offset distance.

In some configurations, the overlap length is greater than or equal to the non-overlap length.

In some configurations, the overlap length is less than the non-overlap length.

In some configurations, the non-overlap length is greater than or equal to a sum of the support wall offset distance and the support wall thickness.

In some configurations, the non-overlap length is less than or equal to a sum of the support wall offset distance and the support wall thickness.

In some configurations, the overlap length equals the collar length.

In some configurations, the overlap length is less than the collar length.

In some configurations, the overlap length is greater than the collar length.

In some configurations, the total friction member length equals a sum of the overlap length and the non-overlap length.

In some configurations, the overlap length is less than the forward length.

In some configurations, the overlap length is greater than or equal to the forward length.

In some configurations, the overlap length is greater than or equal to a sum of the forward length, the rearward length and the support wall thickness.

In some configurations, the overlap length is less than or equal to a sum of the forward length, the rearward length and the support wall thickness.

In some configurations, the support wall thickness is less than or equal to the rearward length.

In some configurations, the support wall thickness is greater than the rearward length.

In some configurations, the embedded portion provides radial support to the elastomeric friction member.

In some configurations, the collar is inboard of an outer perimeter of the frame.

In some configurations, the frame further comprises a conduit connector that is unitarily formed with one or both of the front wall and the collar.

In some configurations, the housing of the cushion module defines an inlet recess positioned below the connection opening, and wherein the inlet recess is configured to accommodate a portion of the conduit connector.

In some configurations, the inlet recess is configured to accommodate a rearward-facing portion of the conduit connector.

In some configurations, the housing of the cushion module comprises a recess that surrounds the connection opening.

In some configurations, the embedded portion is displaced rearward from a main wall of the housing along an axis of the connection opening by a connecting portion.

In some configurations, the housing of the cushion module comprises a recess that extends around only a portion of the connection opening.

In some configurations, the housing of the cushion module comprises a recess that extends around only an upper portion of the connection opening.

In some configurations, the housing of the cushion module comprises a recess that extends around only a lower portion of the connection opening.

In some configurations, at least a portion of the friction member is located within the recess. In some configurations, the portion of the friction member defines a portion of the recess.

In some configurations, at least a portion of the friction member protrudes in front of the recess.

In some configurations, the recess defines a depth that is less than a total length of the friction member or a length of the friction member forward of the embedded portion of the housing.

In some configurations, the recess defines a depth that is greater than or equal to a total length of the friction member or a length of the friction member forward of the embedded portion of the housing.

In some configurations, the housing further comprises a support wall that supports the friction member and defines a portion or an entirety of the embedded portion, and the support wall is connected to the front wall by a connecting portion.

In some configurations, the housing further comprises a support wall that supports the friction member and defines a portion or an entirety of the embedded portion, and a radially-inward end of the support wall is connected to a support flange having a support flange length that is greater than a thickness of the support wall.

In some configurations, the support flange defines a longitudinal axis that is offset from an axis defined by a surface of the friction member that engages the collar.

In some configurations, an opening of the friction member defines a non-circular shape.

In some configurations, the opening of the friction member comprises a first axis and a second axis, the first axis extends in a lateral direction across a widest part of the opening, and the second axis extends in a vertical direction.

In some configurations, the friction member is symmetric about the second axis.

In some configurations, the friction member is asymmetric about the first axis.

In some configurations, the first axis is a major axis and the second axis is a minor axis. In some such configurations, the seal is configured to seal below the bridge of the nose of the user.

In some configurations, the first axis is a minor axis and the second axis is a major axis. In some such configurations, the seal is configured to seal on the bridge of the nose of the user.

In some configurations, a ratio of the first axis to the second axis is between about 1.2-1.4:1, is about 1.3:1, or is about 1.27:1.

In some configurations, a cushion module for a respiratory mask is configured for attachment to a cooperating structure, such as a frame and/or a conduit connector (e.g., elbow). The frame can be configured to connect to headgear. The frame can include a front wall at least partially defining a gas inlet opening and a collar extending away from the front wall, which can at least partially surround the gas inlet opening. The cushion module comprises a housing, a cushion and an elastomeric friction member. The cushion defines a face-contacting surface. The housing defines a connection opening. A portion of the housing defining the connection opening is embedded within the elastomeric friction member to couple the friction member to the housing. The friction member is configured to engage a corresponding portion of the frame, such as the collar, when the cushion module is coupled to the frame.

In some configurations, the housing comprises a wall portion external of the friction member and extending between the friction member and the cushion.

In some configurations, the housing of the cushion module further comprises a support flange. The support flange defines the connection opening and extends within the friction member substantially in a direction of assembly of the cushion module to the frame.

In some configurations, the support flange comprises a plurality of apertures and the friction member extends through each of the plurality of apertures.

In some configurations, the wall portion of the housing comprises a plurality of apertures and where in the friction member extends through each of the apertures.

In some configurations, the housing comprises a plurality of apertures at a junction between the support flange and an adjacent portion of the housing and wherein the elastomeric friction member extends through each of the apertures.

In some configurations, the friction member defines an abutment surface that is configured to contact the front wall of the frame when the cushion module is coupled to the frame.

In some configurations, the friction member defines a first length, the support flange defines a second length, an unsupported portion of the friction member that extends beyond the support flange defines a third length, and the collar defines a fourth length.

In some configurations, the first length is equal to the sum of the second length and the third length.

In some configurations, the first length is greater than the sum of the second length and the third length.

In some configurations, the first length is less than the sum of the second length and the third length.

In some configurations, the friction member defines a first thickness, the support flange defines a second thickness, a portion of the friction member located outside of the support flange defines a third thickness, a portion of the friction member located inside of the support flange defines a fourth thickness, and the collar defines a fifth thickness.

In some configurations, the first thickness equals the sum of the second thickness, the third thickness, and the fourth thickness.

In some configurations, the third thickness and the fourth thickness are equal.

In some configurations, the second thickness is equal to each of the third thickness and the fourth thickness.

In some configurations, the fourth thickness is greater than or equal to the third thickness.

In some configurations, the fourth thickness is less than or equal to the third thickness.

In some configurations, the second thickness and the fifth thickness are equal.

In some configurations, the second thickness is greater than the fifth thickness.

In some configurations, the second thickness is less than the fifth thickness.

In some configurations, the second thickness is less than or equal to either one of the third thickness and the fourth thickness.

In some configurations, the second thickness is greater than or equal to either one of the third thickness and the fourth thickness.

In some configurations, the first thickness is greater than or equal to the fifth thickness.

In some configurations, the first thickness is less than or equal to the fifth thickness.

In some configurations, the fourth thickness varies along at least a part of the length of the friction member.

In some configurations, the fourth thickness varies along at least a part of the length of the friction member from a minimum at or near a proximal end of the friction member to a maximum at or near a distal end of the friction member.

In some configurations, the fourth thickness varies along at least a part of the length of the friction member from a maximum at or near a proximal end of the friction member to a minimum at or near a distal end of the friction member.

In some configurations, the third thickness is zero.

In some configurations, a portion of the friction member that overlaps with the collar defines an overlap length.

In some configurations, the overlap length is greater than or equal to the third length.

In some configurations, the overlap length is greater than or equal to a sum of the second length and the third length.

In some configurations, the overlap length is less than the third length.

In some configurations, the overlap length is less than a sum of the second length and the third length.

In some configurations, the overlap length equals the fourth length.

In some configurations, the overlap length is less than the fourth length.

In some configurations, the overlap length is greater than the fourth length.

In some configurations, a portion of the friction member that does not overlap with the collar defines a non-overlap length and a portion of the front wall adjacent the support flange defines a first front wall thickness.

In some configurations, a sum of the first length and the first front wall thickness equals a sum of the overlap length and the non-overlap length.

In some configurations, a sum of the first length and the first front wall thickness is greater than a sum of the overlap length and the non-overlap length.

In some configurations, a sum of the first length and the first front wall thickness is less than a sum of the overlap length and the non-overlap length.

In some configurations, the support flange comprises a plurality of apertures and the friction member extends through each of the plurality of apertures. A distance between the portion of the front wall and a point on the plurality of apertures furthest from the portion of the front wall defines an aperture offset distance.

In some configurations, the non-overlap length is greater than or equal to a sum of the aperture offset distance and the first front wall thickness.

In some configurations, the non-overlap length is less than or equal to a sum of the aperture offset distance and the first front wall thickness.

In some configurations, a sum of the second length and the third length is greater than or equal to a sum of the overlap length and the aperture offset distance.

In some configurations, a sum of the second length and the third length is less than or equal to a sum of the overlap length and the aperture offset distance.

In some configurations, a sum of the overlap length and the aperture offset distance is less than or equal to first length.

In some configurations, a sum of the overlap length and the aperture offset distance is greater than or equal to the first length.

In some configurations, a relative angle is defined between the collar and at least one of the support flange and a surface of the friction member that engages the collar in a direction of assembly of the cushion module to the frame.

In some configurations, at least one of the support flange and the surface of the friction member that engages the collar is angled relative to the direction of assembly.

In some configurations, a relative angle is defined between the collar and a surface of the friction member that engages the collar in a direction of assembly of the cushion module to the frame.

In some configurations, the surface of the friction member that engages the collar is angled relative to the direction of assembly.

In some configurations, the friction coupling is configured such that a force acting to compress the friction member is equal along a length of the collar.

In some configurations, the friction coupling is configured such that a force acting to compress the friction member is unequal along a length of the collar.

In some configurations, the force is larger at a location closer to the front wall relative to a location further from the front wall.

In some configurations, the force is smaller at a location closer to the front wall relative to a location further from the front wall.

In some configurations, a rearward-most extent of the friction member is at or forward of the front wall.

In some configurations, a rearward-most extent of the friction member is rearward of the front wall such that a portion of the friction member is located adjacent each of a forward surface and a rearward surface of the front wall.

In some configurations, the portion of the friction member adjacent the forward surface of the front wall has a surface that is aligned with a surface of the portion of the friction member adjacent the rearward surface of the front wall.

In some configurations, the connection opening is defined by a support wall, and the support wall is disposed, at least in part, within the friction member such that the friction member is coupled to the cushion module.

In some configurations, the collar defines a collar length, the support wall defines a support wall thickness, and the friction member defines a total friction member length, a forward length forward of the support wall and a rearward length rearward of the support wall.

In some configurations, the total friction member length is equal to a sum of the forward length, the rearward length and the support wall thickness.

In some configurations, the total friction member length is greater than a sum of the forward length, the rearward length and the support wall thickness.

In some configurations, the total friction member length is less than a sum of the forward length, the rearward length and the support wall thickness.

In some configurations, the collar defines a collar thickness and the friction member defines a total thickness, a supported thickness along the support wall and an unsupported thickness inwardly of the support wall.

In some configurations, the total thickness equals a sum of the supported thickness and the unsupported thickness.

In some configurations, the total thickness equals twice the supported thickness.

In some configurations, the total thickness equals twice the unsupported thickness.

In some configurations, the supported thickness is equal to the unsupported thickness.

In some configurations, the supported thickness is greater than or equal to the unsupported thickness.

In some configurations, the supported thickness is less than or equal to the unsupported thickness.

In some configurations, the total thickness is greater than or equal to the collar thickness.

In some configurations, the total thickness is less than or equal to the collar thickness.

In some configurations, a portion of the friction member that overlaps with the collar defines an overlap length, a portion of the friction member that does not overlap with the collar defines a non-overlap length and a distance between a rearward end of the collar and the support wall along the direction of assembly defines a support wall offset distance.

In some configurations, the overlap length is greater than or equal to the support wall offset distance.

In some configurations, the overlap length is less than the support wall offset distance.

In some configurations, the overlap length is greater than or equal to the non-overlap length.

In some configurations, the overlap length is less than the non-overlap length.

In some configurations, the non-overlap length is greater than or equal to a sum of the support wall offset distance and the support wall thickness.

In some configurations, the non-overlap length is less than or equal to a sum of the support wall offset distance and the support wall thickness.

In some configurations, the overlap length equals the collar length.

In some configurations, the overlap length is less than the collar length.

In some configurations, the overlap length is greater than the collar length.

In some configurations, the total friction member length equals a sum of the overlap length and the non-overlap length.

In some configurations, the overlap length is less than the forward length.

In some configurations, the overlap length is greater than or equal to the forward length.

In some configurations, the overlap length is greater than or equal to a sum of the forward length, the rearward length and the support wall thickness.

In some configurations, the overlap length is less than or equal to a sum of the forward length, the rearward length and the support wall thickness.

In some configurations, the support wall thickness is less than or equal to the rearward length.

In some configurations, the support wall thickness is greater than the rearward length.

In some configurations, the embedded portion provides radial support to the elastomeric friction member.

In some configurations, the frame further comprises a conduit connector that is unitarily formed with one or both of the front wall and the collar.

In some configurations, the housing of the cushion module defines an inlet recess positioned below the connection opening, and wherein the inlet recess is configured to accommodate a portion of the conduit connector.

In some configurations, the inlet recess is configured to accommodate a rearward-facing portion of the conduit connector.

In some configurations, the housing of the cushion module comprises a recess that surrounds the connection opening.

In some configurations, the embedded portion is displaced rearward from a main wall of the housing along an axis of the connection opening by a connecting portion.

In some configurations, the housing of the cushion module comprises a recess that extends around only a portion of the connection opening.

In some configurations, the housing of the cushion module comprises a recess that extends around only an upper portion of the connection opening.

In some configurations, the housing of the cushion module comprises a recess that extends around only a lower portion of the connection opening.

In some configurations, at least a portion of the friction member is located within the recess. In some configurations, the portion of the friction member defines a portion of the recess.

In some configurations, at least a portion of the friction member protrudes in front of the recess.

In some configurations, the recess defines a depth that is less than a total length of the friction member or a length of the friction member forward of the embedded portion of the housing.

In some configurations, the recess defines a depth that is greater than or equal to a total length of the friction member or a length of the friction member forward of the embedded portion of the housing.

In some configurations, the housing further comprises a support wall that supports the friction member and defines a portion or an entirety of the embedded portion, and the support wall is connected to the front wall by a connecting portion.

In some configurations, the housing further comprises a support wall that supports the friction member and defines a portion or an entirety of the embedded portion, and a radially-inward end of the support wall is connected to a support flange having a support flange length that is greater than a thickness of the support wall.

In some configurations, the support flange defines a longitudinal axis that is offset from an axis defined by a surface of the friction member that engages the collar.

In some configurations, an opening of the friction member defines a non-circular shape.

In some configurations, the opening of the friction member comprises a first axis and a second axis, the first axis extends in a lateral direction across a widest part of the opening, and the second axis extends in a vertical direction.

In some configurations, the friction member is symmetric about the second axis.

In some configurations, the friction member is asymmetric about the first axis.

In some configurations, the first axis is a major axis and the second axis is a minor axis. In some such configurations, the seal is configured to seal below the bridge of the nose of the user.

In some configurations, the first axis is a minor axis and the second axis is a major axis. In some such configurations, the seal is configured to seal on the bridge of the nose of the user.

In some configurations, a ratio of the first axis to the second axis is between about 1.2-1.4:1, is about 1.3:1, or is about 1.27:1.

In some configurations, a cushion module for a respiratory mask is configured for attachment to a cooperating structure, such as a frame and/or a conduit connector (e.g., elbow). The frame can include a front wall and a collar extending away from the front wall. The cushion module comprises a housing, a cushion and an elastomeric friction member. The cushion defines a face-contacting surface. The housing defines a connection opening and supports the elastomeric friction member. The housing comprises a wall portion, at least a portion of which is external to the elastomeric friction member and extends between the elastomeric friction member and the cushion.

In some configurations, a portion of the housing defining the connection opening is embedded within the elastomeric friction member to couple the elastomeric friction member to the housing.

In some configurations, the housing of the cushion module further comprises a support flange. The support flange defines the connection opening and extends within the friction member substantially in a direction of assembly of the cushion module to the frame.

In some configurations, the support flange comprises a plurality of apertures and the friction member extends through each of the plurality of apertures. In some configurations, the wall portion of the housing comprises a plurality of apertures and the friction member extends through each of the apertures. In some configurations, the housing comprises a plurality of apertures at a junction between the support flange and an adjacent portion of the housing and wherein the elastomeric friction member extends through each of the apertures.

In some configurations, the friction member defines an abutment surface that is configured to contact the front wall of the frame when the cushion module is coupled to the frame.

In some configurations, the friction member defines a first length, the support flange defines a second length, an unsupported portion of the friction member that extends beyond the support flange defines a third length, and the collar defines a fourth length.

In some configurations, the first length is equal to the sum of the second length and the third length.

In some configurations, the first length is greater than the sum of the second length and the third length.

In some configurations, the first length is less than the sum of the second length and the third length.

In some configurations, the friction member defines a first thickness, the support flange defines a second thickness, a portion of the friction member located outside of the support flange defines a third thickness, a portion of the friction member located inside of the support flange defines a fourth thickness, and the collar defines a fifth thickness.

In some configurations, the first thickness equals the sum of the second thickness, the third thickness, and the fourth thickness.

In some configurations, the third thickness and the fourth thickness are equal.

In some configurations, the second thickness is equal to each of the third thickness and the fourth thickness.

In some configurations, the fourth thickness is greater than or equal to the third thickness.

In some configurations, the fourth thickness is less than or equal to the third thickness.

In some configurations, the second thickness and the fifth thickness are equal.

In some configurations, the second thickness is greater than the fifth thickness.

In some configurations, the second thickness is less than the fifth thickness.

In some configurations, the second thickness is less than or equal to either one of the third thickness and the fourth thickness.

In some configurations, the second thickness is greater than or equal to either one of the third thickness and the fourth thickness.

In some configurations, the first thickness is greater than or equal to the fifth thickness.

In some configurations, the first thickness is less than or equal to the fifth thickness.

In some configurations, the fourth thickness varies along at least a part of the length of the friction member.

In some configurations, the fourth thickness varies along at least a part of the length of the friction member from a minimum at or near a proximal end of the friction member to a maximum at or near a distal end of the friction member.

In some configurations, the fourth thickness varies along at least a part of the length of the friction member from a maximum at or near a proximal end of the friction member to a minimum at or near a distal end of the friction member.

In some configurations, the third thickness is zero.

In some configurations, a portion of the friction member that overlaps with the collar defines an overlap length.

In some configurations, the overlap length is greater than or equal to the third length.

In some configurations, the overlap length is greater than or equal to a sum of the second length and the third length.

In some configurations, the overlap length is less than the third length.

In some configurations, the overlap length is less than a sum of the second length and the third length.

In some configurations, the overlap length equals the fourth length.

In some configurations, the overlap length is less than the fourth length.

In some configurations, the overlap length is greater than the fourth length.

In some configurations, a portion of the friction member that does not overlap with the collar defines a non-overlap length and a portion of the front wall adjacent the support flange defines a first front wall thickness.

In some configurations, a sum of the first length and the first front wall thickness equals a sum of the overlap length and the non-overlap length.

In some configurations, a sum of the first length and the first front wall thickness is greater than a sum of the overlap length and the non-overlap length.

In some configurations, a sum of the first length and the first front wall thickness is less than a sum of the overlap length and the non-overlap length.

In some configurations, the support flange comprises a plurality of apertures and the friction member extends through each of the plurality of apertures. A distance between the portion of the front wall and a point on the plurality of apertures furthest from the portion of the front wall defines an aperture offset distance.

In some configurations, the non-overlap length is greater than or equal to a sum of the aperture offset distance and the first front wall thickness.

In some configurations, the non-overlap length is less than or equal to a sum of the aperture offset distance and the first front wall thickness.

In some configurations, a sum of the second length and the third length is greater than or equal to a sum of the overlap length and the aperture offset distance.

In some configurations, a sum of the second length and the third length is less than or equal to a sum of the overlap length and the aperture offset distance.

In some configurations, a sum of the overlap length and the aperture offset distance is less than or equal to first length.

In some configurations, a sum of the overlap length and the aperture offset distance is greater than or equal to the first length.

In some configurations, a relative angle is defined between the collar and at least one of the support flange and a surface of the friction member that engages the collar in a direction of assembly of the cushion module to the frame.

In some configurations, at least one of the support flange and the surface of the friction member that engages the collar is angled relative to the direction of assembly.

In some configurations, a relative angle is defined between the collar and a surface of the friction member that engages the collar in a direction of assembly of the cushion module to the frame.

In some configurations, the surface of the friction member that engages the collar is angled relative to the direction of assembly.

In some configurations, the friction coupling is configured such that a force acting to compress the friction member is equal along a length of the collar.

In some configurations, the friction coupling is configured such that a force acting to compress the friction member is unequal along a length of the collar.

In some configurations, the force is larger at a location closer to the front wall relative to a location further from the front wall.

In some configurations, the force is smaller at a location closer to the front wall relative to a location further from the front wall.

In some configurations, a rearward-most extent of the friction member is at or forward of the front wall.

In some configurations, a rearward-most extent of the friction member is rearward of the front wall such that a portion of the friction member is located adjacent each of a forward surface and a rearward surface of the front wall.

In some configurations, the portion of the friction member adjacent the forward surface of the front wall has a surface that is aligned with a surface of the portion of the friction member adjacent the rearward surface of the front wall.

In some configurations, the connection opening is defined by a support wall, and the support wall is disposed, at least in part, within the friction member such that the friction member is coupled to the cushion module.

In some configurations, the collar defines a collar length, the support wall defines a support wall thickness, and the friction member defines a total friction member length, a forward length forward of the support wall and a rearward length rearward of the support wall.

In some configurations, the total friction member length is equal to a sum of the forward length, the rearward length and the support wall thickness.

In some configurations, the total friction member length is greater than a sum of the forward length, the rearward length and the support wall thickness.

In some configurations, the total friction member length is less than a sum of the forward length, the rearward length and the support wall thickness.

In some configurations, the collar defines a collar thickness and the friction member defines a total thickness, a supported thickness along the support wall and an unsupported thickness inwardly of the support wall.

In some configurations, the total thickness equals a sum of the supported thickness and the unsupported thickness.

In some configurations, the total thickness equals twice the supported thickness.

In some configurations, the total thickness equals twice the unsupported thickness.

In some configurations, the supported thickness is equal to the unsupported thickness.

In some configurations, the supported thickness is greater than or equal to the unsupported thickness.

In some configurations, the supported thickness is less than or equal to the unsupported thickness.

In some configurations, the total thickness is greater than or equal to the collar thickness.

In some configurations, the total thickness is less than or equal to the collar thickness.

In some configurations, a portion of the friction member that overlaps with the collar defines an overlap length, a portion of the friction member that does not overlap with the collar defines a non-overlap length and a distance between a rearward end of the collar and the support wall along the direction of assembly defines a support wall offset distance.

In some configurations, the overlap length is greater than or equal to the support wall offset distance.

In some configurations, the overlap length is less than the support wall offset distance.

In some configurations, the overlap length is greater than or equal to the non-overlap length.

In some configurations, the overlap length is less than the non-overlap length.

In some configurations, the non-overlap length is greater than or equal to a sum of the support wall offset distance and the support wall thickness.

In some configurations, the non-overlap length is less than or equal to a sum of the support wall offset distance and the support wall thickness.

In some configurations, the overlap length equals the collar length.

In some configurations, the overlap length is less than the collar length.

In some configurations, the overlap length is greater than the collar length.

In some configurations, the total friction member length equals a sum of the overlap length and the non-overlap length.

In some configurations, the overlap length is less than the forward length.

In some configurations, the overlap length is greater than or equal to the forward length.

In some configurations, the overlap length is greater than or equal to a sum of the forward length, the rearward length and the support wall thickness.

In some configurations, the overlap length is less than or equal to a sum of the forward length, the rearward length and the support wall thickness.

In some configurations, the support wall thickness is less than or equal to the rearward length.

In some configurations, the support wall thickness is greater than the rearward length.

In some configurations, the embedded portion provides radial support to the elastomeric friction member.

In some configurations, the frame further comprises a conduit connector that is unitarily formed with one or both of the front wall and the collar.

In some configurations, the housing of the cushion module defines an inlet recess positioned below the connection opening, and wherein the inlet recess is configured to accommodate a portion of the conduit connector.

In some configurations, the inlet recess is configured to accommodate a rearward-facing portion of the conduit connector.

In some configurations, the housing of the cushion module comprises a recess that surrounds the connection opening.

In some configurations, the embedded portion is displaced rearward from a main wall of the housing along an axis of the connection opening by a connecting portion.

In some configurations, the housing of the cushion module comprises a recess that extends around only a portion of the connection opening.

In some configurations, the housing of the cushion module comprises a recess that extends around only an upper portion of the connection opening.

In some configurations, the housing of the cushion module comprises a recess that extends around only a lower portion of the connection opening.

In some configurations, at least a portion of the friction member is located within the recess. In some configurations, the portion of the friction member defines a portion of the recess.

In some configurations, at least a portion of the friction member protrudes in front of the recess.

In some configurations, the recess defines a depth that is less than a total length of the friction member or a length of the friction member forward of the embedded portion of the housing.

In some configurations, the recess defines a depth that is greater than or equal to a total length of the friction member or a length of the friction member forward of the embedded portion of the housing.

In some configurations, the housing further comprises a support wall that supports the friction member and defines a portion or an entirety of the embedded portion, and the support wall is connected to the front wall by a connecting portion.

In some configurations, the housing further comprises a support wall that supports the friction member and defines a portion or an entirety of the embedded portion, and a radially-inward end of the support wall is connected to a support flange having a support flange length that is greater than a thickness of the support wall.

In some configurations, the support flange defines a longitudinal axis that is offset from an axis defined by a surface of the friction member that engages the collar.

In some configurations, an opening of the friction member defines a non-circular shape.

In some configurations, the opening of the friction member comprises a first axis and a second axis, the first axis extends in a lateral direction across a widest part of the opening, and the second axis extends in a vertical direction.

In some configurations, the friction member is symmetric about the second axis.

In some configurations, the friction member is asymmetric about the first axis.

In some configurations, the first axis is a major axis and the second axis is a minor axis. In some such configurations, the seal is configured to seal below the bridge of the nose of the user.

In some configurations, the first axis is a minor axis and the second axis is a major axis. In some such configurations, the seal is configured to seal on the bridge of the nose of the user.

In some configurations, a ratio of the first axis to the second axis is between about 1.2-1.4: 1, is about 1.3: 1, or is about 1.27:1.

In some configurations, a respiratory mask includes a frame configured to connect to headgear. The frame includes a front wall at least partially defining a gas inlet opening. The frame further includes a collar extending away from the front wall. The collar at least partially surrounds the gas inlet opening. A cushion module includes a housing, a cushion and an elastomeric friction member. The cushion defines a face-contacting surface. The housing includes a main wall that supports the cushion. The housing further includes a support wall that defines a connection opening. At least a portion of the support wall is embedded within the elastomeric friction member to couple the elastomeric friction member to the housing. The support wall is offset from the main wall to define a recess of the housing. The elastomeric friction member is configured to selectively connect the cushion module to the collar of the frame with a friction fit so that a flow of gas can be delivered to the breathing chamber through the gas inlet opening of the frame and the connection opening of the cushion module.

In some configurations, the support flange is offset rearward from the main wall along an axis of the connection opening to define the recess.

In some configurations, the support flange is connected to the main wall by a connecting portion, and a surface of the connecting portion and a surface of the support flange define at least a portion of the recess.

In some configurations, the recess surrounds the connection opening.

In some configurations, the recess extends around only a portion of the connection opening.

In some configurations, the recess extends around only an upper portion of the connection opening.

In some configurations, the recess extends around only a lower portion of the connection opening.

In some configurations, at least a portion of the friction member is located within the recess. In some configurations, the portion of the friction member defines a portion of the recess.

In some configurations, at least a portion of the friction member protrudes in front of the recess.

In some configurations, the recess defines a depth that is less than a total length of the friction member or a length of the friction member forward of the support wall.

In some configurations, the recess defines a depth that is greater than or equal to a total length of the friction member or a length of the friction member forward of the embedded portion of the housing.

In some configurations, a radially-inward end of the support wall is connected to a support flange having a support flange length that is greater than a thickness of the support wall.

In some configurations, the support flange defines a longitudinal axis that is offset from an axis defined by a surface of the friction member that engages the collar.

In some configurations, an opening of the friction member defines a non-circular shape.

In some configurations, the opening of the friction member comprises a first axis and a second axis, wherein the first axis extends in a lateral direction across a widest part of the opening and the second axis extends in a vertical direction.

In some configurations, the friction member is symmetric about the second axis.

In some configurations, the friction member is asymmetric about the first axis.

In some configurations, the first axis is a major axis and the second axis is a minor axis. In some such configurations, the seal is configured to seal below the bridge of the nose of the user.

In some configurations, the first axis is a minor axis and the second axis is a major axis. In some such configurations, the seal is configured to seal on the bridge of the nose of the user.

In some configurations, a ratio of the first axis to the second axis is between about 1.2-1.4:1, is about 1.3:1, or is about 1.27:1.

In some configurations, a respiratory mask includes a frame configured to connect to headgear. The frame includes a front wall at least partially defining a gas inlet opening. The frame further includes a collar extending away from the front wall. The collar at least partially surrounds the gas inlet opening. A cushion module includes a housing, a cushion and an elastomeric friction member. The cushion defines a face-contacting surface. The housing includes a main wall that supports the cushion and a support wall that defines a connection opening. The support wall is offset from the main wall. The elastomeric friction member is configured to selectively connect the cushion module to the collar of the frame with a friction fit so that a flow of gas can be delivered to the breathing chamber through the gas inlet opening of the frame and the connection opening of the cushion module.

In some configurations, the support wall is connected to the main wall by a connecting portion.

In some configurations, the support wall is offset from the main wall by the connecting portion to define a recess of the housing.

In some configurations, at least a portion of the support wall is embedded within the elastomeric friction member to couple the elastomeric friction member to the housing.

In some configurations, the support wall is offset rearward from the main wall along an axis of the connection opening.

In some configurations, the support wall is offset from the main wall around an entirety of the connection opening.

In some configurations, the support wall is offset from the main wall around only a portion of the connection opening.

In some configurations, a respiratory mask includes a frame configured to connect to headgear. The frame includes a front wall at least partially defining a gas inlet opening. The frame further includes a collar extending away from the front wall. The collar at least partially surrounds the gas inlet opening. A cushion module includes a housing, a cushion and an elastomeric friction member. The cushion defines a face-contacting surface. The housing includes a main wall that supports the cushion and a support wall connected to the main wall by a connecting portion. The support wall defines a connection opening. The connecting portion and the support wall at least partially define a recess of the housing. The elastomeric friction member is configured to selectively connect the cushion module to the collar of the frame with a friction fit so that a flow of gas can be delivered to the breathing chamber through the gas inlet opening of the frame and the connection opening of the cushion module.

In some configurations, at least a portion of the support wall is embedded within the elastomeric friction member to couple the elastomeric friction member to the housing.

In some configurations, the recess extends rearward from the main wall along an axis of the connection opening.

In some configurations, the recess extends around an entirety of the connection opening.

In some configurations, the recess extends around only a portion of the connection opening.

In some configurations, at least a portion of the recess is defined by a peripheral surface of the elastomeric friction member.

In some configurations, at least a portion of the elastomeric friction member is disposed in the recess.

In some configurations, a cushion module for a respiratory mask, the cushion module configured for attachment to a frame configured to connect to a headgear, includes a housing, a cushion, and an elastomeric friction member. The cushion defines a face-contacting surface and a cushion outlet opening. The elastomeric friction member is configured to form a friction coupling with the frame. The housing includes a main wall and a support flange extending from the main wall. At least a portion of the support flange is embedded within the elastomeric friction member to couple the elastomeric friction member to the housing. The cushion module defines a connection opening. The support flange includes a first flange portion surrounding a first perimeter portion of the connection opening and defining a first front edge portion of the support flange, and a second flange portion surrounding a second perimeter portion of the connection opening and defining a second front edge portion of the support flange. The second flange portion is offset from the first flange portion.

In some configurations, the second front edge portion of the support flange is offset from the first front edge portion of the support flange.

In some configurations, the second front edge portion of the support flange is rearwardly offset from the first front edge portion of the support flange.

In some configurations, the second flange portion includes lateral portions of the support flange.

In some configurations, the first flange portion includes top and bottom portions of the support flange.

In some configurations, a length of the support flange is constant around an entire perimeter of the connection opening.

In some configurations, a respiratory mask includes the cushion module, the frame, and headgear. In some configurations, the frame includes a front wall and a collar extending away from the front wall. In some configurations, the collar extends rearwardly relative to the front wall.

In some configurations, at least one surface of the housing of the cushion module, such as one or two surfaces, is not aligned with the assembly direction or extends substantially in a radial direction or a direction substantially perpendicular to the assembly direction. In some such configurations, the friction member is removable from the housing.

While various embodiments envisage embedding of a part of the housing of the cushion module within the elastomeric friction member, the elastomeric friction member may be otherwise coupled or provided to the housing. Further, any of the embodiments described herein may be modified such that the friction member is provided only on the one or more engaging surfaces that engage the frame. However, as described herein, there may be advantages to embedding surfaces other than the engaging surface, including all surfaces defined by the engaging structure.

### BRIEF DESCRIPTION OF THE DRAWINGS

Throughout the drawings, reference numbers can be reused to indicate general correspondence between reference elements. The drawings are provided to illustrate example embodiments described herein and are not intended to limit the scope of the disclosure.
Figure 1 is a side view of a patient interface comprising a frame and a cushion module shown separated from one another.
Figure 2 is a side view of the patient interface of Figure 1 with the frame and the cushion module assembled.
Figure 3 is an enlarged view of a portion of the patient interface of Figure 1 showing a friction coupling of the interface with the frame and the cushion module separated from one another and illustrating several length dimensions.
Figure 4 is an enlarged view of the portion of the patient interface of Figure 3 illustrating several thickness dimensions.
Figure 5 is an enlarged of the portion of the patient interface of Figure 3 with the frame and the cushion module assembled and illustrating several length dimensions.
Figure 6 illustrates an alternative friction coupling in which a relative angle is illustrated between a portion of the frame and a portion of the cushion module.
Figure 7 is an enlarged view of the friction coupling illustrating a retention force at several axial locations.
Figure 8 is a side view of another patient interface comprising a frame and a cushion module shown separated from one another.
Figure 9 is a side view of the patient interface of Figure 8 with the frame and the cushion module assembled.
Figure 10 is an enlarged view of a portion of the patient interface of Figure 8 showing a friction coupling of the interface with the frame and the cushion module separated from one another and illustrating several length dimensions.
Figure 11 is an enlarged view of the portion of the patient interface of Figure 10 illustrating several thickness dimensions.
Figure 12 is an enlarged of the portion of the patient interface of Figure 10 with the frame and the cushion module assembled and illustrating several length dimensions.
Figure 13 is a partial view of the friction coupling of the interface of Figures 1-7 with mold shut-off portions illustrated.
Figure 14 is a partial view of the friction coupling of the interface of Figures 8-12 with mold shut-off portions illustrated.
Figure 15 is a front perspective view of a patient interface comprising a frame and a cushion module coupled to one another by a friction coupling.
Figure 16 is a front perspective view of the cushion module of the patient interface of Figure 1 separate from the frame.
Figure 17 is a central, vertical cross-sectional view of the cushion module of Figure 16.
Figure 18 is a rear perspective view of the frame of the patient interface of Figure 1.
Figure 19 is a central, vertical cross-sectional view of the patient interface of Figure 1.
Figure 20 is an enlarged view of a portion of the cross-sectional view of Figure 19.
Figure 21 is a front perspective view of a patient interface including a frame, a cushion module and a conduit connector elbow, wherein the cushion module is removably coupled to the frame by a friction coupling.
Figure 22 is a front perspective view of the cushion module of Figure 21 separate from the frame and conduit connector elbow.
Figure 23 is a front view of the cushion module of Figure 22 with a friction coupling portion of the cushion module removed to expose an underlying support structure.
Figure 24 is a side view of the cushion module of Figure 23.
Figure 25 is a front view of the cushion module of Figure 22.
Figure 26 is a side view of the cushion module of Figure 22.
Figure 27 is a sectional view of the cushion module of Figure 22 taken along a vertical, central plane.
Figure 28 is a top view of the cushion module of Figure 22.
Figure 29 is a bottom view of the cushion module of Figure 22.
Figure 30 is an enlarged view of a portion of the cushion module indicated by the perimeter 30 in Figure 27 illustrating the friction coupling portion.
Figure 31 is an enlarged view of a portion of the cushion module indicated by the perimeter 31 in Figure 30 illustrating an upper portion of the friction coupling portion.
Figure 32 is an enlarged view of a portion of the cushion module indicated by the perimeter 32 in Figure 30 illustrating a lower portion of the friction coupling portion.
Figure 33 is a front perspective view of the frame of the patient interface of Figure 21 separate from the cushion module and the conduit connector elbow.
Figure 34 is a front view of the frame of Figure 33.
Figure 35 is a sectional view of the frame of Figure 33 taken along the line 35-35 in Figure 34.
Figure 36 is an enlarged view of a portion of the frame indicated by the perimeter 36 in Figure 35 illustrating a collar of the frame.
Figure 37 is a side view of the frame of Figure 33.
Figure 38 is a rear view of the frame of Figure 33.
Figure 39 is a rear perspective view of the frame of Figure 33.
Figure 40 is a sectional view of the frame similar to Figure 35 but including the conduit connector elbow.
Figure 41 is a front view of the patient interface of Figure 21.
Figure 42 is a sectional view of the patient interface taken along the line 42-42 in Figure 41.
Figure 43 is an enlarged view of a portion of the patient interface indicated by the perimeter 43 in Figure 42 illustrating an upper portion of the friction coupling.
Figure 44 is an enlarged view of a portion of the patient interface indicated by the perimeter 44 in Figure 43 illustrating the friction coupling.
Figure 45 is an enlarged view of a portion of the patient interface indicated by the perimeter 45 in Figure 42 illustrating a lower portion of the friction coupling.
Figure 46 is a front perspective view of a patient interface including a cushion module and a frame having an integrated a conduit connector, wherein the cushion module is removably coupled to the frame by a friction coupling.
Figure 47 is a front perspective view of the cushion module separate from the frame.
Figure 48 is a front view of the cushion module of Figure 47.
Figure 49 is a side view of the cushion module of Figure 47.
Figure 50 is a front view of the cushion module of Figure 47 with the friction coupling portion of the cushion module removed to expose underlying support structure.
Figure 51 is a front perspective view of the cushion module of Figure 50.
Figure 52 is a sectional view of the cushion module of Figure 47 taken along a vertical, central plane passing through the cushion module in a forward-rearward direction.
Figure 53 is a top view of the cushion module of Figure 47.
Figure 54 is a bottom view of the cushion module of Figure 47.
Figure 55 is a rear view of the cushion module of Figure 47.
Figure 56 is a front view of the cushion module of Figure 47, wherein the viewing axis is aligned with an axis of an inlet opening and/or the friction coupling portion of the cushion module.
Figure 57 is an enlarged view of the cushion module indicated by the perimeter 57 in Figure 52 illustrating an upper portion of the friction coupling portion.
Figure 58 is an enlarged view of the cushion module indicated by the perimeter 58 in Figure 52 illustrating a lower portion of the friction coupling portion.
Figure 59 is a front perspective of the frame separate from the cushion module.
Figure 60 is a front view of the frame of Figure 59.
Figure 61 is a side view of the frame of Figure 59.
Figure 62 is a sectional view of the frame of Figure 59 taken along a vertical, central plane passing through the frame in a forward-rearward direction.
Figure 63 is a rear view of the frame of Figure 59.
Figure 64 is a front view of the patient interface of Figure 46.
Figure 65 is a side view of the patient interface of Figure 46.
Figure 66 is a rear view of the patient interface of Figure 46.
Figure 67 is a sectional view of the patient interface of Figure 46 taken along a vertical, central plane passing through the patient interface in a forward-rearward direction.
Figure 68 is an enlarged view of a portion of the patient interface indicated by the perimeter 68 in Figure 67 illustrating an upper portion of the friction coupling portion.
Figure 69 is an enlarged view of a portion of the patient interface indicated by the perimeter 69 in Figure 67 illustrating a lower portion of the friction coupling portion.
Figure 70 is a schematic representation of a respiratory system configured to supply pressurized and humidified breathing gases to a user through a patient interface.
Figure 71 is a front perspective view of a patient interface comprising a frame and a cushion module coupled to one another by a friction coupling, with an elbow coupled to the interface.
Figure 72 is a front perspective view of the cushion module of Figure 71.
Figure 73 is a front perspective view of the cushion module of Figure 72 with a friction member deleted to show underlying structure.
Figure 74 is a front view of the cushion module of Figure 73.
Figure 75A is a side view of the cushion module of Figure 73.
Figure 75B is a side view of the cushion module of Figure 73, highlighting certain dimensions.
Figure 76 is a front view of the cushion module of Figure 72.
Figure 77 is a side view of the cushion module of Figure 72.
Figure 78 is a section view of the cushion module of Figure 72, taken along line 78 in Figure 76.
Figure 79 is a top view of the cushion module of Figure 72.
Figure 80 is a bottom view of the cushion module of Figure 72.
Figure 81 is a magnified section view of the region identified by box 81 in Figure 78.
Figure 82 is a magnified section view of the region identified by box 82 in Figure 81.
Figure 83 is a front view of the cushion module of Figure 72.
Figure 84 is a section view of the cushion module of Figure 72, taken along line 84 in Figure 83.
Figure 85 is a magnified section view of the region identified by box 85 in Figure 84.
Figure 86 is a front perspective view of the frame of Figure 71.
Figure 87 is a front view of the frame of Figure 86.
Figure 88 is a side view of the frame of Figure 86.
Figure 89 is a rear view of the frame of Figure 86.
Figure 90 is a section view of the frame of Figure 86, taken along line 90 in Figure 87.
Figure 91 is a magnified section view of the region identified by box 91 in Figure 90.
Figure 92 is a rear perspective view of the frame of Figure 71.
Figure 93 is a section view of the elbow and frame of Figure 71.
Figure 94 is a front view of the assembly of Figure 71.
Figure 95 is a section view of the assembly of Figure 71, taken along line 95 in Figure 94.
Figure 96 is a magnified section view of the region indicated by box 96 in Figure 95 with the elbow removed.
Figure 97 is a magnified section view of the region indicated by box 97 in Figure 95 with the elbow removed.
Figure 98 is a front view of the assembly of Figure 71.
Figure 99 is a section view of the assembly of Figure 71, taken along line 99 in Figure 98.
Figure 100 is a magnified section view of the region indicated by box 100 in Figure 99.
Figure 101 is a front perspective view of a patient interface comprising a frame and a cushion module coupled to one another by a friction coupling, with an elbow coupled to the interface.
Figure 102 is a front perspective view of the cushion module of Figure 101 with a friction member removed to show underlying structure.
Figure 103 is a front view of the cushion module of Figure 102.
Figure 104 is a side view of the cushion module of Figure 102.
Figure 105 is a front perspective view of the cushion module of Figure 101.
Figure 106 is a front view of the cushion module of Figure 101.
Figure 107 is a side view of the cushion module of Figure 101.
Figure 108 is a section view of the cushion module taken along line 108 in Figure 106.
Figure 109 is a top view of the cushion module of Figure 101.
Figure 110 is a bottom view of the cushion module of Figure 101.
Figure 111 is a magnified section view of the region indicated by box 111 in Figure 108.
Figure 112 is a magnified section view of the region indicated by box 112 in Figure 108.
Figure 113 is the magnified section view of Figure 112, indicating certain dimensions.
Figure 114 is the magnified section view of Figure 112, indicating certain dimensions.
Figure 115 is a front perspective view of the frame of Figure 101.
Figure 116 is a front view of the frame of Figure 115.
Figure 117 is a side view of the frame of Figure 115.
Figure 118 is a rear view of the frame of Figure 115.
Figure 119 is a section view of the frame of Figure 115, taken along line 119 in Figure 116.
Figure 120 is a magnified section view of the region indicated by box 120 in Figure 119.
Figure 121 is a front perspective view of a frame insert of the frame of Figure 115.
Figure 122 is a front view of the frame insert of Figure 121.
Figure 123 is a top view of the frame insert of Figure 121.
Figure 124 is a bottom view of the frame insert of Figure 121.
Figure 125 is a side view of the frame insert of Figure 121.
Figure 126 is a rear view of the frame insert of Figure 121.
Figure 127 is a rear perspective view of the frame insert of Figure 121.
Figure 128 is a section view of the frame insert of Figure 121, taken along line 128 in Figure 122.
Figure 129 is a front perspective view of the frame of the frame insert of Figure 121 assembled with the frame of Figure 115.
Figure 130 is a rear perspective view of the assembled frame of Figure 129.
Figure 131 is a front perspective view of the assembly of Figure 101.
Figure 132 is a front view of the assembly of Figure 131 with the elbow removed.
Figure 133 is a section view of the assembly of Figure 131, taken along line 133 in Figure 132.
Figure 134 is a magnified section view of the region indicated by box 134 in Figure 133.
Figure 135 is a magnified section view of the region indicated by box 135 in Figure 133.
Figure 136 is a front view of the assembly of Figure 132.
Figure 137 is a section view of the assembly of Figure 132, taken along line 137 in Figure 136.
Figure 138 is a magnified section view of the region indicated by box 138 in Figure 137.

### DETAILED DESCRIPTION

Embodiments of systems, components and methods of assembly and manufacture will now be described with reference to the accompanying figures, wherein like numerals refer to like or similar elements throughout. Although several embodiments, examples and illustrations are disclosed below, it will be understood by those of ordinary skill in the art that the disclosures described herein extends beyond the specifically disclosed embodiments, examples and illustrations, and can include other uses of the disclosures and obvious modifications and equivalents thereof. The terminology used in the description presented herein is not intended to be interpreted in any limited or restrictive manner simply because it is being used in conjunction with a detailed description of certain specific embodiments of the disclosures. In addition, embodiments of the disclosures can comprise several novel features and no single feature is solely responsible for its desirable attributes or is essential to practicing the disclosures herein described.

Certain terminology may be used in the following description for the purpose of reference only, and thus are not intended to be limiting. For example, terms such as "above" and "below" refer to directions in the drawings to which reference is made. Terms such as "front," "back," "left," "right," "rear," and "side" describe the orientation and/or location of portions of the components or elements within a consistent but arbitrary frame of reference which is made clear by reference to the text and the associated drawings describing the components or elements under discussion. In addition, the orientation and/or location of the assembly or portions thereof (e.g., components or elements) can be described with reference to the planes of the human body, in which the sagittal plane or median plane (longitudinal, anteroposterior) divides the body into left and right, the coronal plane or frontal plane (vertical) divides the body into dorsal and ventral (back and front, posterior and anterior) portions, and the transverse plane or axial plane (lateral, horizontal) divides the body into cranial and caudal (head and tail, upper and lower) portions. Such references to these planes assume the assembly is properly positioned for the intended use on a user. Moreover, terms such as "first," "second," "third," and so on may be used to describe separate components. Such terminology may include the words specifically mentioned above, derivatives thereof, and words of similar import.

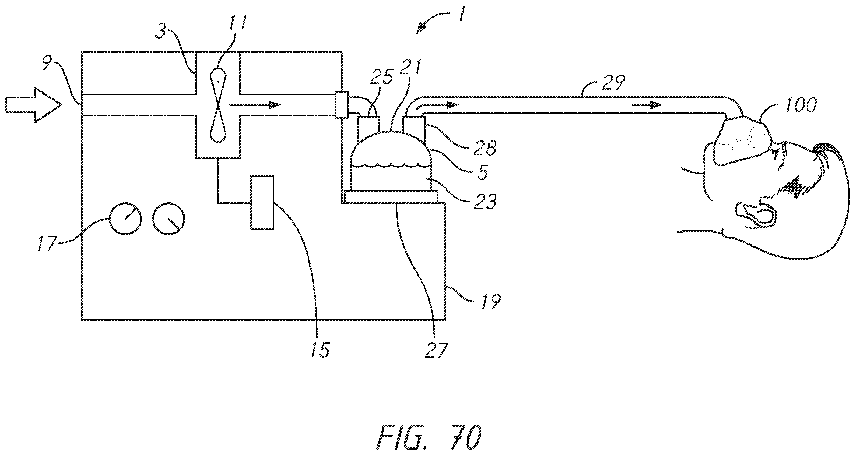

An example respiratory therapy system suitable for supplying breathing gases to a user for positive airway pressure (PAP) therapy (e.g., continuous positive airway pressure (CPAP) therapy) or non-invasive ventilation (NIV) therapy is illustrated in Figure 70. The example respiratory therapy system 1 may include a gas source 3, a humidifier 5, a patient interface 100 and a breathing gas circuit 29 that connects the humidifier (or gas source) to the patient interface 100. The gas source 3 can provide a supply of breathing gas to the humidifier 5. The gas source 3 may comprise a blower in which breathing gas, e.g., ambient air, is drawn into the gas source 3 through an inlet 9 in the gas source casing by an impeller 11. The rotational speed of the impeller 11 may be modulated to regulate the quantity of air drawn into the gas source 3 and the supply of breathing gas delivered to the respiratory therapy system 1. Breathing gas may include any single gas or multiple gases that are breathable by a user of the system 1.

The pressure and/or flow rate of breathing gas exiting the gas source 3 may be regulated by a controller 15. The controller 15 may modulate the rotational speed of the impeller 11 according to one or more predetermined algorithms and in accordance with one or more user inputs that may be provided via a user input 17.

The gas source 3 represents an actively controlled flow generator. Other gas sources, such as a compressed air cylinder with suitable pressure or flow regulation, may also be used to supply breathing gas. The outlet of the gas source 3 may be coupled to a separate humidifier 5. The humidifier 5 may be configured to heat and/or humidify the breathing gas prior to delivery, e.g., delivery to the user. In some embodiments, the humidifier is integrated with the gas supply. The humidifier 5 may include a base 19 and a humidifier chamber 21. The chamber 21 may be configured to hold humidification fluid 23, such as water, and may be disengaged, e.g., temporarily disengaged or permanently disengaged, from the humidifier base 19 to allow it to be filled or replaced. The humidifier 5 receives gases from the gas source 3 through chamber inlet 25. The humidifier base 19 can include a heater such as a heater plate 27. The chamber 21 rests on the heater plate 27 when engaged with the humidifier base 19. The heater plate 27 dissipates heat, e.g., heat generated by electrical resistance, to the chamber 21. The chamber 21 preferably has a heat conductive base to enable the heat generated by the heater plate 27 to pass efficiently to the humidification fluid 23. Controller 15 can also control the humidifier 5, and in particular the supply of electrical energy to the heater plate 27, to regulate any function of the humidifier 5, e.g., the temperature and humidity of the breathing gas supplied to the user.

The breathing gas can be supplied to the user via a chamber outlet 28 and the breathing gas circuit 29 in the form of a conduit which may incorporate a heating or warming element, e.g., a heater wire, to heat or warm (e.g., keep hot or warm) the breathing gases during transportation to the patient interface 7. The electrical energy supplied to the heater wire may be controlled by the controller 15. The controller 15 may receive feedback from one or more sensors incorporated in a control network throughout the respiratory therapy system to monitor properties of the breathing gas, such as, but not limited to, pressure, flow, temperature, and/or humidity.

The patient interface 100 couples the user with the respiratory therapy system 1, such that gases, e.g., heated and humidified gases from the humidifier 5, may be delivered to the user's respiratory system. Breathing gases can be delivered to the user at, or near, optimal temperature and humidity (e.g., warmed and fully saturated with water vapor at temperatures of between 27 and 37 °C) as the gases are delivered to the user's nares and or mouth. Emulating the conditions within healthy adult lungs (37 °C, 44 mg/L humidity) can help maintain healthy mucocilliary function in users with respiratory disorders affecting secretion and for all patients humidifying the gas helps maintain comfort and compliance. A number of different styles of patient interface 100, such as those disclosed herein, may be used in the example system 1 or a similar system.

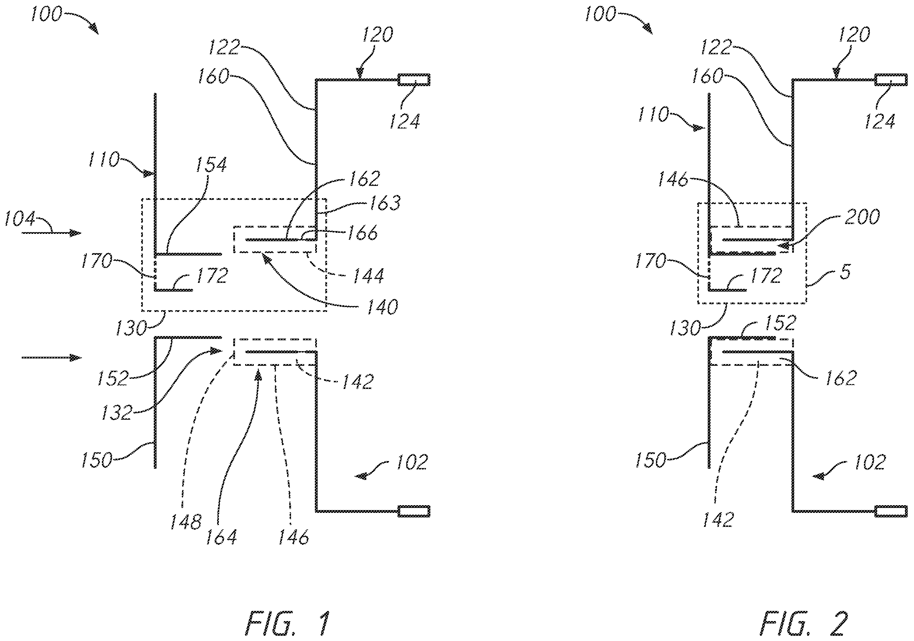

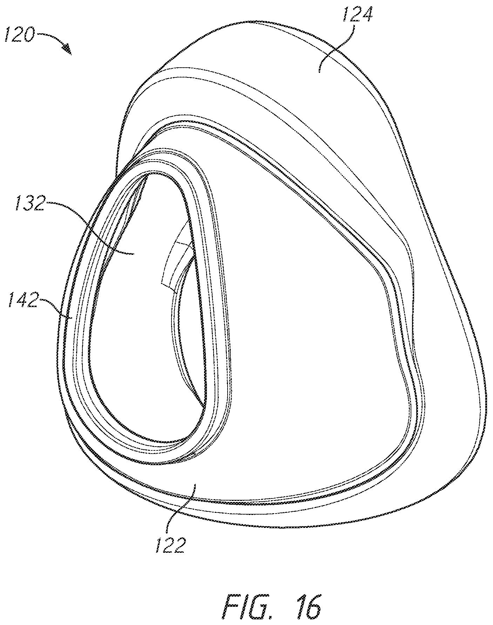

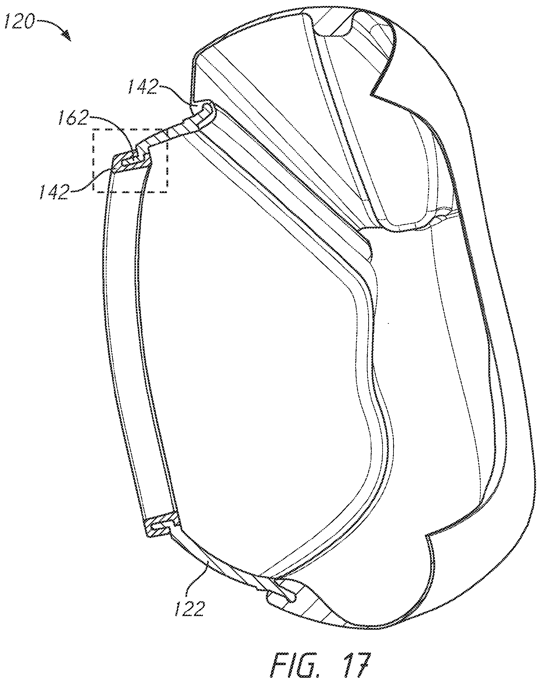

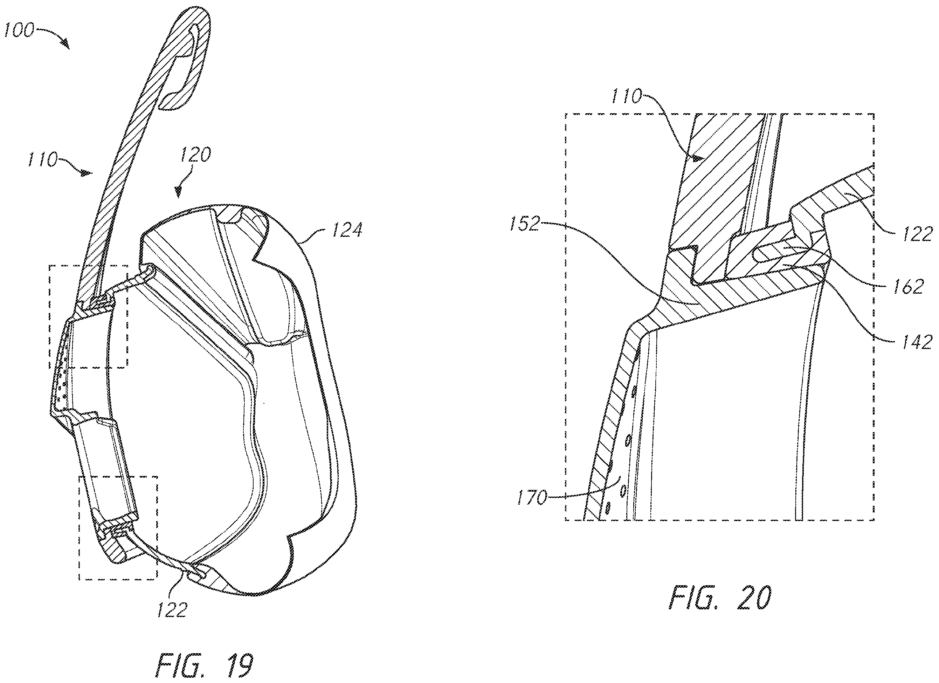

Figures 1, 2 and 15-20 illustrate a patient interface 100 configured for the delivery of respiratory therapy to a patient. The illustrated patient interface 100 is in the form of a mask, which is secured against the face of the user to define a substantially sealed breathing chamber 102. The respiratory therapy often involves the delivery of a flow of pressurized breathing gas (e.g., air) to the breathing chamber 102 of the patient interface 100 and, eventually, to the user's airways.

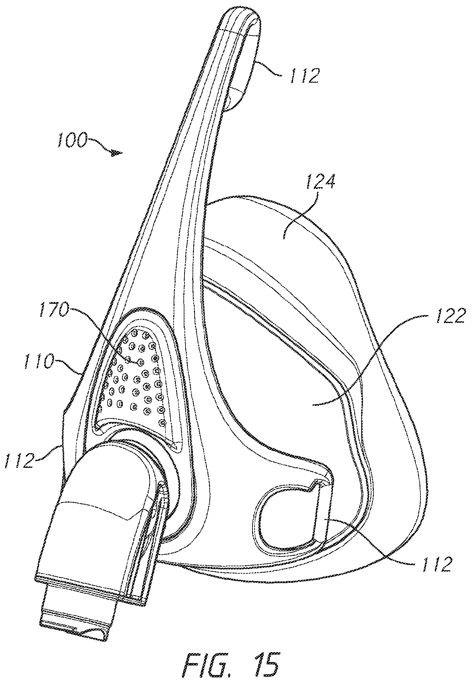

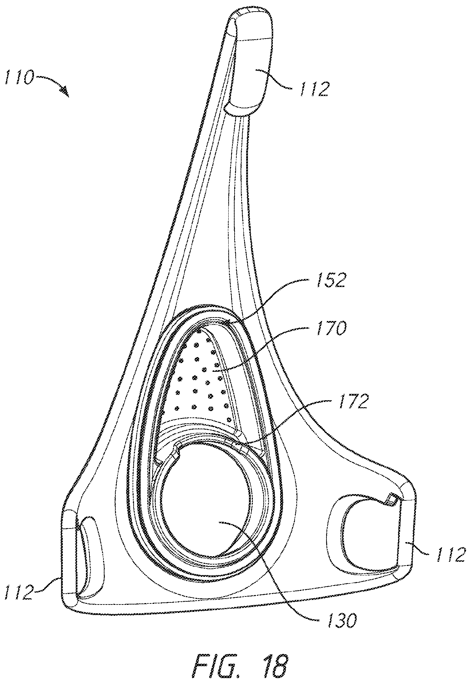

The illustrated patient interface 100 includes a frame 110 and a cushion module 120. In the illustrated arrangement, the cushion module 120 can be removably coupled to the frame 110. The frame 110 is configured to be connected to headgear (not shown), which holds the patient interface 100 in a sealed relation to the user's face. The frame 110 can include any suitable number or type of headgear mounts. In some configurations, the frame 110 includes two, three, four, five or more headgear mounts configured to receive two, three, four, five or more straps of a headgear or headgear assembly. In some configurations, a single headgear mount can be configured to receive more than one headgear strap. The mounts can be configured to directly or indirectly receive the headgear strap. For example, in some configurations, the headgear mount comprises an opening and/or a mounting post through which or around which the headgear strap is looped. In other configurations, the headgear strap is secured to the headgear mount by an intermediate structure, such as a headgear clip. The frame 110 can include a single type of mount (e.g., direct or indirect) or multiple types of mounts (e.g., both direct and indirect). For example, as illustrated in Figures 15 and 18, the frame 110 includes a single upper headgear mount 112, which is configured to receive a headgear clip that connects to two upper headgear straps. The frame 110 also includes two lower headgear mounts 112, with one mount located on each side of the frame 110.

The cushion module 120 defines at least a portion of the breathing chamber 102 of the patient interface 100. In the illustrated arrangement, the cushion module 120 includes a housing 122 and a cushion or seal 124. Preferably, the housing 122 includes a wall that extends in both a radial and axial direction relative to an axis 104 along which the frame 110 and the cushion module 120 overlap or are coupled. In some configurations, the axis 104 along which the frame 110 and the cushion module 120 overlap is generally or substantially aligned with a direction along which the frame 110 and the cushion module 120 are assembled. That is, in some arrangements, the frame 110 and the cushion module 120 are assembled by relative linear movement along the axis 104. However, in other arrangements, the frame 110 and/or the cushion module 120 can experience relative movement in directions other than along the axis 104. For example, assembly of the cushion module 120 to the frame 110 can employ a rocking motion, such that engagement between the frame 110 and the cushion module 120 occurs generally along the axis 104, but with relative movement in additional directions. For convenience, the axis 104 is referred to as the assembly axis or direction of assembly, which includes movement solely along the axis or movement along the axis with relative movement in additional direction, unless indicated otherwise. In some configurations, the assembly direction 104 or assembly axis can generally or substantially lie within the sagittal plane. Thus, the housing 122 defines a depth in a front-back or anteroposterior direction. As used herein, the terms "radial" and "axial" refer to directions relative to the assembly axis 104, which can be parallel to or coincident with an axis of a gas inlet opening of the frame 110 and/or the cushion module 120. The gas inlet opening axis is often referred to as the z-axis. Unless indicated otherwise, the terms "radial" and "axial" are not intended to refer to exact directions, but are used to distinguish between the two directions or to describe relative locations of two or more different locations.

The seal 124 is coupled to a rear or posterior portion of the housing 122. The seal 124 defines a face-contacting surface configured to contact the face of a user of the interface 100. Preferably, the face-contacting surface of the seal 124 creates a sufficient seal with the user's face such that the desired positive pressure can be maintained throughout the vast majority of the therapy session when properly fitted and absent extenuating circumstances. The seal 124 can be configured such that it surrounds either or both of the nose and mouth of the user. The seal 124 can be an inflation-type seal having an interior-concave curved wall that inflates when the interior of the interface 100 is pressurized to provide a good seal and better conform to the face of the particular user. However, other types of seals can be used, if desired. Moreover, certain features, aspects and advantages of the present disclosure can be utilized with other types of patient interfaces, such as direct nasal interfaces, among others.

In some configurations, the housing 122 is harder and/or more rigid than the seal 124. In any of the embodiments described herein, the housing 122 can be constructed from a material having a higher modulus of elasticity than the material of the seal 124. The material utilized to construct a portion or an entirety of the housing 122 can exhibit a greater rigidity or stiffness for a solid body of a given size and shape than a solid body of the same size and shape constructed from the material of the seal 124. Thus, a solid body constructed from the material of the seal 124 can have less rigidity or stiffness (or greater flexibility or compliance) than a solid body of the same size and shape constructed from the material of the housing 122. Unless indicated otherwise, descriptions of relative rigidity or stiffness of materials herein refer to differences in the modulus of elasticity between the materials or differences in rigidity or stiffness of solid bodies of the same size and shape constructed from the materials being compared. Descriptions of relative rigidity or stiffness of particular structures can refer to resistance to deformation of those structures, taking into account both material properties and physical characteristics of the structures (e.g., size, shape, wall thickness).

In some configurations, the housing 122 can be constructed from a relatively stiff or rigid plastic (e.g., polycarbonate). The seal 124 can be constructed from a relatively soft, flexible or pliable material, such as an elastomer (e.g., silicone). In some configurations, each of the housing 122 and the seal 124 are molded (e.g., injection molded). Thus, the housing 122 and the seal 124 can be constructed from moldable materials. In some configurations, the seal 124 is coupled to the housing 122 during a molding process, such as an over-molding process. In addition, or in the alternative, the seal 124 may be mechanically coupled to the housing 122. For example, the housing 122 can include a plurality of apertures through which the material of the seal 124 passes to create a mechanical interlock between the seal 124 and the housing 122.

As described above, the frame 110 defines or otherwise includes a gas inlet opening or gas inlet 130 that permits a flow of breathing gas to pass through the frame 110. In at least one configuration, the gas inlet 130 can be in the form of a gas inlet opening. The cushion module 120 defines or otherwise includes a connection opening 132 that permits a flow of breathing gas to pass through the cushion module 120. In at least one configuration, the connection opening 132 can be in the form of a gas inlet opening 132. In at least one configuration, the connection opening 132 can be in the form of a cushion module opening 132. When the cushion module 120 is assembled to the frame 110, the connection opening 132 of the cushion module 120 is aligned with the gas inlet 130 of the frame 110 such that a flow of breathing gas is permitted to pass through the gas inlet 130 of the frame 110 and the connection opening 132 of the cushion module 120 into the breathing chamber 102.

In some configurations, the patient interface 100 includes a friction coupling 140 that couples the cushion module 120 and the frame 110. In at least some configurations, the friction coupling 140 comprises overlapping structures that cooperate to create an interference fit or a friction fit between the structures. In some arrangements, the friction coupling 140 is the only coupling between the frame 110 and the cushion module 120. In some configurations, the friction force provided by the friction coupling 140 is the only retention force or the primary retention force that holds the cushion module 120 and the frame 110 together. That is, the friction coupling 140 can be the only direct coupling between the frame 110 and the cushion module 120. However, in other arrangements, the patient interface 100 can include additional couplings between the frame 110 and the cushion module 120. In some configurations the additional couplings do not provide any or any substantial coupling forces between the frame 110 and the cushion module 120, while in other configurations the additional couplings can provide substantial coupling forces between the frame 110 and the cushion module 120.

In the illustrated arrangement, the friction coupling 140 is or comprises an elastomeric friction member 142 that in addition to coupling the cushion module 120 to the frame 110 also creates an airtight or substantially airtight seal between the frame 110 and the cushion module 120. With such an arrangement, leakage of breathing gas (e.g., air) between the frame 110 and the cushion module 120 can be prevented or sufficiently inhibited such that the desired positive pressure can be maintained within the breathing chamber 102. The elastomeric friction member 142 can have a greater hardness than the seal 124. For example, the friction member 142 can have a 60-80 shore A hardness and the seal 124 can have a lower shore A hardness, such as about a 40 shore A hardness, for example. In some configurations, the elastomeric friction member 142 can have the same hardness as the seal 124 and/or can be constructed from the same material. For example, the elastomeric friction member 142 and the seal 124 can each have a 40 shore A hardness. In some configurations, the seal 124 can have a greater hardness than the elastomeric friction member 142. At least a surface of the friction member 142 that engages the frame 110 can have a textured surface finish, which can provide a desirable balance between retention and allowing sliding movement for assembly and disassembly. A very smooth finish of the elastomeric friction member 142 can inhibit sliding. Thus, a frosted or textured surface finish - or another finish that provides some reasonable degree of roughness - can provide a desirable compromise between retention and sliding.

The illustrated frame 110 includes at least a main wall or a front wall 150 and a collar 152, which can be in the form of a tubular wall that extends rearwardly (posteriorly or toward the user in an assembled and fitted in-use orientation) from the front wall 150 to a rearward or free end, edge or rim and surrounds the gas inlet 130. In at least one embodiment, the collar 152 can be in the form of an inlet collar 152. The perimeter defined by the collar 152 can be circular, ovate, oval, triangular or other polygonal shapes, or other combinations thereof, for example. For example, the perimeter defined by the collar 152 can be substantially triangular in shape with rounded corners. Further, the sides of the triangular shapes could be curved or straight. Non-circular shapes can advantageously rotationally fix the frame 110 and cushion module 120 about the assembly axis 104. Moreover, asymmetric shapes can inhibit or prevent the cushion module 120 from being assembled to the frame 110 in an upside-down or otherwise improper orientation. Advantageously, a tapered ovate or triangular shape, such as that illustrated in the interface 100 of Figures 15-20, provides for both non-rotation and proper alignment. The front wall 150 and the collar 152 can be unitarily formed of a material with sufficient strength and rigidity to maintain a breathing chamber 102 during use, such as a relatively rigid polymeric material (e.g., polycarbonate), by a process such as molding (e.g., injection molding).

The housing 122 of the illustrated cushion module 120 includes a main wall 160 and a support flange 162 that extends forwardly (anteriorly or away from the user in an assembled and fitted in-use orientation). The main wall 160 and/or the support flange 162 surrounds and/or defines the connection opening 132. The housing 122 also includes a support wall 163 that surrounds and is adjacent the connection opening 132 and/or the support flange 162. In the arrangement of Figures 1-7, the support wall 163 is a portion of the main wall 160 and connects the support flange 162 to a remainder of the main wall 160. However, the support wall 163 could be separate from the main wall 160, such as spaced forwardly or rearwardly from the main wall 160. The support flange 162 defines a perimeter shape that preferably corresponds to the perimeter shape of the collar 152, but preferably is somewhat larger to accommodate the portion of the friction member 142 therebetween. The support flange 162 of the housing 122 could extend in a rearward direction (toward the user) in addition or in the alternative of the forward-extending support flange 162.

In the illustrated arrangement, the elastomeric friction member 142 is carried by or coupled to the cushion module 120. More specifically, in the illustrated arrangement, the elastomeric friction member 142 is carried by or coupled to the housing 122. In particular, the friction member 142 is coupled to the support flange 162. However, in other arrangements, the friction member 142 could be instead carried by or coupled to the frame 110. In the illustrated arrangement, at least a portion of the main wall 160 is located external to the friction member 142 and extends rearwardly and outwardly from the friction member 142 toward or to the seal 124. Preferably, the main wall 160 provides sufficient strength and rigidity to maintain a breathing chamber 102 during use. The illustrated friction member 142 includes a peripheral surface 146. In at least one configuration, the peripheral surface 146 can be an outward-facing surface 146. In at least one configuration, the peripheral surface 146 can be an exterior surface 146. In the illustrated arrangement, the housing 122 of the cushion module 120 extends through the friction member 142 such that a portion of the housing 122 is embedded within the friction member 142. This embedded portion forms a mechanical anchor to secure the friction member 142 to the housing 122. In addition, the embedded portion can provide hoop strength to the friction member 142 or limit outward expansion of the friction member 142 to enhance the friction fit with the collar 152 of the frame 110. However, in other configurations, a portion of the housing 122 (e.g., the support flange 162) may not be embedded in the friction member 142, but can extend along a surface of the friction member 142 (e.g., along the peripheral surface 146). In some configurations, such an arrangement provides hoop strength to or limit the deformation of the friction member 142. In some arrangements, such as any of those described herein, the friction member 142 is not permanently fixed to the housing 122 and may be removable from the housing 122, such as for cleaning or replacement.

In the illustrated arrangement, the support flange 162 is disposed, at least in part, within the friction member 142 and the housing 122 extends through the peripheral surface 146 of the friction member 142. With such an arrangement, the support flange 162 reinforces the friction member 142 such that the retention force between the frame 110 and the cushion module 120 is determined by compression of the friction member 142 to a greater extent than similar arrangements without the support flange 162. In addition, such an arrangement can facilitate or allow for the friction coupling 140 to have a single overlap engagement, in which a surface of the friction member 142 on an opposite side of the engaging surface is exposed - in contrast to an arrangement in which the friction member 142 is received within a channel and engaged on both sides. A single overlap engagement can be easier to assemble than arrangements in which an elastomer seal is received within a channel, which can be difficult or impossible to engage the entire seal at once and typically require sections of the seal to be engaged sequentially.

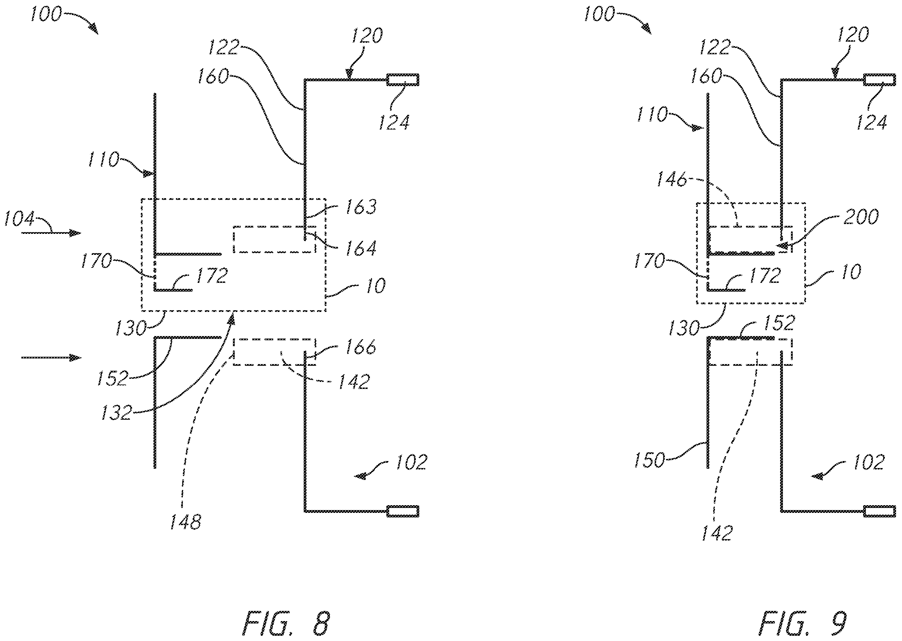

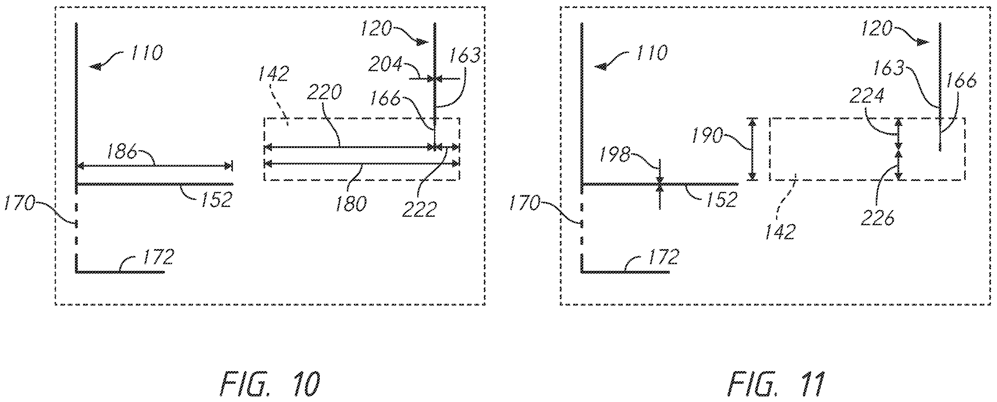

In the illustrated arrangement, the housing 122 extends into the friction member 142 in a direction defining a non-zero angle relative to the peripheral surface 146 of the friction member 142 and/or to an axis of the friction member 142, which can be coincident with the assembly axis 104. In the illustrated arrangement, a rearward end of the friction member 142 is flush or substantially flush with a rearward surface of the housing 122 adjacent the friction member 142. Thus, the housing 122 extends through a rearward end portion of the friction member 142. However, in other arrangements, the friction member 142 can extend rearwardly of the rearward surface of the housing 122 adjacent the friction member 142, as illustrated and described in connection with Figures 8-12. In such an arrangement, the housing 122 extends through a portion of the friction member 142 spaced from the rearward end, as well as the forward end.

The friction member 142 engages the insert collar 152 to couple the cushion module 120 to the frame 110 in a sealed or substantially sealed manner. In the illustrated arrangement, the friction member 142 comprises an engagement surface 144 that engages an engagement surface 154 of the collar 152. In at least one configuration, the engagement surface 144 is an interior or radially inward-facing surface of the friction member 142. In at least one configuration, the engagement surface 154 is an exterior or radially outward-facing surface of the collar 152. However, in other arrangements, the friction member 142 could engage an interior surface of the collar 152. Preferably, with either arrangement the frame 110 defines at least a portion of the breathing chamber 102. For example, in the illustrated configuration, an interior space defined by the collar 152 defines a portion of the breathing chamber 102. As described above, in the illustrated arrangement, the peripheral surface 146 is exposed or is not directly covered when the cushion module 120 is assembled to the frame 110. With such an arrangement, the friction member 142 is compressed from a single surface (e.g., the engagement surface 144). Accordingly, dimensional variations as a result of the manufacturing process will not impact the assembly and removal force to as great an extent as a design in which both the interior and exterior surfaces of the friction member 142 engage a surface of a cooperating structure. Moreover, the single collar 152 arrangement is easier to clean than an arrangement having a channel, which can be difficult to access by the user.

In the illustrated arrangement, the friction member 142 defines an abutment surface 148 that abuts or contacts the front wall 150 of the frame 110 when the cushion module 120 is coupled to the frame 110. The abutment surface 148 is a forward-facing surface of the friction member 142 and is configured to contact a portion of the rearward-facing surface of the front wall 150 of the frame 110 surrounding and/or adjacent the collar 152. Contact of the abutment surface 148 with the frame 110 can provide feedback to the user that the assembly of the cushion module 120 to the frame 110 is sufficient or complete. Although contact of the engagement surfaces 144 and 154 in most cases will be sufficient to create a reliable seal, in some cases or in some designs, contact of the abutment surface 148 with the frame 110 can also contribute to creating or maintaining a seal between the cushion module 120 and the frame 110. Contact between the friction member 142 and the frame 110 can be advantageous for at least the reasons described above; however, such a feature is not essential and can be omitted if desired. If desired, other feedback arrangements can be provided to give user tactile, auditory, visual or other feedback of complete engagement between the cushion module 120 and the frame 110.

In some configurations, the friction member 142 is permanently coupled to the housing 122. The friction member 142 can be permanently coupled to the main wall 160 and/or the support flange 162. For example, the friction member 142 can be coupled to the support flange 162 by an over-molding process. In some configurations, the housing 122 comprises one or more apertures 166 through which the material of the friction member 142 extends as a result of the over-molding process. In the illustrated arrangement, at least a portion of the apertures 166 are located in the support flange 162. In other arrangements, a portion or an entirety of the apertures 166 are located in a portion of the main wall 160 surrounding and relatively adjacent to or at the connection opening 132. Such an arrangement provides a mechanical interlock between the friction member 142 and the support flange 162 and/or the main wall 160 of the housing 122. In the illustrated arrangement, the support flange 162 includes the plurality of apertures 166, each of which passes radially through the support flange 162. In some configurations, the apertures 166 are located closer to the main wall 160 of the housing 122 than a free end of the support flange 162. In some configurations, the apertures 166 are evenly distributed around the perimeter of the support flange 162. In other configurations, the apertures 166 are unevenly distributed around the perimeter of the support flange 162. Although the apertures 166 are shown solely on the support flange 162, in some configurations, apertures 166 could be provided on the main wall 160 as well.

In the illustrated arrangement, the patient interface 100 comprises a vent 170 in fluid communication with the breathing chamber 102. The vent 170 is configured to allow a flow of breathing gases and/or gases exhaled by the user to pass from the breathing chamber 102 to the atmosphere external of the patient interface 100. In some configurations, the vent 170 is a bias flow vent configured to maintain a desired or sufficient positive pressure with the breathing chamber 102 in use. The vent 170 can comprise a plurality of small openings.

In some configurations, the vent 170 is carried by or located in the frame 110. However, in other configurations, the vent can be located on the cushion module 120 (e.g., the housing 122) or another component of the patient interface 100. In the illustrated arrangement, the vent 170 is located within the perimeter of the collar 152. In particular, the vent 170 is located in the front wall 150 of the frame 110.

In some configurations, the frame 110 includes a divider wall 172 between the vent 170 and the gas inlet 130. The divider wall 172 can be positioned between and/or separate the vent 170 from the gas inlet 130. In some configurations, the divider wall 172 extends from one side to the other side of the collar 152. In such an arrangement, the ends of the divider wall 172 can be connected to the interior surface of the collar 152. However, in other arrangements, the divider wall 172 is interrupted or spans only a portion of the distance from one side to the other side of the collar 152. In some configurations, a forward edge of the divider wall 172 can be connected to the front wall 150 of the frame 110. In the illustrated arrangement, a portion or an entirety of a rearward edge of the divider wall 172 is located forward of or closer to the front wall 150 of the frame 110 relative to a rearward edge or rim of a portion or an entirety of the collar 152. An upper surface or the surface of the divider wall 172 adjacent the vent 170 can extend generally in an anteroposterior direction or in a direction of gas flow through the vent 170. In some configurations, a portion or an entirety of the upper surface of the divider wall 172 can be angled relative to the anteroposterior direction or the direction of gas flow through the vent 170, but preferably does not substantially or completely overlap the vent 170 in the direction of gas flow through the vent 170. In some configurations, a space defined by an interior surface of the collar 152, an upper surface of the dividing wall 172 and a rearwardly-facing surface of a vent wall of the vent 170 forms at least a portion of the breathing chamber 102.

The vent 170, the collar 152 and the divider wall 172 can be constructed in any suitable manner. For example, any one or more of the vent 170, the collar 152 and the divider wall 172 can be constructed in a unitary manner with another portion of the frame 110 (e.g., the front wall 150). In other arrangements, any one or more of the vent 170, the collar 152 and the divider wall 172 can be formed as a separate structure and coupled to another portion of the frame 110 (e.g., the front wall 150). For example, the vent 170 can be formed as a separate structure and coupled to a portion or the remainder of the frame 110. In some configurations, the vent 170, divider wall 172 and collar 152 are formed as a unitary structure and coupled to a portion or the remainder of the frame 110. Such separate structures can be coupled to a portion or the remainder of the frame 110 by any suitable method or arrangement, such as by adhesives, over-molding, welding (e.g., ultrasonic welding), mechanical fasteners or snap-fits.

In the illustrated arrangement, the frame 110 defines a socket configured to receive a conduit connector, such as an elbow, configured to connect a breathing circuit to the patient interface 100. However, in other arrangements, the conduit connector can be connected to other portions or components of the patient interface 100, such as a portion of the cushion module 120 (e.g., the housing 122). In some configurations, the collar 152 defines at least a portion of the elbow socket. In the illustrated arrangement, the divider wall 172 and the collar 152 cooperate to define a portion or an entirety of the elbow socket. In some configurations, the conduit connector or elbow is capable of pivoting about one or more axes relative to the frame 110. For example, the conduit connector or elbow can have a swivel connection with the frame 110.

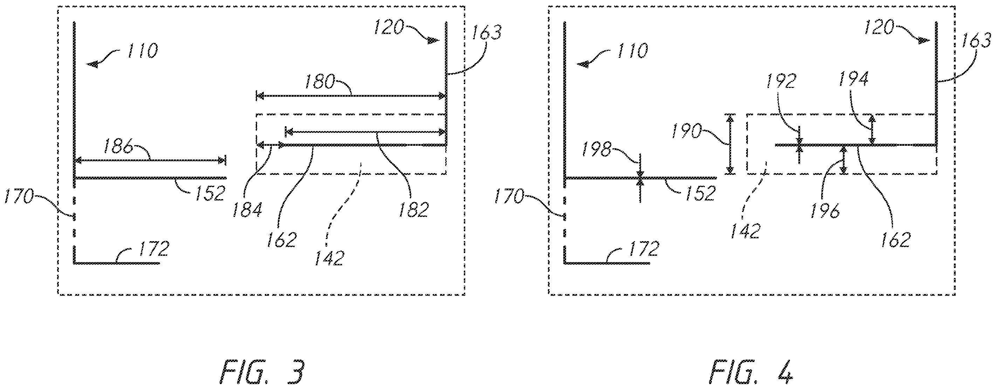

The friction coupling 140 is configured to provide any one or number of desirable characteristics related to the assembly and disassembly of the cushion module 120 to the frame 100 or use of the assembly. Such characteristics can include, for example and without limitation: a desired magnitude or range of assembly force, a desired magnitude or range of disassembly force, relatively consistent assembly or disassembly forces amongst different components (e.g., so that the forces will not vary drastically from what the user is accustomed to when using a replacement cushion module 120), relatively smooth assembly or disassembly, feedback upon complete assembly, reliable seal between the cushion module 120 and the frame 110. Any one or combination of these characteristics, or other advantages, can be provided or facilitated by the selection of suitable dimensions or proportions of the different structures of the friction coupling 140. Examples of such dimensions or proportions, and related advantages, are provided below with reference to Figures 3-5.

In the illustrated arrangement, the friction member 142 defines a first length 180 that extends in or substantially in an axial direction or the direction of assembly 104. The support flange 162 defines a second length 182 that extends in or substantially in an axial direction or the direction of assembly 104. An unsupported portion of the friction member 142 that extends beyond a free end of the support flange 162 defines a third length 184 that extends in or substantially in an axial direction or the direction of assembly 104. The collar 152 defines a fourth length 186 that extends in or substantially in an axial direction or the direction of assembly 104.

In some configurations, the first length 180 is equal to the sum of the second length 182 and the third length 184. With such an arrangement, the elastomeric friction member 142 is compact since the entire support flange 162 is embedded in the elastomeric friction member 142. Accordingly, the arrangement makes efficient use of available space in at least an axial direction. As used herein, the term "equal" or "substantially equal" means that the identified dimensions are equivalent within measurement error using common or reasonable measurement techniques and/or within normal or reasonable manufacturing tolerances.

However, in other configurations, the first length 180 is greater than the sum of the second length 182 and the third length 184 or the first length 180 is less than the sum of the second length 182 and the third length 184. These situations can occur if the end or the abutment surface 148 of the elastomeric friction member 142 is angled or oriented at a non-perpendicular angle relative to the direction of assembly 104 and depending on the radial location at which the lengths are measured. These situations can also occur if the end of the support flange 162 nearest the main wall 160 terminates within or beyond the corresponding end of the elastomeric friction member 142. Advantages of these configurations can include allowing different geometries of the cushion module 120 and/or frame 110. For example, by having a longer support flange 162 than what is necessary or desirable strictly to support the friction member 142, more space can be created for a user's nose within the breathing chamber 102. In other configurations, the first length 180 is less than the second length 182. In such configurations, the elastomeric friction member 142 spans only a portion of the support flange 162. As such, a portion of the support flange 162 is embedded within the elastomeric friction member 142, and a portion of the support flange 162 is exposed outside of the elastomeric friction member 142 (i.e. not embedded within the elastomeric friction member 142).

In the illustrated arrangement, the friction member 142 defines a first thickness 190 that extends in or substantially in a radial direction or perpendicular to the direction of assembly 104. The support flange 162 defines a second thickness 192 that extends in or substantially in a radial direction or perpendicular to the direction of assembly 104. A portion of the friction member 142 located radially outside of the support flange 162 defines a third thickness 194 that extends in or substantially in a radial direction or perpendicular to the direction of assembly 104. A portion of the friction member 142 located radially inside of the support flange 162 defines a fourth thickness 196 that extends in or substantially in a radial direction or perpendicular to the direction of assembly 104. The collar 152 defines a fifth thickness 198 that extends in or substantially in a radial direction or perpendicular to the direction of assembly 104.

In some configurations, the first thickness 190 equals the sum of the second thickness 192, the third thickness 194, and the fourth thickness 196. In such an arrangement, there are no hollow spaces within the friction member 142 and no additional layers of material. Such an arrangement is compact in an axial direction.

In some configurations, the third thickness 194 and the fourth thickness 196 are equal. Such an arrangement provides uniform compression behavior on each side of the friction member 142 in configurations in which each of the engagement surface 144 and the peripheral surface 146 are engaged by a cooperating component of the patient interface 100.

In some configurations, the second thickness 192 is equal to one or both of the third thickness 194 and the fourth thickness 196. When accomplished by a relatively thicker support flange 162, such an arrangement can provide improved strength of the support flange 162 in comparison to thinner support flanges 162. Advantageously, in comparison to thinner support flanges 162, such an arrangement can reduce flex of the support flange 162 such that the application force of the friction fit is determined solely or to a greater extent by compression of the friction member 142 rather than flex of the support flange 162.

In some configurations, the fourth thickness 196 is greater than or equal to the third thickness 194. Advantageously, a larger fourth thickness 196 improves consistency of the removal force between the frame 110 and cushion module 120 for different components in comparison to smaller thicknesses, wherein differences between different components can be the result of normal manufacturing tolerances. With a larger fourth thickness 196, the compression distance as a proportion of the total thickness for a given retention force is reduced. Accordingly, normal variations due to manufacturing tolerances will cause less variation of the retention, removal or application force in comparison to smaller thicknesses. In other configurations, the fourth thickness 196 is less than or equal to the third thickness 194. Such an arrangement can reduce overall material use for the friction member 142 in comparison to greater thicknesses.

In some configurations, the second thickness 192 and the fifth thickness 198 are equal. With such an arrangement, a relatively similar support strength is provided by a similarly thick collar 152 and support flange 162. However, in other configurations, the second thickness 192 is greater than the fifth thickness 198. With such an arrangement, deformation of the support flange 162 is minimized. In yet other configurations, the second thickness 192 is less than the fifth thickness 198. With such an arrangement, deformation of the collar 152 is minimized or at least reduced relative to a thinner collar 152. This minimizes or reduces the effect of the deformation of the collar 152 on the rotation of the elbow in the elbow socket, which in some arrangements is at least partially formed by the collar 152.

In some configurations, the second thickness 192 is less than or equal to either one of the third thickness 194 and the fourth thickness 196. Such an arrangement allows for reduced material of the support flange 162 and, thus, the housing 122 of the cushion module 120 relative to arrangements having a thicker support flange 162. However, in other configurations, the second thickness 192 is greater than or equal to either one of the third thickness 194 and the fourth thickness 196. Such an arrangement provides for increased strength of the support flange 162 in comparison to arrangements having a thinner support flange 162.

In some configurations, the first thickness 190 is greater than or equal to the fifth thickness 198. Such an arrangement allows for reduced material on the frame 110 in comparison to configurations having a thicker collar 152. Such an arrangement also provides a sufficiently thick friction member 142 to allow for a sufficient deformation of the friction member 142. However, in other configurations, the first thickness 190 is less than or equal to the fifth thickness 198. Such an arrangement allows for reduced material on the friction member 142 in comparison to configurations having a thicker friction member 142.

In some configurations, the fourth thickness 196 varies along the length of the friction member 142 or in the assembly direction 104. Such an arrangement provides for an angled or non-parallel orientation of the engagement surface 144 of the friction member 142 and the engagement surface 154 of the collar 152 and a variation in the retention force along the length of the friction coupling 140. In some configurations, the fourth thickness 196 varies along the length of the friction member 142 from a minimum near a forward or free end of the friction member 142 to a maximum near a rearward end of the friction member 142 nearest the main wall 160 of the cushion module 120. In some configurations, the angled or non-parallel orientation can provide a lead-in or tapered connection for the assembly of the friction member 142 and the collar 152. Such an arrangement can facilitate assembly by easing the initial alignment of the collar 152 and the friction member 142 and increasing the assembly or retention force as the overlap increases between the collar 152 and the friction member 142. In other configurations, the fourth thickness 196 varies along the length of the friction member 142 from a maximum near the forward or free end of the friction member 142 to a minimum near the rearward end of the friction member 142 nearest the main wall 160. The engagement surface 144 of the friction member 142 can be non-linear, such that the fourth thickness 196 increases and decreases along the length of the friction member 142. In some configurations, the fourth thickness 196 can be larger at one or more locations (e.g., regions or sections) intermediate the forward and rearward ends relative to a thickness at one or both of the forward and rearward ends of the friction member 142 or relative to a thickness between those locations. In some configurations, the fourth thickness 196 can be smaller at one or more locations (e.g., regions or sections) intermediate the forward and rearward ends relative to a thickness at one or both of the forward and rearward ends of the friction member 142 or relative to a thickness between those locations. For example, in some configurations, the engagement surface 144 can define one or more annular or part-annular retention beads or protrusions and/or one or more annular or part-annular relief channels or notches along the length of the friction member 142, which can result in variations in the fourth thickness 196.

In some configurations, no portion of the friction member 142 is located radially outward of the support flange 162 such that there is no third thickness 194 (i.e., the third thickness 194 is zero). Such an arrangement provides for reduced material on the friction member 142 relative to configurations in which a portion of the friction member 142 is located radially outward of the support flange 162. In some such arrangements, the friction member 142 may be chemically bonded by use of a self-adhesive elastomer, an adhesive or bonding agent, or pre-treatment process to enhance the bond with the support flange 162.

In some configurations, a portion of the friction member 142 that overlaps with the collar 152 defines an overlap length 200. In some configurations, the overlap length 200 is greater than or equal to the third length 184. Such an arrangement allows the support flange 162 to support the friction member 142 as it deforms to enhance the grip of the friction member 142 on the collar 152. In some configurations, the overlap length 200 is greater than or equal to a sum of the second length 182 and the third length 184. Such an arrangement allows for increased or maximum grip of the friction member 142 on the collar152.

In other configurations, the overlap length 200 is less than the third length 184. In such an arrangement, since the thickness of the friction member 142 that can deform in the portion defining the third length 184 is greater than in the portion defining the fourth thickness 196, more reliable grip or retention forces are able to be maintained between different cushion modules 120 and frames 110, which may vary due to normal manufacturing tolerances. In some configurations, the overlap length 200 is less than a sum of the second length 182 and the third length 184. Such an arrangement allows for efficient use of space.

In some configurations, the overlap length 200 equals the fourth length 186. In such an arrangement, the entire collar 152 is utilized for engagement with the friction member 142. Such an arrangement provides for efficient use of space and material. In other configurations, the overlap length 200 is less than the fourth length 186. In yet other configurations, the overlap length 200 is greater than the fourth length 186. Such an arrangement is possible where the collar 152 has, for example, different lengths around the perimeter and the fourth length 186 is taken as a shorter length that is less than the first length 180 of the friction member 142. Such an arrangement provides for varying amounts of overlap around the perimeter of the collar 152.

In some configurations, a portion of the friction member 142 that does not overlap with the collar 152 defines a non-overlap length 202 and wherein a portion (e.g., the support wall 163) of the main wall 160 adjacent the support flange 162 defines a support wall thickness 204, which can also be referred to as a first main wall thickness 204. In some configurations, a sum of the first length 180 and the first main wall thickness 204 equals a sum of the overlap length 200 and the non-overlap length 202. With such an arrangement, the full length of the friction member 142 is utilized for gripping the collar 152 without excess material or space being used. However, in other configurations, a sum of the first length 180 and the first main wall thickness 204 is greater than a sum of the overlap length 200 and the non-overlap length 202. With such an arrangement, the frame 110 is spaced further away from the face of the user. In yet other configurations, a sum of the first length 180 and the first main wall thickness 204 is less than a sum of the overlap length 200 and the non-overlap length 202. Such an arrangement provides for a reduced amount of material required for the friction member 142 in comparison to configurations having a longer friction member 142.

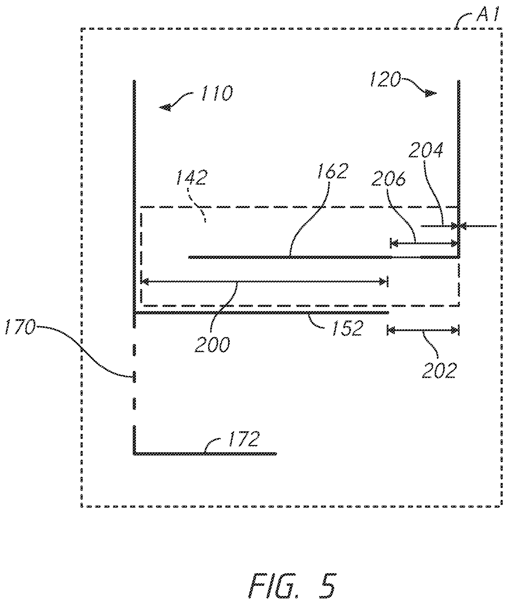

With reference to Figure 5, in some configurations, a distance between the portion of the main wall 160 surrounding and/or adjacent the connection opening 132 and a point on the plurality of apertures 166 furthest from that portion of the main wall 160 defines an aperture offset distance 206. In some configurations, the non-overlap length 202 is greater than or equal to a sum of the aperture offset distance 206 and the first main wall thickness 204. With such an arrangement, the rearward or free end of the collar 152 is located forward of the forward or closest edge or point of the apertures 166. That is, the collar 152 is shorter than when the non-overlap length 202 is smaller or non-existent. A shorter or smaller collar 152 allows for less material and smaller parts in comparison to longer or larger collars 152. In other configurations, the non-overlap length 202 is less than or equal to a sum of the aperture offset distance 206 and the first main wall thickness 204. Such an arrangement allows for a stronger connection between the collar 152 and the friction member 142 with a longer overlap length 200 (shorter non-overlap length 202).

In some configurations, a sum of the second length 182 and the third length 184 is greater than or equal to a sum of the overlap length 200 and the aperture offset distance 206. With such an arrangement, a longitudinal gap is provided between the apertures 166 and the free end of the collar 152. The presence of such a longitudinal gap can provide for or facilitate more flex of the friction member 142 in a cantilever or cantilever-like manner, which may be beneficial in certain embodiments. In other configurations, a sum of the second length 182 and the third length 184 is less than or equal to a sum of the overlap length 200 and the aperture offset distance 166. With such an arrangement, a stronger connection can be made between the collar 152 and the friction member 142 with a relatively longer overlap length 200 in comparison to configurations having a smaller or shorter overlap length 200.

In some configurations, a sum of the overlap length 200 and the aperture offset distance 206 is less than or equal to first length 180. With such an arrangement, a longitudinal gap is provided between the apertures 166 and the free end of the collar 152. The presence of such a longitudinal gap can provide for or facilitate more flex of the friction member 142, as described above. However, in other configurations, a sum of the overlap length 200 and the aperture offset distance 206 is greater than or equal to the first length 180. Such an arrangement can be accomplished by providing a collar 152 having a sufficient length to overlap in a longitudinal direction with the apertures 166. Such an arrangement provides for more secure engagement between the collar 152 and the friction member 142 and, in some embodiments, may facilitate or provide for better direction of a flow of breathing gases airflow into the breathing chamber 102.

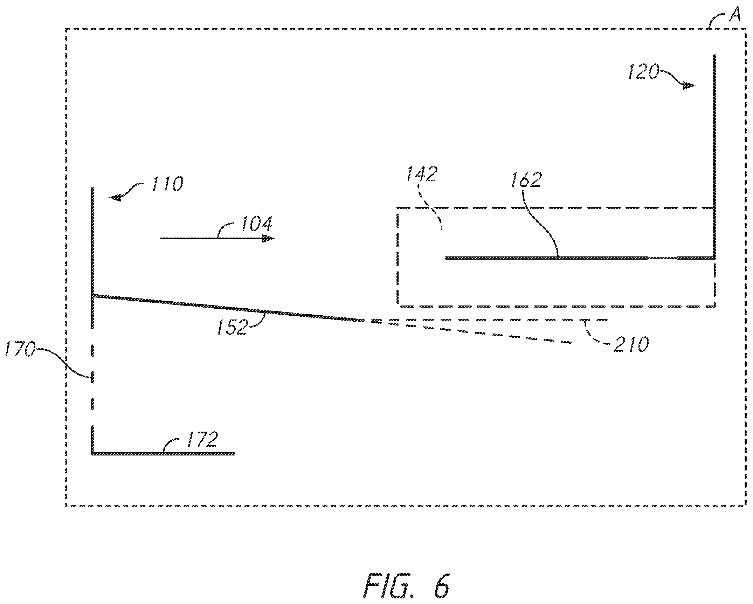

With reference to Figure 6, in some configurations, one or both of the friction member 142 and the collar 152 are configured such that a relative angle 210 is defined between the engagement surface 144 of the friction member 142 and the engagement surface 154 of the collar 152. Such an arrangement can be created by, for example, providing a tapered collar 152 and/or a tapered friction member 142 or support flange 162 in a longitudinal direction or along the assembly direction 104. In some configurations, the collar 152 is angled relative to the direction of assembly 104. In some configurations, at least one of the support flange 162 and the engagement surface 144 of the friction member 142 is angled relative to the direction of assembly 104. Such arrangements allow for a variable assembly, removal or retention force along the length of the collar 152 to be controlled between the collar 152 and the friction member 142. Such an arrangement can also or alternatively allow for simpler tooling by providing a draft angle in the mold tool to facilitate removal of the molded part.

With the configurations described above, the friction coupling 140 can be selectively configured to provide desired assembly, removal or retention force characteristics. For example, the friction coupling 140 can be configured such that a force acting to compress the friction member 142 is equal along a length of the collar 152. Such arrangements can provide for a more predictable deformation of the friction member 142 (or support flange 162 and/or collar 152). In other configurations, the friction coupling 140 can be configured such that a force acting to compress the friction member 142 is unequal along a length of the collar 152. Such arrangements can provide for a variable force along the length of the collar 152 to be controlled between the collar 152 and the friction member 142.

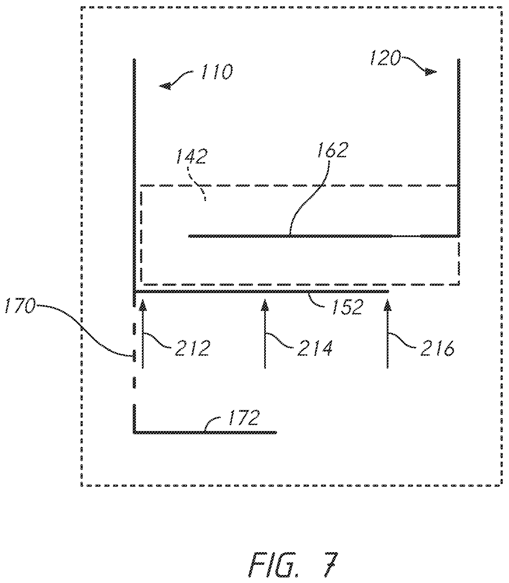

For the sake of comparison, Figure 7 illustrates a retention force or force acting to compress the friction member 142 at three different locations on the collar 152. A first retention force 212 is located at or near an end of the collar 152 closest to the front wall 150 of the frame 110. A second retention force 214 is located at an intermediate position on the collar 152. A third retention force 216 is located at or near a free end of the collar 152. In some configurations, more 'grip' or a larger retention force is provided in certain areas of the friction coupling 140, such as close to the front wall 150 of the frame 110. That is, in such configurations, the first retention force 212 can be greater than either or both of the second retention force 214 and the third retention force 216. Such an arrangement can reduce deformation of the collar 152 by having a relatively higher retention force at or nearer the front wall 150 where the collar 152 is supported and a relatively lower retention force at or nearer the free end where the collar 152 is unsupported by another structure. In other configurations, the retention force is configured to be smaller at a location closer to the front wall 150 relative to a location further from the front wall 150. That is, in such arrangements, the first retention force 212 can be less than either or both of the second retention force 214 and the third retention force 216. Such an arrangement can result, for example, from having a chamfer or other shaping of the forward end of the friction member 142 to avoid interference with the transition between the collar 152 and the front wall 150.

In some configurations, a rearward-most extent of the friction member 142 is at or forward of a portion of the main wall 160 surrounding and/or adjacent to the connection opening 132. Such an arrangement avoids the friction member 142 extending into the breathing chamber 102 thereby providing more room and/or avoiding interference with the user's nose. In other configurations, a rearward-most extent of the friction member 142 is rearward of the main wall 160 such that a portion of the friction member 142 is located along the main wall 160 in a longitudinal direction. That is, a portion of the friction member 142 is located adjacent each of a forward surface and a rearward surface of the main wall 160. Such configurations can include a friction member 142 that projects rearwardly from the main wall 160 and into the breathing chamber 102. With such an arrangement, the frame 110 can be brought closer to the face of the user while provide for the same length of overlap of the friction member 142 and the collar 152, thereby reducing the profile of the patient interface 100. In such arrangements, the friction member 142 may occupy a portion of the breathing chamber 102. Accordingly, such arrangements may be preferable in circumstances in which the location of the connection opening 132 is positioned below the user's nose or in another location in which contact with the user's face will be avoided.

Figures 8-12 illustrate another patient interface 100 that is similar to the interface 100 of Figures 1-7 with the notable exception that the friction member 142 is unsupported along a substantial portion of its length. In other respects, the patient interface 100 of Figures 8-12 is substantially similar to the patient interface 100 of Figures 1-7. Accordingly, the same reference numbers are used to refer to the same or similar features or components between the two interfaces 100. Any features or components of the interface 1 00 of Figures 8-12 that are not described in detail can be the same as or similar to the corresponding feature or component of the interface 100 of Figures 1-7, or can be of another suitable arrangement.

With reference to Figures 8-12, in some configurations, an embedded portion of the cushion module 120 is defined by a portion of the housing 122 located within the friction member 142. In the illustrated arrangement, the embedded portion is defined by a portion of the main wall 160, or a support wall 163, located radially outward of an opening rim 164, which surrounds and defines the connection opening 132. In some configurations, the embedded portion 163 extends substantially in a radial direction or a direction substantially perpendicular to the assembly direction 104 of the cushion module 120 to the frame 110. Such an arrangement provides at least one surface of the housing 122 (e.g., a surface of the support wall 163) that is not aligned with the assembly direction 104 or extends substantially in a radial direction or a direction substantially perpendicular to the assembly direction 104. For example, such an arrangement can provide two surfaces - a front and a rear surface - of the housing 122 that extends substantially in a radial direction or a direction substantially perpendicular to the assembly direction 104. Such an arrangement can apply a force to the friction member 142 in one or both directions (assembly and disassembly) along the assembly direction 104 without tending to separate the friction member 142 from the housing 122. Such an arrangement can be beneficial in embodiments in which the friction member 142 is removable from the housing 122, as described above. In other configurations, the embedded portion can extend in both an axial and a radial direction (or have both an axial and radial component). In some configurations, the embedded portion comprises forward and/or rearward-extending support flange portions, which can provide a mechanical interlock between the housing 122 and the friction member 142. However, preferably, the embedded portion does not extend along a substantial length of the friction member 142 in contrast to the arrangement of Figures 1-7.

In some configurations, the friction member 142 extends in both forward and rearward directions from the portion of the housing 122 to which the friction member 142 is coupled. In the illustrated arrangement, the friction member 142 has a portion that extends in a forward direction from a forward-facing surface of the main wall 160 and a portion that extends in a rearward direction from a rearward-facing surface of the main wall 160. In some configurations, the portion of the friction member 142 adjacent the forward surface of the main wall 160 has a surface (e.g., a radially-outward-facing surface) that is aligned in a radial direction with a surface (e.g., a radially-outward-facing surface) of the portion of the friction member 142 adjacent the rearward surface of the main wall 160.

In the illustrated arrangement, the collar 152 defines a collar length 186, which corresponds to the fourth length 186 of the interface 100 of Figures 1-7. A portion of the housing 122 embedded within the friction member 142 (e.g., the support wall 163 and/or the opening rim 164) defines a thickness 204, which is referred to herein for convenience as a support wall thickness 204. The support wall thickness 204 corresponds to the first main wall thickness 204 of the interface of Figures 1-7. The friction member 142 defines a total friction member length 180, which corresponds to the first length 180 of the interface 100 of Figures 1-7. The friction member 142 also defines a forward length 220 forward of the opening rim 164/support wall 163 and a rearward length 222 rearward of the opening rim 164/support wall 163.

In some configurations, the total friction member length 180 is equal to a sum of the forward length 220, the rearward length 222 and the support wall thickness 204. However, in other configurations, the total friction member length 180 is greater than or less than a sum of the forward length 220, the rearward length 222 and the support wall thickness 204. Such situations can result from, for example, the friction member 142 having angled front and/or rear end surfaces or front and/or rear end surfaces that are irregular in shape or otherwise not normal to the length direction and the length measurements are taken at a portion of the friction member 142 that has a greater or lesser length than the average length.

In the illustrated arrangement, the collar 152 defines a collar thickness 198, which corresponds to the fifth thickness 198 of the interface 100 of Figures 1-7. The friction member 142 defines a total thickness 190, which corresponds to the first thickness 190 of the interface 100 of Figures 1-7. A portion of the friction member 142 that extends along the embedded portion of the housing 122 (the opening rim 164 and/or the support wall 163) defines a supported thickness 224 and a portion of the friction member 142 that extends inwardly of the embedded portion of the housing 122 (the opening rim 164 and/or the support wall 163) defines an unsupported thickness 226.

In some configurations, the total thickness 190 equals a sum of the supported thickness 224 and the unsupported thickness 226. In some configurations, the total thickness 190 equals twice the supported thickness 224. Such an arrangement provides or allows for space for the apertures 166 on the embedded portion of the housing 122 through which the friction member 142 can project while also providing a sufficiently large unsupported thickness 226 to allow for a desired amount of deformation of the friction member 142 when the friction member 142 is connected to the collar 152. In some configurations, the total thickness 190 equals twice the unsupported thickness 226. Such an arrangement allows for a sufficiently large unsupported thickness 226 to allow for a desired amount of deformation of the friction member 142 when the friction member 142 is connected to the collar 152.

In some configurations, the supported thickness 224 is equal to the unsupported thickness 226. Such an arrangement can provide a balance between support or engagement between the housing 122 and the friction member 142 and allowing for deformation of the friction member 142 when the cushion module 120 is assembled to the frame 110. In some configurations, these characteristics are equally desirable and an equal balance between the two can be desirable. However, in other configurations, the supported thickness 224 is greater than or equal to the unsupported thickness 226. Such an arrangement can allow for or facilitate a stronger connection of the friction member 142 to the housing 122 as a result of an increased area of engagement and/or using larger apertures 166 to provide for a mechanical interlock between the friction member 142 and the housing 122. In yet other configurations, the supported thickness 224 is less than or equal to the unsupported thickness 226. Such an arrangement can allow for reduced material use and/or a smaller profile of the friction coupling 140 or entire interface 100 for a given size of gas inlet 130.

In some configurations, the total thickness 190 is greater than or equal to the collar thickness 198. Such an arrangement allows for more substantial compression of the friction member 142 and/or a more flex of the collar 152 to provide an increased retention force and/or to facilitate assembly. However, in other configurations, the total thickness 190 is less than or equal to the collar thickness 198. Such an arrangement allows for reduced material use on the friction member 142, greater expansion of the friction member 142 for ease of assembly or reduced flex of the collar 152.

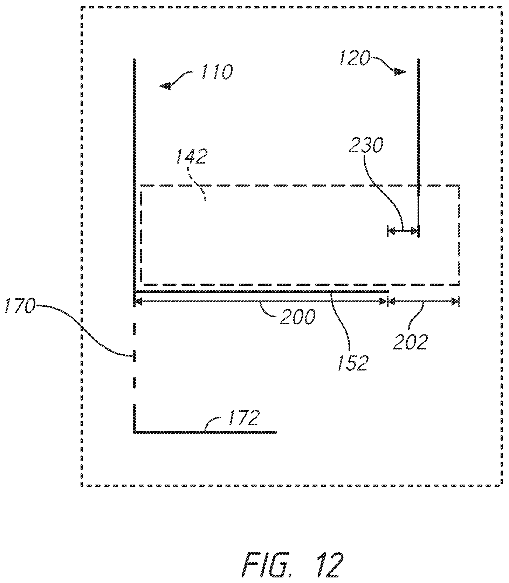

As shown in Figure 12, and as described with respect to the interface 100 of Figures 1-7, a portion of the friction member 142 that overlaps with the collar 152 defines an overlap length 200. A portion of the friction member 142 that does not overlap with the collar 152 defines a non-overlap length 202. A distance between a rearward end of the collar 152 and the embedded portion of the housing 122 (the opening rim 164 and/or the support wall 163) along the direction of assembly 104 defines a support wall offset distance 230. In some configurations, the overlap length 200 is greater than or equal to the support wall offset distance 230. Such an arrangement provides more surface area for engagement between the collar 152 and the friction member 142. However, in other configurations, the overlap length 200 is less than the support wall offset distance 230. Such an arrangement allows for deformation of a larger length of the friction member 142 in comparison to configurations with a larger overlap length 200 and/or a smaller support wall offset distance 230.

In some configurations, the overlap length 200 is greater than or equal to the non-overlap length 202. Such an arrangement provides more surface area for engagement between the collar 152 and the friction member 142. However, in other configurations, the overlap length 200 is less than the non-overlap length 202. Such an arrangement allows for deformation of a larger length of the friction member 142 in comparison to configurations with a larger overlap length 200 and/or a smaller support wall offset distance 230.

In some configurations, the non-overlap length 202 is greater than or equal to a sum of the support wall offset distance 230 and the support wall thickness 204. Such an arrangement can result from the friction member 142 extending rearwardly of the embedded portion of the housing 122, which can provide for a stronger and more durable connection between the friction member 142 and the housing 122. However, in other configurations, the non-overlap length 202 is less than or equal to a sum of the support wall offset distance 230 and the support wall thickness 204. Such an arrangement can allow for the use of a reduced amount of material for the friction member 142.

In some configurations, the overlap length 200 equals the collar length 186. Such an arrangement allows for an efficient use of the length of the collar 152. However, in other configurations, the overlap length 200 is less than the collar length 152. Such an arrangement allows for the possibility of reduced material for the friction member 142 assuming the friction member 142 is made shorter than the collar 152. In yet other configurations, the overlap length 200 is greater than the collar length 186. Such an arrangement is possible where the collar 152 has, for example, different lengths around the perimeter and the collar length 186 is taken as a shorter length than the maximum length of the collar 152 that overlaps with the friction member 142. Such an arrangement provides for varying amounts of overlap around the perimeter of the collar 152.

In some configurations, the total friction member length 180 equals a sum of the overlap length 200 and the non-overlap length 202. In some configurations, the overlap length 200 is less than the forward length 220. Such an arrangement can allow for more cantilever flex of the friction member 142. In other configurations, the overlap length 200 is greater than or equal to the forward length 220. Such an arrangement can allow for a stronger grip between the friction member 142 and the collar 152. In some configurations, the overlap length 200 is greater than or equal to a sum of the forward length 220, the rearward length 222 and the support wall thickness 204. Such an arrangement can allow for a stronger grip between the friction member 142 and the collar 152. In other configurations, the overlap length 200 is less than or equal to a sum of the forward length 220, the rearward length 222 and the support wall thickness 204. Such an arrangement can allow for more cantilever flex of the friction member 142.

In some configurations, the support wall thickness 204 is less than or equal to the rearward length 222. Such an arrangement can allow for more flex or deformation of the friction member 142. In other configurations, the support wall thickness 204 is greater than the rearward length 222. Such an arrangement can allow for a more rigid friction member 142, if desired. Such an arrangement can also allow for a stronger connection between the elastomeric friction member 142 and the housing 122.

The housing 122 of the interface 100 of Figures 8-12 can include apertures 166 through which the material of the friction member 142 extends. Such an arrangement provides for a mechanical interlock between the friction member 142 and the housing 122. In some configurations, at least a portion of the apertures 166 are located between the opening rim 164 and the peripheral surface 146.

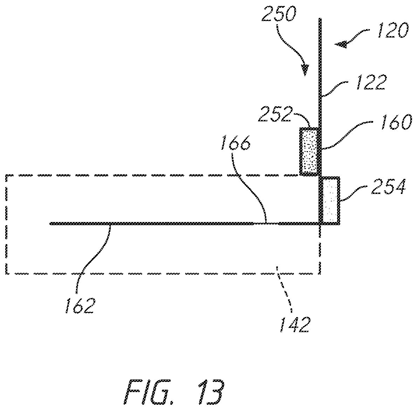

Figure 13 illustrates a portion of the cushion module 120 of Figures 1-7 that includes the friction member 142 and a shut-off arrangement 250 of a molding tool for molding the friction member 142 by an over-molding process. In the illustrated arrangement, a first shut-off portion 252 of a first mold tool portion is located radially outward of and adjacent to the friction member 142. A second shut-off portion 254 of a second mold tool portion is located radially outward of and adjacent to the connection opening 132 or support wall 163. The first shut-off portion 252 and the second shut-off portion 254 are configured to receive the main wall 160 of the housing 122 of the cushion module 120 between them. The first shut-off portion 252 and the second shut-off portion 254 are configured to pinch against the main wall 160 of the housing 122 to provide a physical barrier that retains the injection molded silicone within the mold tool at the surface transitions between the friction member 142 and the housing 122.

As illustrated, a rearward end of the friction member 142 is flush or substantially flush with an interior surface of the main wall 160. To achieve this arrangement, the first shut-off portion 252 and the second shut-off portion 254 are offset from one another in a radial direction. As a result, mold closing forces acting on the shut-off portions 252, 254 are not directly opposed to each other, but are also offset in a radial direction.

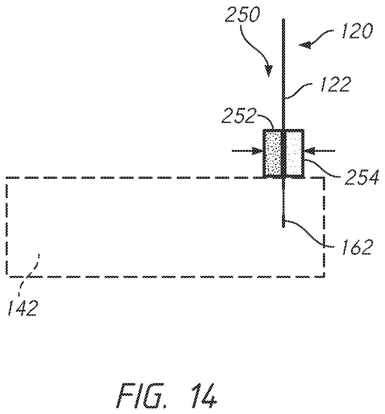

Figure 14 illustrates a portion of the cushion module 120 of Figures 8-12 that includes the friction member 142 and a shut-off arrangement 250 of a molding tool for molding the friction member 142 by an over-molding process. The shut-off arrangement 250 of Figure 14 can be the same as or similar to the shut-off arrangement 250 of Figure 13 except as described below. In the illustrated arrangement, a first shut-off portion 252 of a first mold tool portion is located radially outward of and adjacent to the friction member 142 on an exterior side of the main wall 160. A second shut-off portion 254 of a second mold tool portion is located radially outward of and adjacent to the friction member 142 on an interior side of the main wall 160.

As illustrated, a rearward end of the friction member 142 extends rearwardly of an interior surface of the main wall 160 toward the seal 124 (the seal 124 is not shown in Figure 14 - see, for example, Figure 8) of the cushion module 120. In the illustrated arrangement, a radially outer surface of the friction member 142 on an exterior side of the main wall 160 is aligned in a radial direction with a radially outer surface of the friction member 142 on an interior side of the main wall 160. With such an arrangement, the first shut-off portion 252 and the second shut-off portion 254. are aligned with or oppose one another in a radial direction. As a result, mold closing forces acting on the shut-off portions 252, 254 are directly opposed to each other in a radial direction. Such an arrangement may reduce flexing of the housing 122 when the mold is closed relative to the arrangement of Figure 13, which could improve sealing of the mold portions against the housing 122 and reduce flashing of the friction member 142 material.

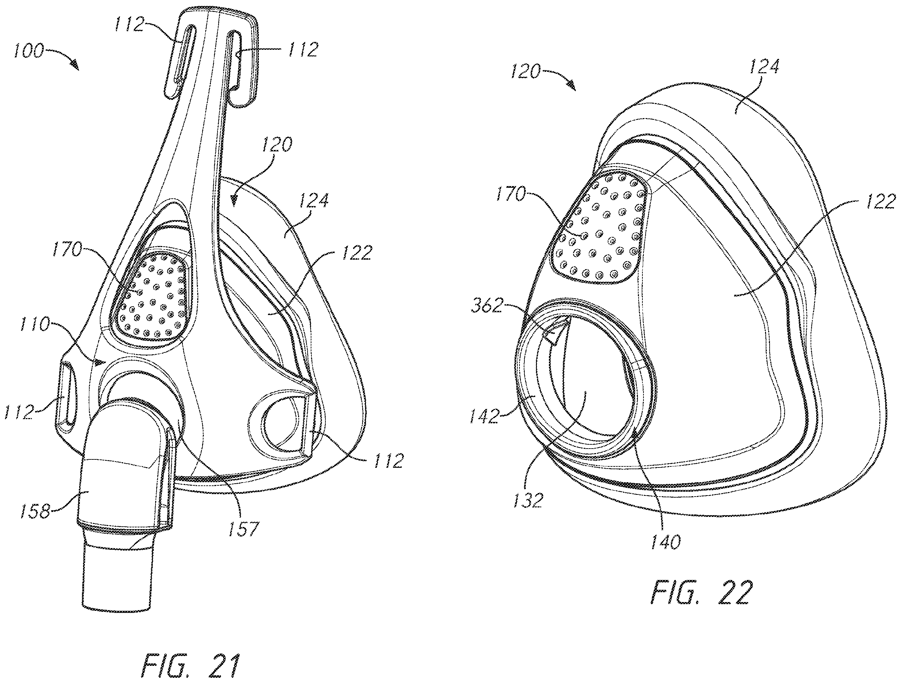

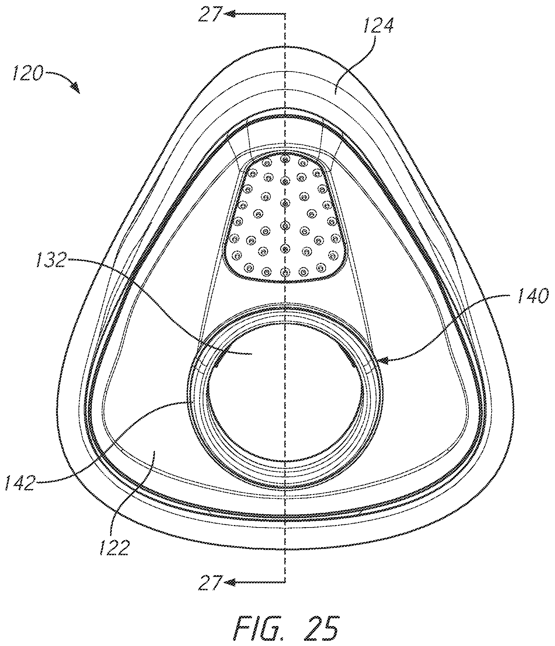

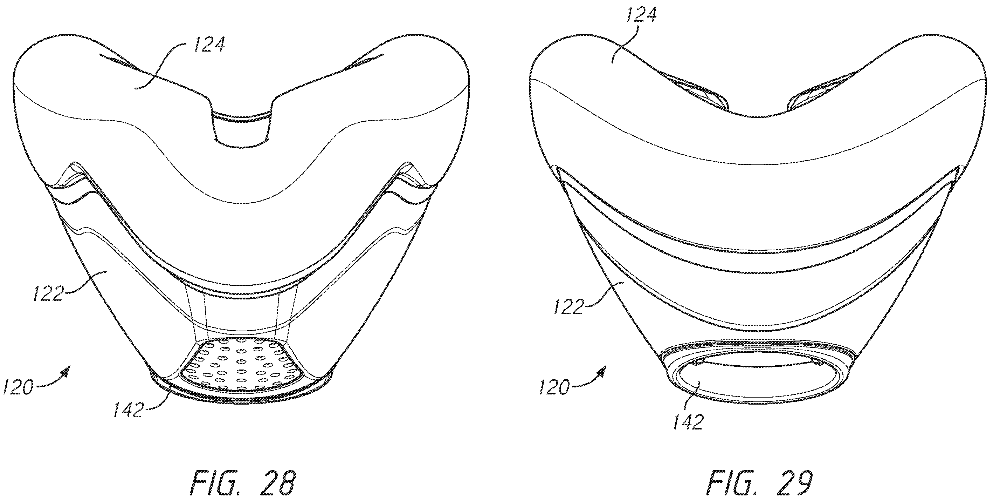

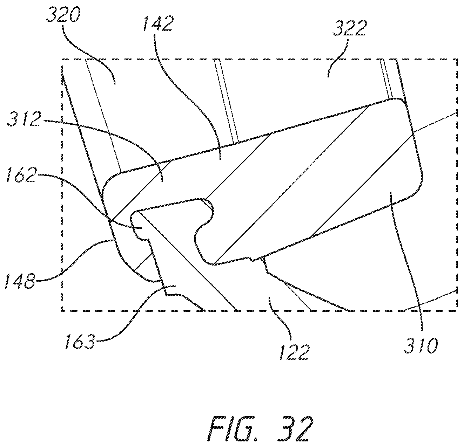

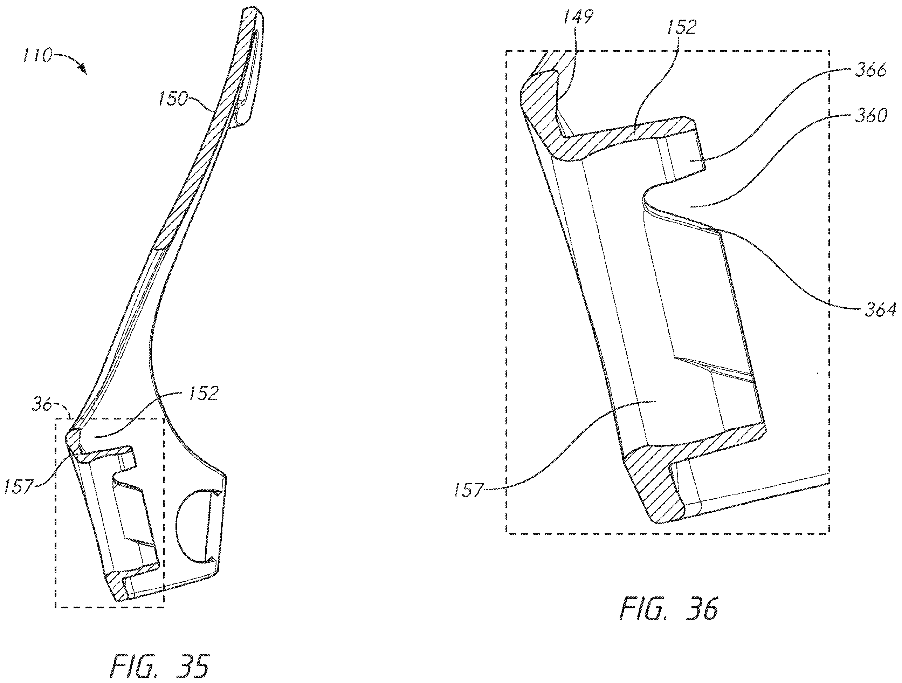

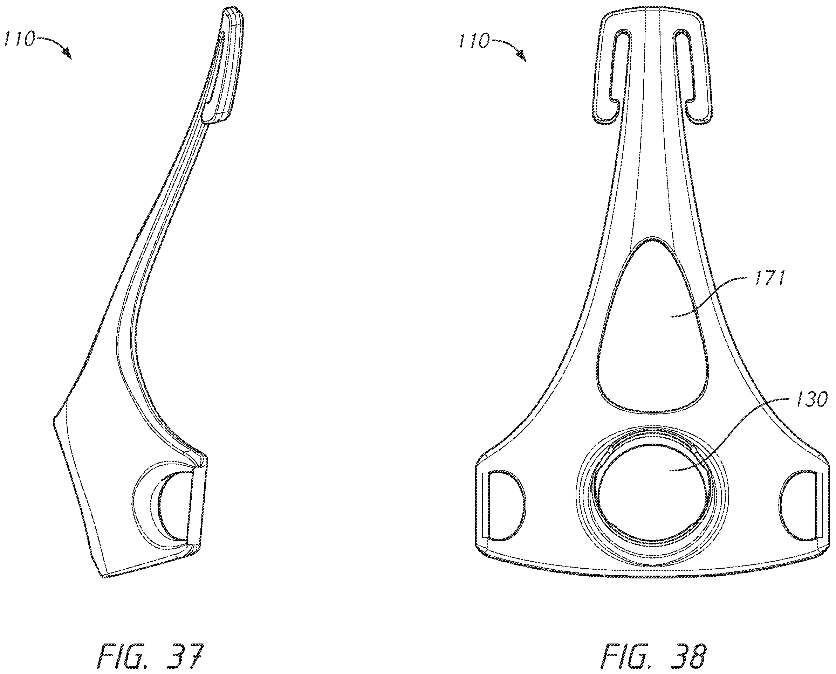

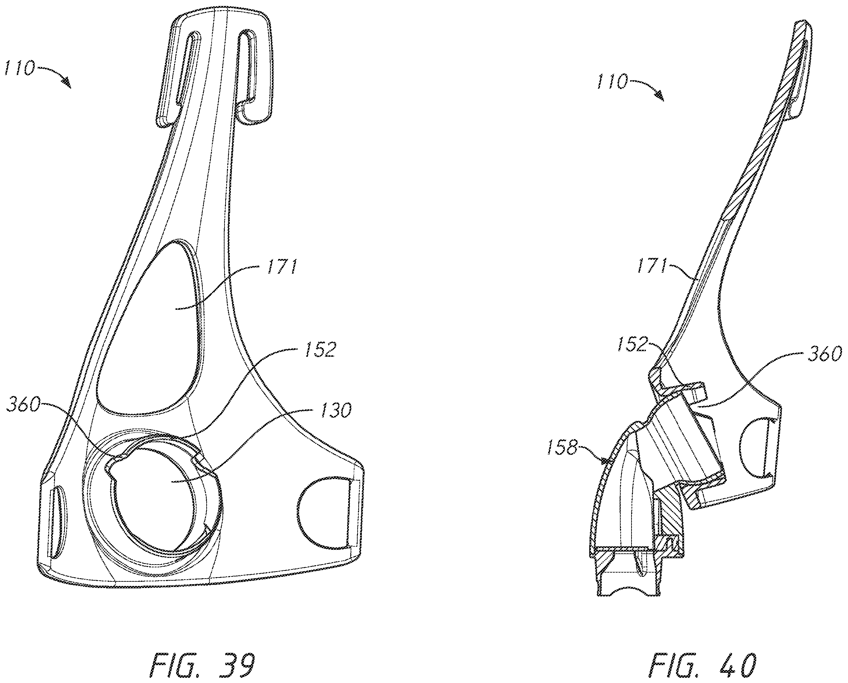

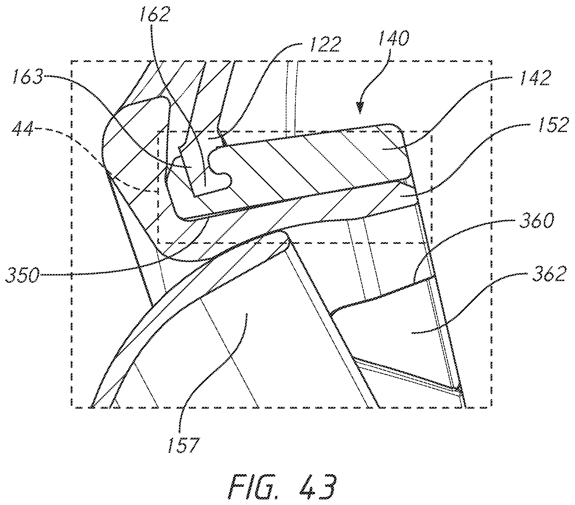

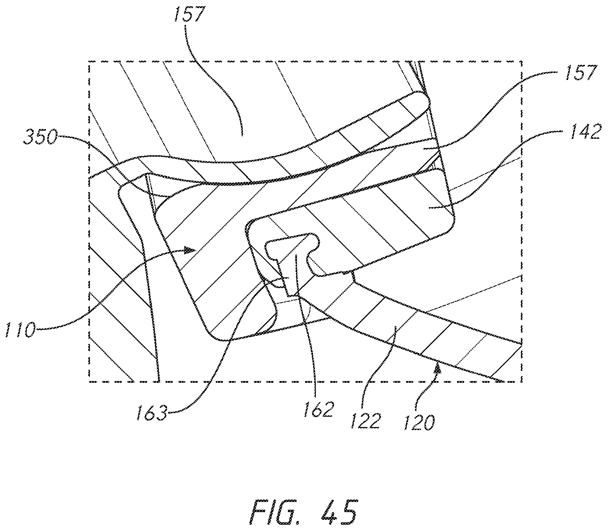

The patient interface 100 and portions thereof shown in Figures 21-45 is similar in many respects to the other interfaces disclosed herein. Accordingly, the same reference characters can be used to refer to the same or similar components or features and the following description is focused on the unique features of the interface 100 relative to the other interfaces. Accordingly, the interface 100 can incorporate features of the other interfaces disclosed herein. In addition, features that are not disclosed in detail can be the same as or similar to other interfaces disclosed herein, or can be of another suitable arrangement.

The patient interface 100 includes a frame 110 and a cushion module 120 removably coupled to the frame 110. The frame 110 is configured to be connected to headgear (not shown), which holds the patient interface 100 in a sealed relation to the user's face. The illustrated frame 110 includes a pair of upper headgear mounts 112 and a pair of lower headgear mounts 112. However, the frame 110 could include other desirable number of headgear mounts depending on the type of headgear desired to be used with the patient interface 100. The mounts 112 can be configured to directly or indirectly receive an associated headgear strap(s).

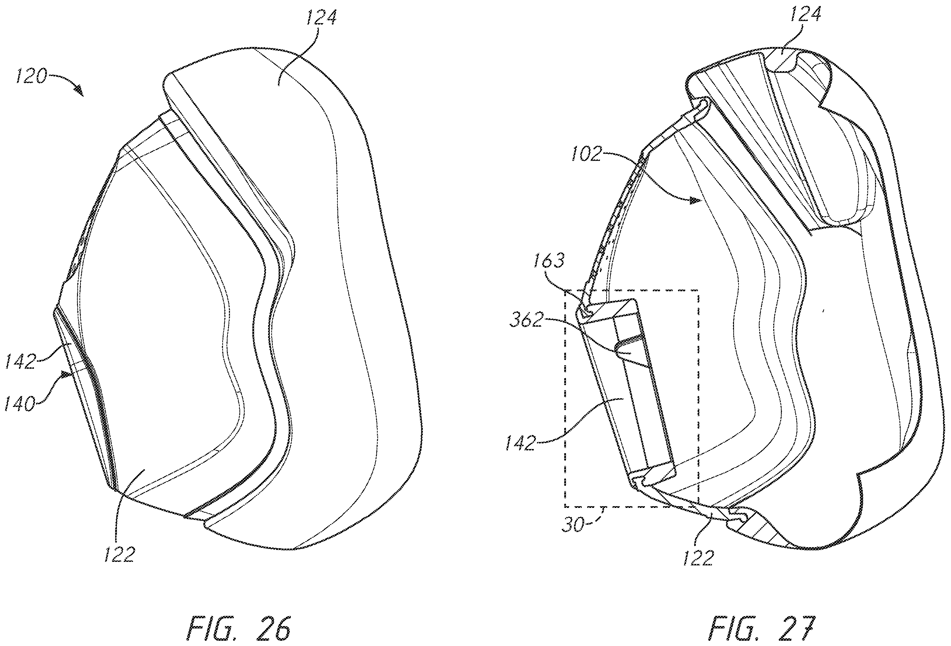

The cushion module 120 defines at least a portion of the breathing chamber 102 (Figure 27) of the patient interface 100. The cushion module 120 includes a housing 122 and a cushion or seal 124. Preferably, the housing 122 includes a wall that extends in both a radial and axial direction relative to an axis 104 along which the frame 110 and the cushion module 120 overlap or are coupled. Thus, the housing 122 defines a depth in an anteroposterior direction. In some configurations, the axis along which the frame 110 and the cushion module 120 overlap is generally or substantially aligned with the assembly axis or direction of assembly between the frame 110 and the cushion module 120. An axis of a gas inlet 130, or z-axis 104, of the frame 110 and/or the cushion module 120 can be aligned with the assembly axis.

The seal 124 is coupled to a rear or posterior portion of the housing 122. The seal 124 defines a face-contacting surface configured to contact the face of a user of the interface 100 and create a seal or substantial seal with the user's face. The seal 124 can be configured such that it surrounds either or both of the nose and mouth of the user. The seal 124 can be an inflation-type seal having an interior-concave curved wall that inflates when the interior of the interface 100 is pressurized to provide a good seal and better conform to the face of the particular user. However, other types of seals can be used, if desired. Moreover, certain features, aspects and advantages of the present disclosure can be utilized with other types of patient interfaces, such as direct nasal interfaces, among others.

With reference to Figure 27, the wall of the housing 122 surrounds, defines or includes a first opening (connection opening 132) at a forward end and a second opening at a rearward end. As discussed above, the seal 124 is connected to the second opening at the rearward end of the housing 122. Accordingly, the wall of the housing 122 extends from the first opening in a rearward direction towards the second opening. Preferably, the first opening is smaller than the second opening. That is, the second opening can have a larger vertical and/or lateral dimension than the first opening. In some configurations, a perimeter of the second opening is larger than a perimeter of the first opening. In some configurations, an area of the second opening is larger than an area of the first opening. In some configurations, because it is configured for connection to the frame 110, which provides for connection to headgear, the cushion module 120 does not include integrated headgear connector structures.

In some configurations, the housing 122 has a higher hardness and/or is more rigid than the seal 124. The housing 122 can be constructed from a more rigid material than the material of the seal 124. For example, the housing 122 can be constructed from a relatively rigid plastic (e.g., polycarbonate). The seal 124 can be constructed partially or completely from a less rigid material than the material of the housing 122, which can be a relatively soft, flexible, pliable, resilient or elastic material, such as an elastomer (e.g., silicone), a foam or a textile or a combination thereof. In some configurations, the seal 124 is coupled to the housing 122 during a molding process, such as an over-molding process. In addition, or in the alternative, the seal 124 may be mechanically coupled to the housing 122. For example, the housing 122 can include a plurality of apertures through which the material of the seal 124 passes to create a mechanical interlock between the seal 124 and the housing 122. Preferably, the housing 122 provides sufficient strength and rigidity to maintain the breathing chamber 102 during use.

In the illustrated arrangement, the housing 122 of the cushion module 120 includes a vent 170 in fluid communication with the breathing chamber 102. The vent 170 is configured to allow a flow of breathing gases and/or gases exhaled by the user to pass from the breathing chamber 102 to the atmosphere external of the patient interface 100. As previously described, the vent 170 can be a bias flow vent comprising a plurality of small holes. The vent 170 can be located in a nasal region of the cushion module 120. The frame 110 can include a vent opening 171 that is aligned with and accommodates the vent 170 when the cushion module 120 is connected to the frame 110.

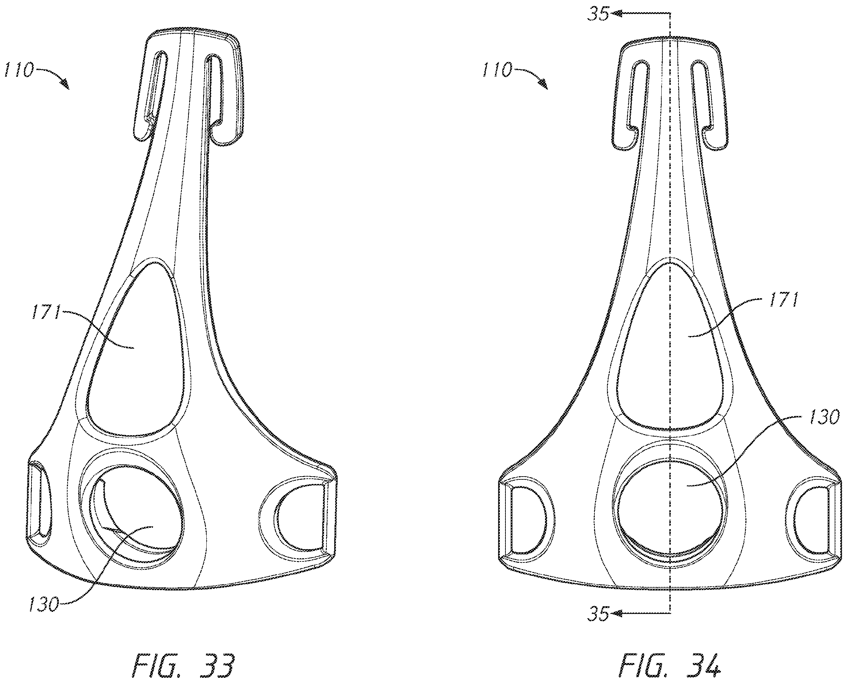

As described above, the frame 110 defines or otherwise includes a frame opening or gas inlet 130 (Figure 33) that permits a flow of breathing gas to pass through the frame 110. The cushion module 120 includes a gas inlet, cushion module opening or connection opening 132 (Figure 22) that permits a flow of breathing gas to pass through the cushion module 120. When the cushion module 120 is assembled to the frame 110, the connection opening 132 of the cushion module 120 is aligned with the gas inlet 130 of the frame 110 such that a flow of breathing gas is permitted to pass through the gas inlet 130 of the frame 110 and the connection opening 132 of the cushion module 120 into the breathing chamber 102.

A friction coupling 140 selectively couples the cushion module 120 and the frame 110. The friction coupling 140 can be designed in accordance with the criteria or methodology described above with reference to the embodiments of Figures 1-20. In some arrangements, the friction coupling 140 is the only coupling between the frame 110 and the cushion module 120. In some configurations, the friction force provided by the friction coupling 140 is the only retention force that holds the cushion module 120 and the frame 110 together when the interface 100 is not positioned on a user. That is, the friction coupling 140 can be the only direct coupling between the frame 110 and the cushion module 120. However, in other arrangements, the patient interface 100 can include additional couplings between the frame 110 and the cushion module 120.

In the illustrated arrangement, the friction coupling 140 is or comprises an elastomeric friction member 142 that creates an airtight or substantially airtight seal between the frame 110 and the cushion module 120. With such an arrangement, leakage of breathing gas (e.g., air) between the frame 110 and the cushion module 120 can be prevented or sufficiently inhibited such that the desired positive pressure can be maintained within the breathing chamber 102. The elastomeric friction member 142 can have a higher hardness than the seal 124. For example, the friction member 142 can have a 60-80 shore A hardness and the seal 124 can have a lower shore A hardness, such as about a 40 shore A hardness, for example. In some configurations, the elastomeric friction member 142 can have the same hardness as the seal 124 and/or can be constructed from the same material. For example, the elastomeric friction member 142 and the seal 124 can each have a 40 shore A hardness. In at least one configuration, the seal 124 can have a higher hardness than the elastomeric friction member 142. For example, the seal 124 can have a 30-50 shore A hardness and the elastomeric friction member 142 can have a lower shore A hardness. At least a surface of the friction member 142 that engages the frame 110 can have a textured surface finish, which as described previously herein can provide a desirable balance between retention and allowing sliding movement for assembly and disassembly. For example, in at least one configuration, at least a surface of the elastomeric friction member 142 that engages the frame 110 can have a textured surface finish formed by using a bead blasted mold tool.

The illustrated frame 110 includes at least a main wall or a front wall 150 and a collar 152. The collar 152 extends rearwardly (e.g., posteriorly or toward the user) from the front wall 150 to a rearward or free end, edge or rim and surrounds the gas inlet 130. In the illustrated arrangement, the perimeter defined by the collar 152 is circular, or generally or substantially circular. However, non-circular shapes could also be used, as described herein. The front wall 150 and the collar 152 can be unitarily formed of a material with sufficient strength and rigidity to transfer headgear forces from headgear to the cushion module 120 and/or maintain a shape of the collar 152 or any portion of the breathing chamber 102 defined by the frame 110 during use. For example, the frame 110 can be formed partially or entirely from a relatively rigid polymeric material (e.g., polycarbonate), by a process such as molding (e.g., injection molding).

In the arrangement of Figures 21-45, and any of the arrangements described elsewhere herein, a property can differ between any one or more of the components of the patient interface 100 and any other of the components. For example, one or more properties can differ between any one of the frame 110, housing 122, friction member 142 and the seal 124. Such properties can include, for example, material, modulus of elasticity, hardness, stiffness, rigidity or any combination thereof. In some configurations, one or both of the frame 110 and the housing 122 is more rigid or harder than one or both of the friction member 142 and the seal 124. In at least one configuration, the housing 122 is more rigid or harder than the friction member 142. In at least one configuration, the housing 122 is more rigid or harder than the seal 124. In at least one configuration, the frame 110 is more rigid or harder than the friction member 142. In at least one configuration, the frame 110 is more rigid or harder than the seal 124. Such arrangements can permit the frame 110 and/or the housing 122 to maintain a desired shape and transfer loads and permit the friction member 142 and/or the seal 124 to better conform to and/or seal against the frame 110 and the user, respectively.

In some configurations, one or both of the frame 110 and the housing 122 is less rigid, less hard or softer than one or both of the friction member 142 and the seal 124. In at least one configuration, the housing 122 is less rigid, less hard or softer than the friction member 142. In at least one configuration, the housing 122 is less rigid, less hard or softer than the seal 124. In at least one configuration, the frame 110 is less rigid, less hard or softer than the friction member 142. In at least one configuration, the frame 110 is less rigid, less hard or softer than the seal 124. Such arrangements can permit the frame 110 and/or the housing 122 to deform in response to loads when desirable, such as when other structures are provided to absorb the applied loads.

In some configurations, one or both of the frame 110 and the housing 122 is of the same rigidity or hardness as one or both of the friction member 142 and the seal 124. Such arrangements can include the housing 122 being formed as a unitary structure with one or both of the friction member 142 and the seal 124. The housing 122 can be the same rigidity or hardness as the frame 110, can be more rigid or harder than the frame 110, or can be less rigid, less hard or softer than the frame 110. Such arrangements allow the relative rigidity or hardness of the frame 110 and the housing 122 to be selected to facilitate deformation in one of the frame 110 or the housing 122 and/or to reduce or prevent deformation in one of the frame 110 or the housing 122, as desired.

As described above, the friction member 142 can have a different rigidity and/or hardness than the seal 124. In at least one configuration, the friction member 142 can be more rigid or harder than the seal 124, or the seal 124 less rigid or softer than the friction member 142. Such an arrangement can provide for improved durability of the friction member 142 and increased comfort of the seal 124. Also as described above, the friction member 142 can be of the same rigidity or hardness as the seal 124. Such an arrangement can provide for improved sealing and/or retention of the friction member 142. In other configurations, the friction member 142 could be less rigid, less hard or softer than the seal 124. Such an arrangement could provide for enhanced conformance of the friction member 142 relative to the frame 110 and/or sealing of the friction member 142 with the frame 110, while the seal 124 is configured for the desired level of support and comfort for the user.

In at least one configuration, the friction member 142 and the seal 124 can comprise a common material, for example a silicone. The friction member 142 and seal 124 of said configuration can have the same or substantially similar rigidity and/or hardness. Alternatively, the friction member 142 and seal 124 of said configuration can have different rigidities. Alternatively, each of the friction member 142 and seal 124 of said configuration can have a different hardness. For example, the friction member 142 and the seal 124 can comprise silicone. The silicone friction member 142 can have a 70 shore A hardness, and the silicone seal 124 can have a 40 shore A hardness. In said configuration, the friction member 142 and the seal 124 comprise a common material, and are of differing hardness, such that the seal 124 has a lower hardness than the friction member 142.

In at least one configuration, the friction member 142 and the seal 124 can comprise an uncommon material. In other words, the friction member 142 can comprise a material that the seal 124 does not comprise, or the seal 124 can comprise a material that the friction member 142 does not comprise. The friction member 142 and seal 124 of said configuration can have the same or substantially similar rigidity and/or hardness. Alternatively, the friction member and seal 124 of said configuration can have different rigidities. Alternatively, each of the friction member 142 and seal 124 of said configuration can have a different hardness. In at least one configuration, the uncommon material can be an additive. In at least one configuration, the friction member 142 and the seal 124 comprise no common materials such that the friction member 142 and seal 124 are formed from entirely different materials, or materials with different atomic structures.

In at least one configuration, the frame 110 and the housing 122 can comprise a common material, for example a polycarbonate. The frame 110 and housing 122 of said configuration can have the same or substantially similar rigidity and/or hardness. Alternatively, the frame 110 and the housing 122 of said configuration can have different rigidities and/or each can have a different hardness.

In at least one configuration, the frame 110 and the housing 122 can comprise an uncommon material. In other words, the frame 110 can comprise a material that the housing 122 does not comprise, or the housing 122 can comprise a material that the frame 110 does not comprise. The frame 110 and the housing 122 of said configuration can have the same or substantially similar rigidity and/or hardness. Alternatively, the frame 110 and the housing 122 of said configuration can have different rigidities. Alternatively, each of the frame 110 and the housing 122 of said configuration can have a different hardness. In at least one configuration, the uncommon material can be an additive. In at least one configuration, the frame 110 and the housing 122 comprise no common materials such that the frame 110 and the housing 122 are formed from entirely different materials, or materials with different atomic structures.

The collar 152 of the frame 110 defines a socket 157 configured to receive a conduit connector, such as a conduit connector portion 158, configured to connect a breathing circuit to the patient interface 100. In at least one embodiment, the conduit connector portion 158 can be in the form of an elbow 158. However, in other arrangements, the conduit connector can be connected to other portions or components of the patient interface 100, such as a portion of the cushion module 120 (e.g., the housing 122). In some configurations, the collar 152 defines at least a portion of the elbow socket 157. The conduit connector or elbow can be capable of pivoting about one or more axes relative to the frame 110. For example, the conduit connector or elbow can have a swivel connection with the frame 110.

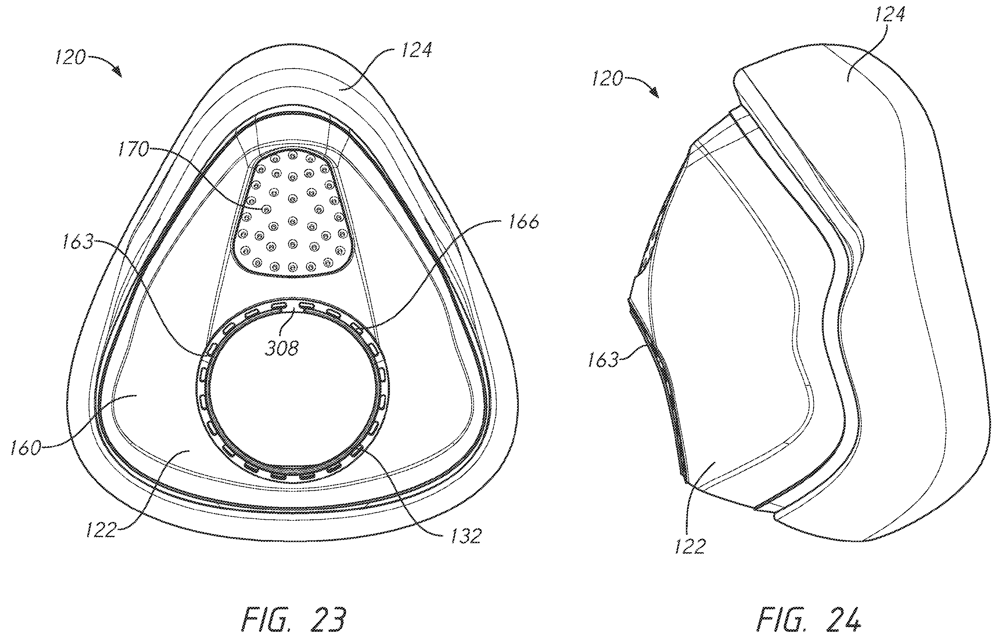

The housing 122 of the cushion module 120 includes a main wall 160 and a support flange 162. The support flange 162 and/or a portion of the main wall 160 surrounds and/or defines the connection opening 132. The housing 122 also includes a support wall 163 that surrounds and is located between the main wall 160 and the support flange 162. The support flange 162 defines a circular, generally circular or substantially circular perimeter shape from a front view that preferably corresponds to the perimeter shape of the collar 152, but preferably is somewhat larger to accommodate the portion of the friction member 142 therebetween. In the illustrated arrangement, the support flange 162 and the support wall 163 are not planar, but are curved from a side view with a slightly forward-facing concave shape, as illustrated in Figure 24. The illustrated support wall 163 extends radially inward from the main wall 160; however, the support wall 163 could also or alternatively extend in a somewhat forward or rearward direction.

The elastomeric friction member 142 is carried by or coupled to the cushion module 120. In particular, the friction member 142 is coupled to the support flange 162 and/or the support wall 163. In the illustrated arrangement, a portion or an entirety of the support wall 163 and the support flange 162are embedded within the friction member 142 to form a mechanical anchor to secure the friction member 142 to the housing 122. The support flange 162 and/or the support wall 163 can also provide hoop strength to the friction member 142 or limit outward expansion of the friction member 142 to enhance the friction fit with the collar 152 of the frame 110.

The friction member 142 can be permanently coupled to the support flange 162 and/or the support wall 163, such as by an over-molding process. The support flange 162 and/or the support wall 163 can include one or more apertures 166 through which the material of the friction member 142 extends as a result of the over-molding process. Such an arrangement provides a mechanical interlock between the friction member 142 and the housing 122. In the illustrated arrangement, the support wall 163 includes the plurality of apertures 166, each of which extends axially through the support wall 163. In some configurations, the apertures 166 are evenly distributed around the perimeter of the support wall 163. In other configurations, the apertures 166 can be unevenly distributed around the perimeter of the support wall 163.

The apertures 166 preferably are sized such that the portions of the friction member 142 passing therethrough are strong enough to resist breaking in response to normal or expected forces acting on the friction member 142 taking into account the material properties of the friction member 142. If the size of the apertures 166 is too small, the portions of the friction member 142 extending through the apertures 166 can possibly tear or break in response to normal or expected forces in use. If a sufficient number of the portions of the friction member 142 extending through the apertures 166 break, the remaining portions can be insufficient to absorb the forces applied to the friction member 142, which can result in cascading breakage of additional portions of the friction member 142 extending through the apertures 166. Conversely, if the apertures 166 are too large, the support wall 163 may not be strong enough to absorb normal or expected forces applied to the cushion module 120, such as from compression or squeezing of the cushion module 120, or large forces applied to the friction member 142. In some configurations, the apertures 166 can have a dimension of about 1 mm in a radial direction and/or about 3 mm in a circumferential direction. In other words, the ratio of a radial dimension of the apertures 166 and a circumferential dimension of the apertures 166 can be about 1:3. Each aperture 166 can be circumferentially spaced apart from each adjacent aperture 166 by an angle of about 10-25°, about 15-20°, or about 18° with respect to a central point of the connection opening 132. In some configurations, the apertures 166 can have a dimension in a radial direction that is approximately equal to the first main wall thickness 204 (support wall thickness 204).

In addition or in the alternative of a mechanical interlock between the friction member 142 and the housing 122 (such as provided by the apertures 166), a self-adhesive elastomer, such as a self-adhesive silicone, can be used to form a chemical bond the friction member 142 and the corresponding adjacent surface(s) of the housing 122. Such an arrangement can be used alone or in combination with an interlock arrangement. For example, the chemical bond of the self-adhesive material can provide enhanced connection on surfaces in which it is difficult, impractical or impossible to form apertures 166. As described herein, it may be beneficial to form the apertures 166 in the pull direction of the mold tools. For example, with reference to a general arrangement as shown in Figures 1-7, if the apertures 166 are formed in a first portion (e.g., the main wall 160) of the housing 122, the chemical bond can enhance the connection with a second portion (e.g., the support wall 163) of the housing 122, which second portion does not include apertures 166 and/or is oriented perpendicular to or at an oblique angle with respect to the first portion.

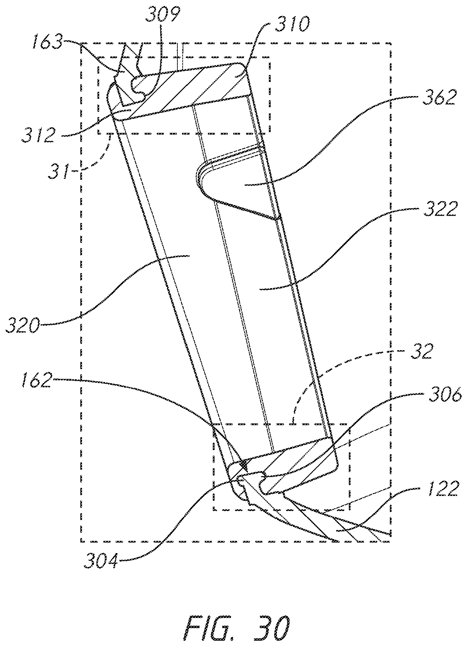

With reference to Figures 30-32, a radially-inward edge of the support wall 163 is coupled to the support flange 162. The support flange 162 defines a length that is larger than a thickness of a portion or an entirety of the support wall 163. In other words, an axial dimension of the support flange 162 is greater than an axial dimension of at least a portion or an entirety of the support wall 163. The support flange 162 increases the strength and reduces deformation of the support wall 163 in comparison to an arrangement without a support flange 162. The support flange 162 can also assist with the mechanical interlock of the friction member 142 to the cushion module 120 and/or can provide support to the friction member 142 such that the friction coupling 140 can exhibit desirable characteristics related to the assembly and disassembly of the cushion module 120, as described above with respect to Figures 1-12, for example.

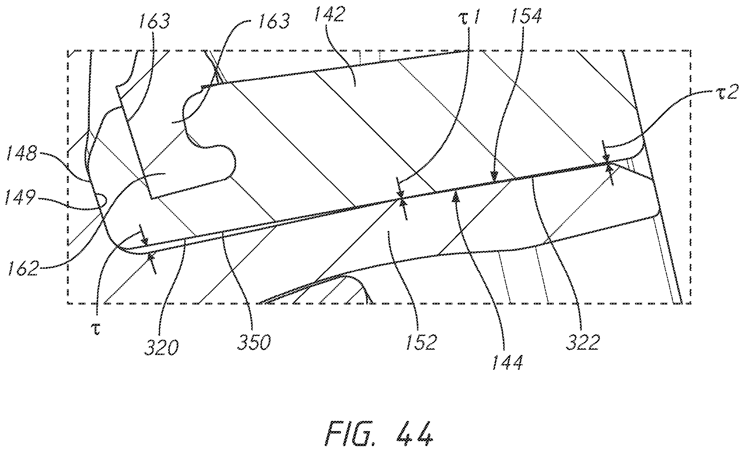

The support flange 162 can extend in either one or both axial directions relative to the support wall 163. In the illustrated arrangement, the support flange 162 includes an outer flange portion 304 (distal relative the user) and an inner flange portion 306 (proximal relative the user). The outer flange portion 304 and/or the inner flange portion 306 can completely surround the connection opening 132. In other words, the outer flange portion 304 and the inner flange portion 306 can define a closed loop. However, either or both of the outer flange portion 304 and the inner flange portion 306 can be interrupted in the circumferential direction. In the illustrated arrangement, the outer flange portion 304 includes an interruption 308 (Figure 23) at an upper portion of the support flange 162. The interruption 308 can accommodate a gate in the molding tool used to mold the housing 122 of the cushion module 120. One or both of an axial dimension (length) or radial dimension (width) of the support flange 162, including one or both of the outer flange portion 304 and the inner flange portion 306, can be constant or variable in a circumferential direction. For example, the axial and/or radial dimension can be relatively larger at or near a location or region in which additional support is desirable, such as within the inner flange portion 306 in the illustrated arrangement in which the interference is the greatest, as described below with reference to Figure 44.

The support flange 162 can be sized and shaped to provide a sufficient or desirable level of reinforcement to the support wall 163 and/or mechanical interlocking with the friction member 142. In the illustrated arrangement, a ratio of the axial dimension of the support flange 162 to an axial dimension of support wall 163 can be between about 1.375:1, 2:1, or 3:1 to about 8:1 (or greater), between about 4:1 to about 8:1, or about 6:1. In some configurations, the support wall 163 has an axial dimension or thickness of about 1.4-1.8 mm, or about 1.6 mm. The support flange 162 can have a total axial dimension or length of between about 2.2 mm, 3.2 mm, or 4.8 mm to about 12.8 mm, between about 6.4 mm to 12.8 mm, or about 9.6 mm or 10 mm. In some configurations, each of the outer flange portion 304 and the inner flange portion 306 can have equal axial dimensions or lengths. In other arrangements, the axial dimension or length of a portion or an entirety of the inner flange portion 306 can be selected to extend within a substantial portion of the friction member 142 in a manner similar to the embodiment of Figures 1-7. In some such arrangements, a length of the support flange 162 or the inner flange portion 306 is variable in the circumferential direction or around the perimeter of the support flange 162, such as relatively longer on the upper portion and relatively shorter on the lower portion. As described above, the thickness of the support wall 163 can also vary in the circumferential direction or around the perimeter of the support wall 163.

One or both of the outer flange portion 304 and the inner flange portion 306 can have rounded shapes to reduce stress concentrations in the support flange 162 and the boundaries between the support flange 162 and the friction member 142 in comparison to an arrangement with sharp corners. For example, an axial end of each of the outer flange portion 304 and the inner flange portion 306 can have a rounded edge 309. In the illustrated arrangement, the rounded edge 309 extends across the entire axial ends of the outer flange portion 304 and the inner flange portion 306. However, in other arrangements, only the corners of the axial ends are rounded. In some configurations, a transition between the support wall 163 and one or both of the outer flange portion 304 and the inner flange portion 306 can be rounded.

As described, the support flange 162 and/or the support wall 163 are embedded within the friction member 142. A portion of the friction member 142 is located on a forward side (distal the user) of the support wall 163. In the illustrated arrangement, the portion of the friction member 142 located on the forward side of the support wall 163 is relatively small compared to a portion of the friction member 142 located on a rearward side (proximal the user) of the support wall 163. In some configurations, the portion of the friction member 142 located on the forward side of the support wall 163 is sufficient to provide a suitable or desirable level of engagement or interlocking with the support flange 162 and/or the support wall 163 and/or to cover the support flange 162 and/or the support wall 163 to provide the cushion module 120 with an attractive appearance. As described above, in some configurations the support wall 163 is non-planar. In some such configurations, an axial dimension or length of the portion of the friction member 142 located on the forward side of the support wall 163 varies around the circumference of the friction member 142. In some configurations, a length of the portion of the friction member 142 located on the forward side of the support wall 163 is greater at the sides than at the top and bottom of the connection opening 132. In some configurations, the support wall 163 defines a planar forward surface.

The portion of the friction member 142 located on the rearward side of the support wall 163 is significantly greater than the portion located on the forward side of the support wall 163. In the illustrated arrangement, the connection opening 132 is positioned below the nasal region of the cushion module 120 and can be within an oral region of the cushion module 120 and/or approximately aligned with a mouth of the user. Accordingly, the primarily rearward projection of the illustrated friction member 142 takes advantage of the available space within the oral region of the breathing chamber 102 located below the user's nose. Such an arrangement allows the frame 110 and the conduit connector portion 158 to be located closer to the user's face, which reduces the effect of hose pull forces on the interface 100.

The portion of the friction member 142 located on a rearward side of the support wall 163 defines an unsupported portion 310 of the friction member 142, which can define an unsupported length dimension in the axial direction. In other words, the unsupported portion 310 is not directly supported in a radial direction by the support wall 163 or the support flange 162. A portion of the friction member 142 radially inward from the support wall 163 and/or the support flange 162 defines a supported portion 312 of the friction member 142, which can define a supported length in the axial direction. The supported portion 312 is directly supported in a radial direction by the support wall 163 and/or the support flange 162. Thus, the unsupported portion 310 can deform radially outward or increase in diameter or circumference with less force than the supported portion 312. In some configurations, as described further herein, the unsupported portion 310 applies less radial force to the collar 152 than the supported portion 312.

In some configurations, the interior or radially inward-facing surface, or engagement surface 144 of the friction member 142 includes a first engagement surface portion 320 and a second engagement surface portion 322, each of which is referred to as an "engagement surface" for convenience. The first engagement surface 320 is located forward (distal relative to the user) of the second engagement surface 322. In other words, the second engagement surface 322 is disposed at a generally greater distance away from the support wall 163 relative to the first engagement surface 320. A portion of the first engagement surface 320 and/or a portion of the second engagement surface 322 can be located within the breathing chamber 102 of the cushion module 120. In some configurations, an entirety of the second engagement surface 322 is located within the breathing chamber 102 of the cushion module 120.

In some configurations, the first engagement surface 320 and the second engagement surface 322 are the same size (e.g., length or area) and/or define about one-half of a length or surface area of the engagement surface 144. In the illustrated arrangement, the supported portion 312 defines a portion of the first engagement surface 320. The unsupported portion 310 also defines a portion of the first engagement surface 320. In addition, the unsupported portion 310 defines at least a portion or an entirety of the second engagement surface 322. The engagement surface 144, including the first engagement surface 320 and the second engagement surface 322, faces the collar 152 in use when the cushion module 120 is connected to the frame 110. At least a portion of the engagement surface 144 can be in contact with the collar 152 when the cushion module 120 is connected to the frame 110. In some configurations, as described further below, a portion of the engagement surface 144 can be spaced from the collar 152 when the cushion module 120 is connected to the frame 110. Thus, the use of the term "engagement surface" is used for convenience and does not necessarily require contact with another surface unless otherwise indicated.

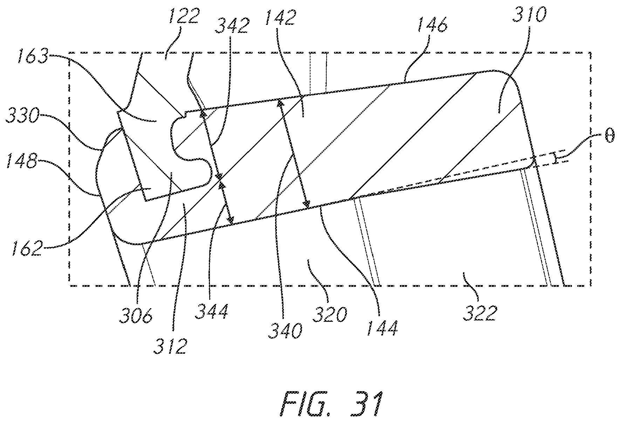

The first engagement surface 320 and the second engagement surface 322 can provide or contribute to the friction coupling 140 having different characteristics between a proximal or rearward portion and a distal or forward portion. In other words, the respective perimeters defined by the first and second engagement surfaces 320, 322 can be different in size (e.g., in perimeter length or perimeter area). In the illustrated arrangement, the first engagement surface 320 and the second engagement surface 322 can be angled (i.e., define an angle θ other than 0 or 180 degrees) relative to one another. The first engagement surface 320 and the second engagement surface 322 can define different angles relative to a longitudinal axis of the friction member 142, which can coincide with the assembly axis and/or z-axis 104. In Figure 31, the angle θ is illustrated between the second engagement surface 322 and a projection of the first engagement surface 320. The angle θ can be adjusted depending on the material of the friction member 142 (e.g., the type of elastomer or the grade of silicone) and/or the desired force profile for connection and disconnection of the cushion module 120 to the frame 110. For example, the angle θ can be between about 1-5 degrees. In some configurations, the angle θ can be about 3 degrees when a material such as a 70 shore A hardness elastomer (e.g., Silopren^{®} LSR 4070 silicone or a similar material) is used for the friction member 142.

In the illustrated arrangement, at least a substantial portion or an entirety of the first engagement surface 320 is tapered or angled relative to the axis of the friction member 142. At least a substantial portion or an entirety of the second engagement surface 322 is cylindrical or parallel to the axis of the friction member 142. In the illustrated arrangement, the radius or radial dimension of the first engagement surface 320 increases in a forward-to-rearward direction. In other words, an (e.g., circular) area defined within the friction member 142 nearer a forward (distal the user) end of the first engagement surface 320 is smaller than an (e.g., circular) area defined within the friction member 142 nearer a rearward (proximal the user) end of the first engagement surface 320. With such an arrangement, assuming the collar 152 is cylindrical, an engagement or retention force generated by the friction coupling 140 is larger at or near the forward end relative to the forces elsewhere along the friction member 142. The above discussion ignores the rounded forward and rearward-most portions of the interior surface of the friction member 142, which may be provided to reduce stress concentrations and/or to provide a lead-in to facilitate assembly of the cushion module 120 to the frame 110.

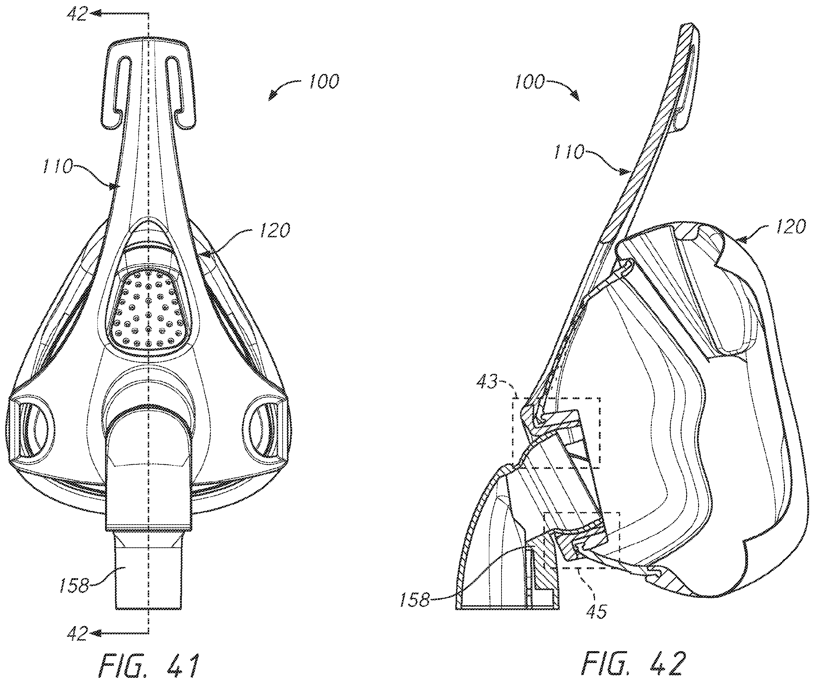

In some configurations, the friction member 142 defines an abutment surface 148 configured to contact a corresponding frame abutment surface 149 the frame 110 when the cushion module 120 is fully assembled to or engaged with the frame 110. In the illustrated arrangement, the abutment surface 148 is defined by a forward end of the friction member 142. The abutment surface 148 is planar in the illustrated arrangement, but can have any suitable or desirable shape that corresponds to or complements the shape of a surface of the frame 110 contacted by the abutment surface 148. As illustrated in Figures 42, 43 and 44, the abutment surface 148 contacts a rearward surface of the front wall 150 adjacent to and/or surrounding the collar 152. The abutment surface 148 can provide feedback to a user that the assembly or engagement of the cushion module 120 to the frame 110 is complete. In some configurations, the abutment surface 148 can provide a seal or substantial seal with the frame 110 in addition to or in place of a seal or substantial seal between the friction member 142 and the collar 152.

In the illustrated arrangement, the friction member 142 includes a chamfered or curved surface 330 radially outward of the abutment surface 148. The curved surface 330 can define a gap or space along with the frame 110, which provides a void into which portions of the friction member 142 can enter as a result of deformation of the friction member 142 when the cushion module 120 is fully engaged with the frame 110. In other words, the void can accommodate expansion of the portion of the friction member 142 forward of the support wall 163 when the cushion module 120 is assembled to or fully engaged with the frame 110. Such an arrangement can improve the performance of the cushion module 120-to-frame 110 connection by allowing for or facilitating complete engagement despite deformation of the forward end of the friction member 142.

With reference to Figure 31, the friction member 142 can define a wall thickness 340 between the engagement surface 144 and the peripheral surface 146. As described above, because one or both of the first engagement surface 320 and the second engagement surface 322 can be angled relative to the longitudinal axis of the friction member 142, the wall thickness 340 can vary along the length of the friction member 142. The wall thickness 340 can generally correspond to the first thickness or total thickness 190 described with respect to prior embodiments.

In the illustrated arrangement, an embedded portion of the housing 122 defined by the support wall 163 and/or the support flange 164 defines a radial dimension or supported thickness 342. A portion of the friction member 142 radially inward of the support wall 163 (e.g., the supported portion 312) defines a radial dimension or an inner thickness 344. The inner thickness 344 generally corresponds to the fourth thickness 196 described with respect to prior embodiments. A ratio of the supported thickness 342 to the inner thickness 344 or to the wall thickness 340 can be selected to provide or contribute to desirable attributes of the friction coupling 140, as described herein. For example, the supported thickness 342 can be selected to provide for a desirable radial dimension of the apertures 166 (e.g., 1 mm) and/or a desirable radial dimension of the support flange 162 (e.g., 1 mm). The supported thickness 342 can also be selected to provide for a desirable amount of support for the supported portion 312.

The inner thickness 344 can be selected to provide for or facilitate a desirable interference (e.g., frictional) fit with the frame 110, which can provide a sufficient retention force to maintain the cushion module 120 to the frame 110 during normal use and assembly and removal (disassembly) forces that are low enough for easy or relatively easy assembly and removal for an intended user. In some configurations, the assembly and/or removal forces are desirably between about 10-50 Newtons. For example, the inner thickness 344 can be selected such that the compression of the portion of the friction member 142 defining the inner thickness 344 after assembly of the cushion module 120 to the frame 110 provides a desirable retention force or a desirable contribution to the overall retention force. It can be desirable for the inner thickness 344 to be large enough to ensure that manufacturing variations are small enough to have a small impact on the retention force. Such an arrangement provides for a desirable amount of consistency in the assembly/disassembly force and the retention force of the cushion module 120 to the frame 110. A reasonable degree of consistency in the "feel" of assembly and disassembly can be desirable to provide the user with a level of comfort when using a new combination of a frame 110 and a cushion module 120. In some configurations, the inner thickness 344 can be about 1 mm.

Figures 42-45 illustrate the friction member 142 of the cushion module 120 engaged with the collar 152 of the frame 110. As described, when the cushion module 120 is fully assembled to the frame 110, the abutment surface 148 of the friction member 142 can contact the frame abutment surface 149 of the frame 110. The engagement surface 144 of the friction member 142 engages the peripheral surface 154 of the collar 152. The engagement surface 144 can engage the peripheral surface 154 with an interference fit along at least a portion of the friction member 142 or the collar 152. As a result of the interference fit, one or both of the friction member 142 and the collar 152 is deformed when the cushion module 120 is assembled to the frame 110.

In some configurations, a retention force generated by the interference fit varies along the length of the friction coupling 140. For example, the collar 152 can be tapered along a portion or an entirety of its length such that a first perimeter defined by a first perimeter portion at a first axial location or section of the collar 152 closer to the front wall 150 is different than a second perimeter defined by a second perimeter portion at a second axial location or section of the collar 152 further from the front wall 150. In the illustrated arrangement, the collar 152 tapers in a forward-rearward direction such that the first perimeter is greater than the second perimeter. Accordingly, the embedded portion of the housing 122, such as the support wall 163 and/or support flange 162, is located adjacent the first perimeter portion or closer to the first perimeter portion than the second perimeter portion. In such an arrangement, the embedded portion can support the friction member 142 to provide a higher retention force than when the embedded portion is located further from the first perimeter portion. However, in other arrangements, the embedded portion could be adjacent to the second perimeter portion or closer to the second perimeter portion than the first perimeter portion. Such an arrangement can be desirable if a lower retention force and/or a forward-extending friction member 142 is desired.

In some configurations, an interference fit is provided along only a portion of the length of the friction coupling 140. In other words, the retention force can be zero or negligible along at least a portion of the length of the friction coupling 140. For example, as illustrated in Figure 44, an interference fit 350 can be created along a portion or an entirety of the first engagement surface 320. In contrast, no interference fit or a relatively insignificant interference fit can be created along a portion or an entirety of the second engagement surface 322.

Figures 42-45 illustrate the first engagement surface 320 in an as-formed state to illustrate the positive interference fit 350 between the friction member 142 and the collar 152 when the cushion module 120 is assembled to the frame 110. The interference fit 350 represents the total amount of deformation required by the friction member 142 and/or the collar 152 for the cushion module 120 to fit onto the frame 110. In reality, one or both of the friction member 142 and the collar 152 would deform so that the first engagement surface 320 and the corresponding surface of the collar 152 are coincident.

With particular reference to Figure 44, due to the tapered shape or angle θ of the first engagement surface 320 with respect to the second engagement surface 322 and/or the collar 152, the amount or the radial dimension of the interference fit 350 varies along a length of the first engagement surface 320 or the friction member 142. In the illustrated arrangement, the degree of the interference fit 350 is at a maximum at or near a forward end of the friction member 142 and decreases in a direction moving toward the rearward end of the friction member 142. A maximum amount or degree of interference is designated by the reference character τ. The reference character τ1 indicates the location of minimum interference or an edge of the interference fit 350. The location indicated by the reference character τ1 can substantially coincide with a transition between the first engagement surface 320 and the second engagement surface 322. The reference character τ2 indicates a location or region of no interference. For example, the entire second engagement surface 322 can define a region of no interference in which the second engagement surface 322 does not contact or barely contacts a corresponding portion of the collar 152 (e.g., an inner diameter of the second engagement surface 322 is greater than an outer diameter of the corresponding portion of the collar 152).

A length of the friction member 142 and/or the collar 152 can be related to the size and/or the diameter (cross-sectional dimensions) of the opening 130 or 132. For example, a ratio of a length of the friction member 142 and/or the collar 152 to a diameter or a cross-sectional dimension (e.g., vertical or horizontal dimension) can be about 1:2 to about 1:4, or about 1:3. In at least one embodiment, the ratio can be a ratio of the thickness of the friction member 142 and/or the collar 152 to a maximum diameter or maximum cross sectional dimension of the opening 130 or 132. In at least one embodiment, the ratio can be a ratio of the thickness of the friction member 142 and/or the collar 152 to a minimum diameter or minimum cross sectional dimension of the opening 130 or 132. Increasing the relative length of the friction member 142 and/or the collar 152 provides resistance to removal as a result of rotation about a lateral or vertical axis, that is, an axis other than the z-axis. The second engagement surface 322 (or a friction member 142 surface of little or no interference) can be useful to engage the collar 152 when relative non-z-axis rotational forces are applied to the frame 110 and the cushion module 120 to resist instability and/or disconnection of the cushion module 120 from the frame 110.

In at least one configuration, a length of the support flange 162 can be related to the size and/or the diameter (cross sectional dimensions) of the opening 130 or 132. For example, a ratio of the thickness or length of the support flange 162 (which may correspond to the second length 182 described with reference to, for example, Figure 3) to a diameter or cross sectional dimension of the opening 130 or 132 can be about 1:2 to about 1:4, or about 1:3. In at least one embodiment, the ratio can be a ratio of the thickness or length of the support flange 162 (which may correspond to the second length 182 described with reference to, for example, Figure 3) to a maximum diameter or maximum cross sectional dimension of the opening 130 or 132. In at least one embodiment, the ratio can be a ratio of the thickness or length of the support flange 162 (which may correspond to the second length 182 described with reference to, for example, Figure 3) to a minimum diameter or minimum cross sectional dimension of the opening 130 or 132. Increasing the relative length of the support flange 162 provides resistance to removal as a result of rotation about a lateral or vertical axis. That is, an axis other than the z-axis. The second engagement surface 322 (or a friction member 142 surface of little or no interference) can be useful to engage the collar 152 when relative non-z-axis rotational forces are applied to the frame and the cushion module 120 to resist instability and/or disconnection of the cushion module 120 from the frame 110.

An arrangement in which the interference is greater near the forward end of the friction coupling 140 or is only provided within a forward portion (e.g., a portion defined by the first engagement surface 320) advantageously improves the performance of the movable conduit connector portion 158, such as when the conduit connector portion 158 is supported relative to the frame 110 by a swivel arrangement or a ball joint arrangement having one axis or two axes of rotation. Such an arrangement limits or avoids compression forces acting on the rearward portion of the collar 152, which is unsupported by the front wall 150 and more susceptible to deformation. Deformation of the rearward end of the collar 152 in some cases can reduce the size of the elbow socket 157 to such an extent that binding occurs within the ball joint or swivel joint arrangement and movement of the conduit connector portion 158 is impinged. By locating a majority of the interference fit 350 or compressive forces near the forward end (e.g., in the forward half) of the collar 152 where it is supported by the front wall 150 reduces the deformation of the collar 152 such that restriction of movement of the conduit connector portion 158 is reduced or eliminated.

In some configurations, the radial dimension at the axial location identified by the reference character τ with respect to the axis of the connection opening 132 is greater than the radial dimension at the axial location identified by the reference character τ1 with respect to a corresponding point disposed along the axis aligned with the location of τ1. In some configurations, the radial dimension at the location identified by the reference character τ is greater than 0 (zero) mm (i.e., positive interference). In some configurations, the radial dimension at the location identified by the reference character τ1 is equal to 0 (zero) mm. In some configurations, the radial dimension at the location identified by the reference character τ2 is less than 0 (zero) mm (i.e., there is no interference). Presently less preferred alternatives include having a constant interference along an entire length of the overlap between the friction member 142 and the collar 152 (τ = τ1 = τ2 > 0), having more interference at the front and rear of the overlap between the friction member 142 and the collar 152 and less in the middle (τ > τ1 < τ2), having more interference at the rear of the overlap between the friction member 142 and the collar 152 (τ2 ≥ τ1 > τ), and having more interference at the center of the overlap between the friction member 142 and the collar 152 and less at the front and rear (τ < τ1 > τ2).

Although a friction member 142 having two discrete engagement surface portions 320, 322 is illustrated, other arrangements that provide for a variable retention force along a length of the friction member 142 (or friction coupling 140) can also be used to vary the retention force as desired, such as in accordance with the principles discussed herein. For example, a curved (e.g., continuously curved) engagement surface 144 could be provided. Such an engagement surface 144 could define a curve between a first end and a second end or between a first axial location and a second axial location. Such an arrangement could include a single curved surface having a constant or variable radius or could comprise multiple curved portions. In other configurations, as described herein, the friction member 142 could include a conical engagement surface, such as in the form of a truncated cone.

The cushion module 120 and the frame 110 can incorporate structures to indicate correct assembly orientation, to inhibit or prevent incorrect assembly and/or to guide the cushion module 120 and the frame 110 towards the correct assembly orientation. In some configurations, such structures can inhibit or prevent relative movement (e.g., rotational movement) after the cushion module 120 is correctly and fully assembled to the frame 110. In some configurations, the cushion module 120 and the frame 110 can incorporate one or more sets of cooperating protrusions and recesses that provide any or all of the above-described functions.

In the illustrated arrangement, the collar 152 of the frame 110 includes a pair of recesses 360 extending in a forward direction from a rearward end of the collar 152. However, in other arrangements the collar 152 can include a single recess 360 or more than two recesses 360. In the illustrated arrangement, the recesses 360 are located in an upper portion (e.g., upper half) of the collar 152 and on opposite sides of a central, vertical plane passing in a forward-rearward direction through the frame 110. The friction member 142 of the cushion module 120 includes one or more protrusions 362 corresponding to some or all of the recesses 360. In the illustrated arrangement, the friction member 142 includes a pair of protrusions 362, each configured to be received by one of the pair of recesses 360 of the collar 152 when the cushion module 120 is assembled to the frame 110.

The recesses 360 and the protrusions 362 can have any suitable corresponding or complementary shapes. In some configurations, each protrusion 362 occupies an entirety of the corresponding recess 360 to substantially inhibit or prevent relative rotational movement of the cushion module 120 and the frame 110 about an axis of the friction coupling 140, the friction member 142 and/or the collar 152 (e.g., the z-axis). Rotation about the other axes (e.g., the x-axis and the y-axis) can be substantially inhibited or prevented as a result of the overlap of the friction member 142 and the collar 152. In other configurations, each protrusion 362 can be configured to occupy only a portion of the corresponding recess 360 such that some rotational movement is permitted. However, such an arrangement can provide an indication or guidance as to the correct assembly orientation and can inhibit or prevent incorrect assembly (e.g., the cushion module 120 being oriented upside-down relative to the frame 110).

The recesses 360 can each include at least one angled surface 364 that is angled with respect to, or non-parallel to, the assembly axis 104 or the longitudinal axis of the collar 152. The angled surface 364 can act as a lead-in for the corresponding protrusion 362 so that the cushion module 120 can be guided into the correct orientation during assembly if it is initially misaligned. In the illustrated arrangement, each of the recesses 360 also includes a straight surface 366 that is parallel to, or substantially parallel to, the assembly axis 104 or the longitudinal axis of the collar 152. However, both surfaces 364, 366 could be angled, if desired.

In the illustrated arrangement, the angled surface 364 and the straight surface 366 of each recess 360 are connected by a rounded transition, which reduces stress concentrations. The angled surface 364 and the straight surface 366 cooperate to provide the recess 360 with a generally triangular shape. In the illustrated arrangement, the angled surfaces 364 are located further from a vertical centerline of the frame 110 and the straight surfaces 366 are located closer to the vertical centerline of the frame 110. As described above, each of the protrusions 362 have a shape that corresponds to the generally triangular shape of the recesses 360. Accordingly, each of the protrusions 362 has an angled edge surface and a straight edge surface.

One type of patient interface used with PAP therapy or other respiratory therapies involving the administration of gas to a user, such as those described above, includes a seal that contacts the bridge of the user's nose. The bridge of the nose is sensitive to pressure applied by the seal of the interface assembly. More recently, interface assemblies have been developed that do not contact the bridge of the nose. Such interface assemblies can be referred to as "under-nose" interface assemblies. Such under-nose interfaces can be nasal or oro-nasal ("full face") interfaces.

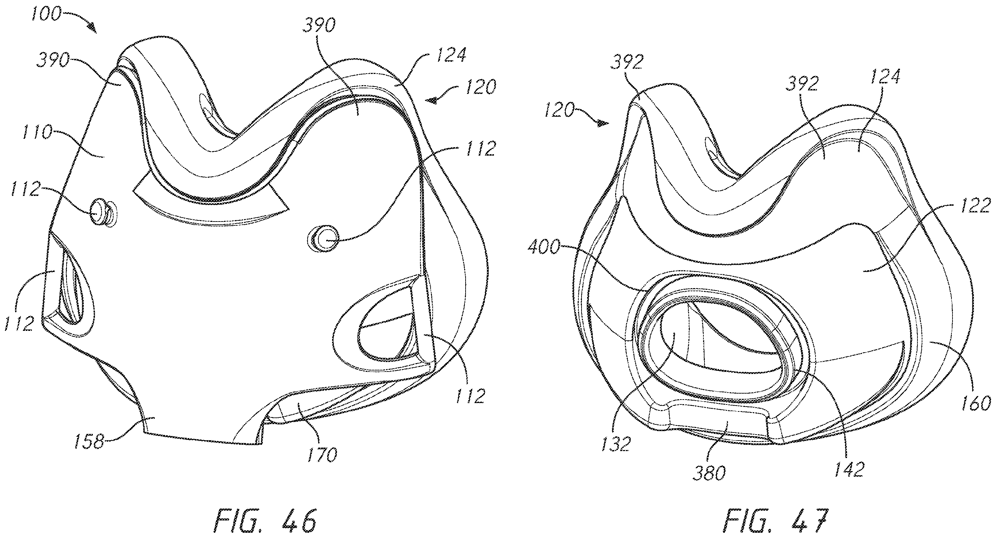

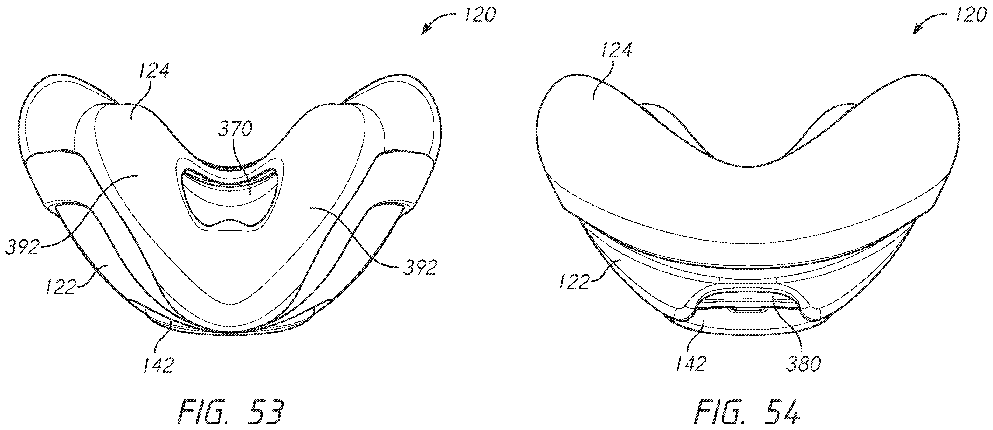

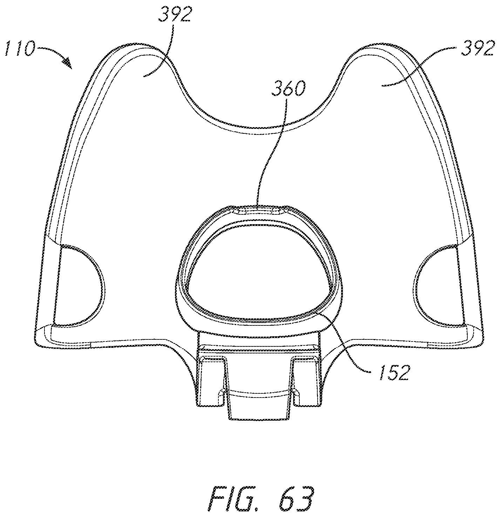

Figures 46-69 illustrate an embodiment of an oro-nasal or full-face under-nose interface assembly 100, which is similar in many respects to the other interfaces disclosed herein. Accordingly, the same reference characters can be used to refer to the same or similar components or features. The following description is focused on the unique features of the interface 100 relative to the other interfaces described herein. The interface 100 can incorporate compatible features of the other interfaces disclosed herein. In addition, features that are not disclosed in detail can be the same as or similar to other interfaces disclosed herein, or can be of another suitable arrangement.

Similar to other embodiments described herein, the patient interface 100 includes a frame 110 and a cushion module 120 removably coupled to the frame 110 by a friction coupling 140. The frame 110 is configured to be connected to headgear (not shown), which holds the patient interface 100 in a sealed relation to the user's face. The illustrated frame 110 includes a pair of upper headgear mounts 112 and a pair of lower headgear mounts 112. However, the frame 110 could include other desirable number of headgear mounts depending on the type of headgear desired to be used with the patient interface 100. The mounts 112 can be configured to directly or indirectly receive an associated headgear strap(s).

In the illustrated arrangement, each of the lower headgear mounts 112 is configured to receive a lower headgear connector clip of a corresponding lower headgear strap. Each of the lower headgear mounts 112 includes a post extending alongside an edge of the frame 110. The posts of the lower headgear mounts 112 can be spaced away from a portion of the frame 110 to define an aperture sized and shaped to accommodate a hook portion of the lower headgear connector clip. Each of the upper headgear mounts 112 is in the form of a mushroom-shaped post extending from a front surface of the frame 110. The posts of the upper headgear mounts 112 are configured to receive a headgear connector element. In some configurations, the headgear connector element is a single yoke, each end of which connects to an upper headgear strap.

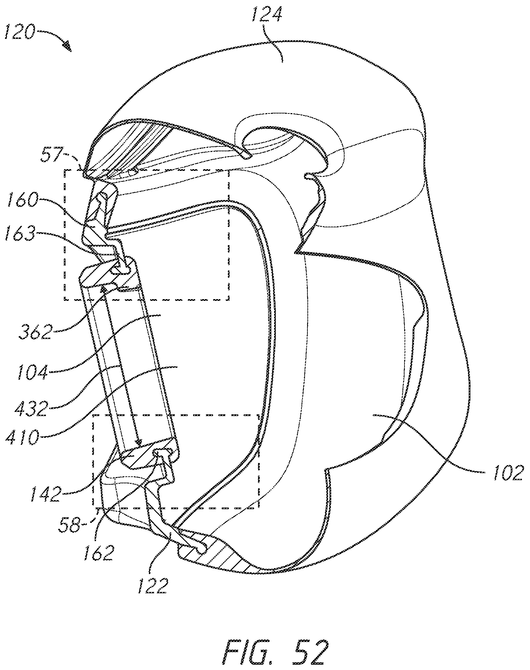

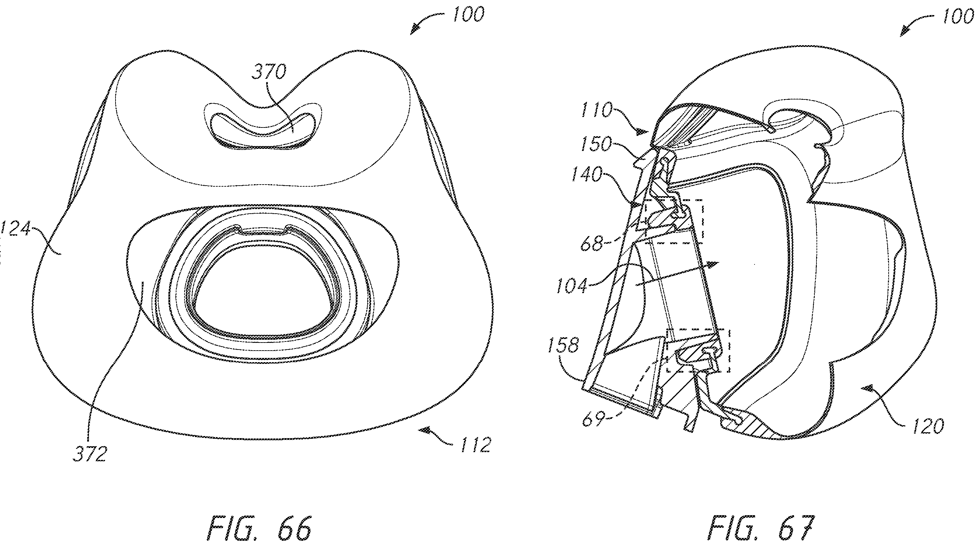

The cushion module 120 defines at least a portion of the breathing chamber 102 (Figure 52) of the patient interface 100. The cushion module 120 includes a housing 122 and a cushion or seal 124. Preferably, the housing 122 includes a wall that extends in both a radial and axial direction relative to an axis 104 (Figure 67) along which the frame 110 and the cushion module 120 overlap or are coupled. Thus, the housing 122 defines a depth in an anteroposterior direction. In some configurations, the axis 104 along which the frame 110 and the cushion module 120 overlap is generally or substantially aligned with the assembly axis or direction of assembly between the frame 110 and the cushion module 120.

The seal 124 is coupled to a rear or posterior portion of the housing 122 and defines a face-contacting surface configured to contact the face of a user of the interface 100 in a sealing or substantially sealing manner. The seal 124 can be an inflation-type seal configured such that it surrounds either or both of the nose and mouth of the user. In the illustrated arrangement, the seal 124 includes a nasal opening 370 and a separate oral or mouth opening 372. The portion of the seal 124 surrounding the nasal opening 370 is configured to contact an underside of the user's nose. The portion of the seal 124 surrounding the mouth opening 372 is configured to contact the upper lip, cheeks and lower lip/chin region of the user's face. In other configurations, the seal 124 could include a single opening or more than two openings (e.g., two nasal openings and a mouth opening). In addition, the seal 124 could include one or more superstructures (e.g., nasal pillows or prongs) for engaging the nares of the user.

The wall of the housing 122 surrounds, defines or includes a first opening (connection opening 132) at a forward end and a second opening at a rearward end. As discussed above, the seal 124 is connected to the second opening at the rearward end of the housing 122. Accordingly, the wall of the housing 122 extends from the first opening in a rearward direction towards the second opening. Preferably, the first opening is smaller than the second opening. That is, the second opening can have a larger vertical and/or lateral dimension than the first opening. In some configurations, a perimeter of the second opening is larger than a perimeter of the first opening. In some configurations, an area of the second opening is larger than an area of the first opening. In some configurations, because it is configured for connection to the frame 110, which provides for connection to headgear, the cushion module 120 does not include integrated headgear connector structures.

In some configurations, the housing 122 has a higher hardness and/or is more rigid than the seal 124. The housing 122 can be constructed from a more rigid material than the material of the seal 124. For example, the housing 122 can be constructed from a relatively rigid plastic (e.g., polycarbonate). The seal 124 can be constructed partially or completely from a less rigid material than the material of the housing 122, which can be a relatively soft, flexible, pliable, resilient or elastic material, such as an elastomer (e.g., silicone), a foam or a textile, or a combination thereof. In some configurations, the seal 124 is coupled to the housing 122 during a molding process, such as an over-molding process. In addition, or in the alternative, the seal 124 may be mechanically coupled to the housing 122. For example, the housing 122 can include a plurality of apertures through which the material of the seal 124 passes to create a mechanical interlock between the seal 124 and the housing 122. Preferably, the housing 122 provides sufficient strength and rigidity to maintain the breathing chamber 102 during use.

In some embodiments, the frame 110 can include upwardly extending support 390. In at least one configuration, the upwardly extending supports 390 can be in the form of upper lateral support portions 390. In at least one configuration, the upwardly extending supports 390 can be in the form of frame paddles 390. The upwardly extending supports 390 are configured to provide support to forward facing lateral sides of a nasal region of the mask seal 124 when the cushion module 120 is assembled to the frame 110. The lateral sides of the nasal region are referred to herein as upwardly extending portions or upward extensions 392. In at least one configuration, the upwardly extending portions 392 can be in the form of seal paddles 392. In the illustrated arrangement, the upwardly extending portions 392 are positioned above the relatively rigid housing 122. Such an arrangement allows the upwardly extending portions 392 to deflect or deform in shape to accommodate variations in the facial geometry of the individual user. For example, the upwardly extending portions 392 can deflect inwardly away from the upwardly extending supports 390 when the user's nose is pressed against the nasal portion of the seal 124. Such deflection can be advantageous in maintaining a seal with the sides of the user's nose. However, the upwardly extending supports 390 can help to minimize undesirable deflection of portions of the mask seal 124. For example, the upwardly extending supports 390 can help to maintain contact between the nasal region and the user's nose by contacting the upwardly extending portions 392 to inhibit or prevent the nasal region and/or the upwardly extending portions 392 of the seal 124 from inflating or moving outwardly or forwardly away from and/or otherwise disengaging from the user's nose. In the illustrated arrangement, the upwardly extending supports 390 are unitarily-formed with a remainder or a primary portion of the frame 110. Therefore, the upwardly extending supports 390 can have the same hardness or rigidity as the frame 110 or can be constructed of a material having the same hardness or rigidity as the material of the frame 110, including the variations of hardness or rigidity relative to other portions of the interface 100 as described in connection with the frame 110.

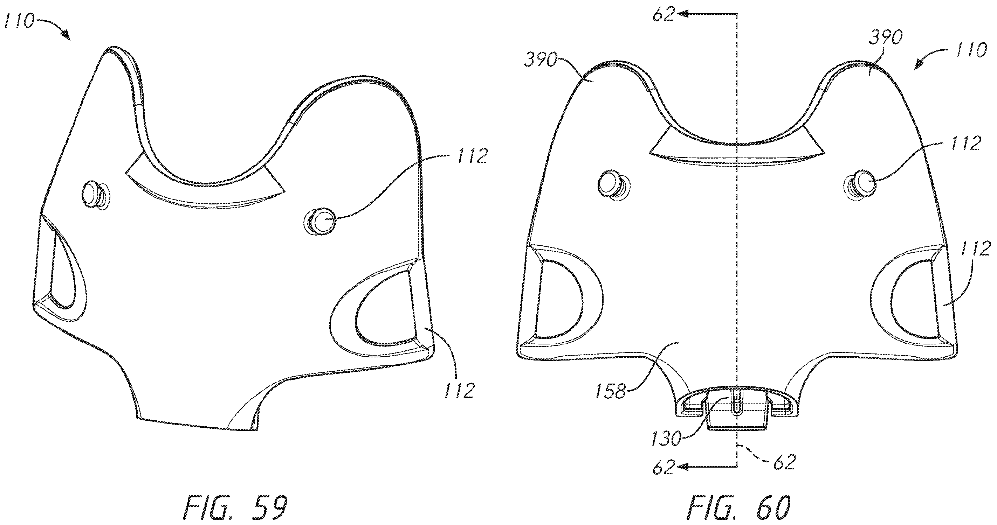

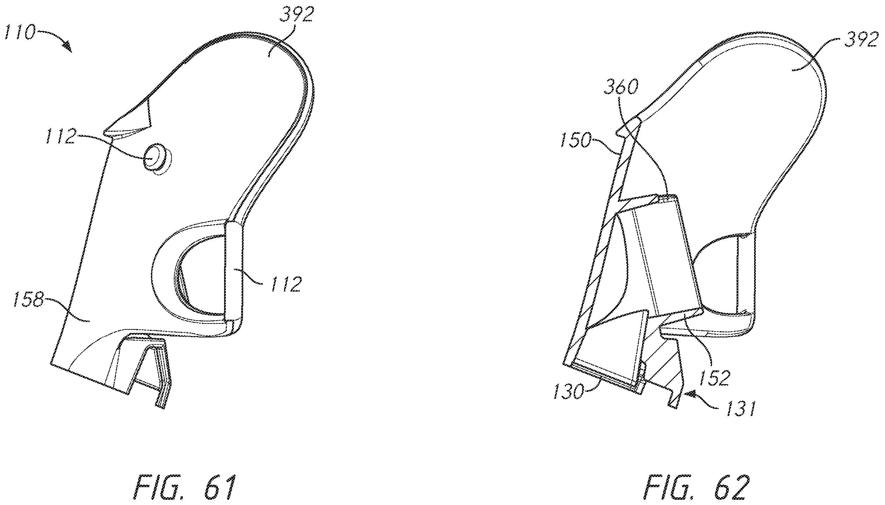

As described above, the frame 110 defines or otherwise includes a gas inlet 130 (Figures 60 and 62) that permits a flow of breathing gas to pass through the frame 110. The cushion module 120 includes a connection opening 132 (Figure 47) that permits a flow of breathing gas to pass through the cushion module 120. When the cushion module 120 is assembled to the frame 110, the gas inlet 130 of the frame 110 communicates with the connection opening 132 of the cushion module 120 such that a flow of breathing gas is permitted to pass through the frame 110 and the cushion module 120 into the breathing chamber 102.

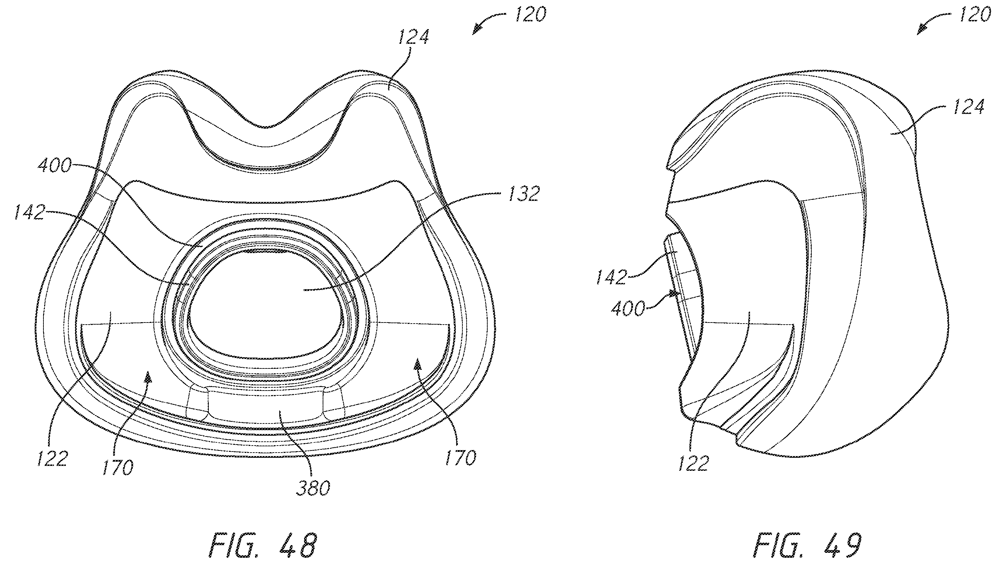

In the illustrated arrangement, the housing 122 of the cushion module 120 includes a vent 170 (Figure 48) in fluid communication with the breathing chamber 102. The vent 170 is configured to allow a flow of breathing gases and/or gases exhaled by the user to pass from the breathing chamber 102 to the atmosphere external of the patient interface 100. As previously described, the vent 170 can be a bias flow vent comprising a plurality of small holes. A portion or an entirety of the vent 170 can be located below the connection opening 132. In some configurations, the vent 170 is provided in multiple discrete portions (e.g., two portions), which can be spaced apart from one another in a lateral direction on each side of a central, vertical plane of the cushion module 120 or interface 100. In the illustrated arrangement, the vent 170 is located below an edge of the frame 110 on each side of the gas inlet 130 when the cushion module 120 is connected to the frame 110.

A friction coupling 140 (Figures 67-69) selectively couples the cushion module 120 and the frame 110. The friction coupling 140 can be designed in accordance with the criteria or methodology described above with reference to the embodiments of Figures 1-45. As described above, the friction force provided by the friction coupling 140 can be the only retention force that holds the cushion module 120 and the frame 110 together when the interface 100 is not positioned on a user. That is, the friction coupling 140 can be the only direct coupling between the frame 110 and the cushion module 120. However, in other arrangements, the patient interface 100 can include additional couplings between the frame 110 and the cushion module 120.

In the illustrated arrangement, the friction coupling 140 is or comprises an elastomeric friction member 142 that creates at least a portion of a retention coupling between the frame 110 and the cushion module 120. In some configurations, the friction member 142 can provide or contribute to creating an airtight or substantially airtight seal between the frame 110 and the cushion module 120. With such an arrangement, leakage of breathing gas (e.g., air) between the frame 110 and the cushion module 120 can be prevented or sufficiently inhibited such that the desired positive pressure can be maintained within the breathing chamber 102.

As described above, the elastomeric friction member 142 can have a greater hardness than the seal 124. However, in other configurations the elastomeric friction member 142 can have the same hardness as the seal 124. In at least one configuration, the elastomeric friction member 142 can be constructed from the same material as the seal 124. For example, in at least one embodiment, the elastomeric friction member 142 and the seal 124 can each be formed from a silicone material and, thus, can be formed from the same material. In at least one configuration, the elastomeric friction member 140 can have a lower hardness than the seal 124. At least a surface of the friction member 142 that engages the frame 110 can have a textured surface finish, which as described previously herein can provide a desirable balance between retention and allowing sliding movement for assembly and disassembly.

The illustrated frame 110 includes at least a main wall or a front wall 150 and a collar 152, which extends rearwardly (e.g., posteriorly or toward the user) from the front wall 150 to a rearward or free end, edge or rim and surrounds the gas inlet 130. In the illustrated arrangement, the perimeter defined by the collar 152 is non-circular, which can inhibit or prevent relative rotation between the frame 110 and the cushion module 120 about an axis of the collar 152. The collar 152 can be or include a somewhat rounded 'D' shape, a rounded trapezoidal shape, elliptical shape and/or oval shape, among other possible shapes. In at least one embodiment, the collar 152 can be asymmetric about at least one axis. The collar 152 being a somewhat rounded 'D' shape, a rounded trapezoidal shape, elliptical shape, oval shape, and/or asymmetric about at least one axis can be advantageous as the correct orientation of the frame 110 with respect to the cushion module 120 is more easily determined. The aforementioned configuration can also be advantageous as said configuration can provide a self-aligning connection between the frame 110 and the cushion module 120. At least one advantage of the aforementioned configuration with the elastomeric friction member 142 is that the elastomeric friction member 142 can deform during connection of the frame 110 and the cushion module 120 to improve the performance of the self-aligning connection when the frame 110 is connected to the cushion module 120.

The illustrated frame 110 also includes an integrated conduit connector portion 158, which can serve the same or a similar function as the conduit connector portion 158 of other embodiments disclosed herein. In particular, the conduit connector portion 158 couples a gas delivery conduit or breathing circuit (not shown) to the frame 110 for delivery of a flow of breathing gases to the gas inlet 130. The conduit connector portion 158 defines a portion of the gas inlet 130 along with the collar 152 and the front wall 150. As described herein, the conduit connector portion 158 can include a swivel connector, which provides for a rotational connection to the associated gas delivery conduit or breathing circuit.

In the illustrated arrangement, the cushion module 120 includes an inlet recess 380 (Figure 47) configured to accommodate at least a portion of the conduit connector portion 158. The inlet recess 380 can receive within it a rearward-facing portion of the conduit connector portion 158. The inlet recess 380 is positioned below the connection opening 132 and extends in a lateral direction along a substantial portion of a width of the connection opening 132 and/or the conduit connector portion 158. The inlet recess 380 allows the conduit connector portion 158 and/or the frame 110 to be positioned closer to the user's face in comparison to a design without the inlet recess 380. Such an arrangement can reduce the likelihood that hose pull forces will disrupt the seal of the cushion module 120 with the user's face. At least one advantage of the inlet recess 380 can be a reduced size, profile or depth of the interface 100, or at least an improved perception of the interface 100 by users who may be intimidated by larger masks.

The conduit connector portion 158 can include an anti-asphyxiation valve (AAV) 131, which can be positioned rearwardly of the gas inlet 130. The AAV 131 opens to allow ambient air to enter the breathing chamber 102 via the gas inlet 130 in the event that the flow of breathing gases is disrupted or ceases, or if the pressure within the breathing chamber 102 otherwise falls below the ambient pressure. The AAV 131 often includes a flap valve that selectively opens or closes an auxiliary passage between the gas inlet 130 and the ambient air. However, the AAV 131 can be of any suitable arrangement.

The front wall 150, the collar 152 and the conduit connector portion 158 can be unitarily formed of a material with sufficient strength and rigidity to transfer headgear forces from headgear to the cushion module 120 and/or maintain a shape of the collar 152, the conduit connector portion 158 or any portion of the breathing chamber 102 defined by the frame 110 during use. For example, the frame 110 can be formed partially or entirely from a relatively rigid polymeric material (e.g., polycarbonate), by a process such as molding (e.g., injection molding). The axis of the gas inlet 130 of the frame 110 and/or the connection opening 132 of the cushion module 120, or the z-axis 104, can be aligned with the assembly axis.

In some configurations, the integration of the conduit connector portion 158 with the frame 110 can allow the gas delivery conduit to extend in a generally downward direction from a lower front portion of the frame 110. Such a configuration can reduce the overall bulkiness of the patient interface. For example, in such configurations, the gas delivery conduit can be positioned closer to the user in use. In some configurations, a dimension of the gas inlet 130 in a forward-rearward direction is smaller than a dimension of the gas inlet 130 in a lateral direction. Accordingly, extra space is provided to accommodate the AAV 131 in a forward-rearward direction in comparison to a design in which the gas inlet 130 is circular without increasing the forward-rearward dimension of the frame 110 or moving the gas inlet 130 further away from the user's face.

The housing 122 of the cushion module 120 includes a main wall 160 and a support wall 163 (Figure 52). The support wall 163 and/or a portion of the main wall 160 surrounds and/or defines the connection opening 132. The support wall 163 defines a non-circular perimeter shape from a front view that preferably corresponds to the perimeter shape of the collar 152, but preferably is somewhat larger to accommodate a portion of the friction member 142 therebetween.

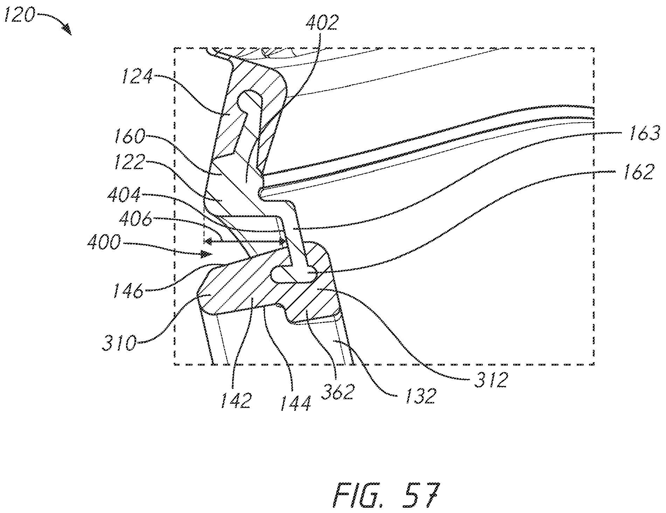

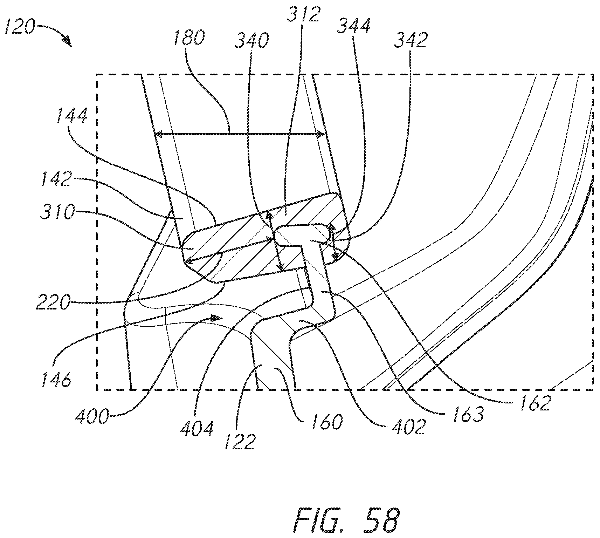

With reference to Figures 57 and 58, the illustrated support wall 163 extends radially inward from the main wall 160 and is offset from a portion of the main wall 160 immediately surrounding the support wall 163. In the illustrated arrangement, the support wall 163 is rearwardly offset from the main wall 160 to define a recess 400 of the housing 122 of the cushion module 120. The support wall 163 is connected to the main wall 160 by a connecting portion 402. In some forms, the recess 400 can be defined by the connecting portion 402 and the non-embedded portion of the support wall 163. In one form the recess 400 is bound by the connecting portion 402, the non-embedded portion of the support wall 163, and a portion of the peripheral surface 146 of the friction member 142. An axial dimension or length of the connecting portion 402 can be variable around its periphery and, in some cases, can have no axial dimension along a portion of the periphery of the recess 400. The support wall 163 defines a recessed surface 404 that is offset from a portion or an entirety of the main wall 160 by a recess distance 406. In other arrangements, the support wall 163 could be aligned with the main wall 160 or offset in a forward direction. The recess distance 406 can be constant around a periphery of the support wall 163. The recess distance 406 can be variable around a periphery of the support wall 163. The dimensions (e.g., lengths) of the structures defining the recess 400 (e.g., one or more of the connecting portion 402, the non-embedded portion of the support wall 163, and a portion of the peripheral surface 146 of the friction member 142) can be selected to create a recess 400 of a desirable size, shape or orientation to position the friction member 142 relative to the user when the cushion module 120 is properly positioned on the user's face, relative to other portions of the cushion module 120, relative to the frame 110 or relative to any other portions of the interface 100, in accordance with the considerations discussed herein, including but not limited to reducing the effect of hose pull forces acting on the interface 100.

A variable recess distance 406 around a periphery of the support wall 163 can advantageously angle the connection opening 132 with respect to the rest of the interface 100. In at least one configuration, providing the variable recess distance 406 angles the connection opening 132 such that the connection opening 132 is effectively rotated about a lateral axis of the interface 100. In at least one configuration, the variable recess distance 406 provides a connection opening 132 that is angled forward or downward about the lateral axis of the interface 100. In such a configuration, the variable recess distance 406 is greater towards a lower portion or bottom of the connection opening 132 than toward an upper portion or top. In such a configuration, the assembly axis 104 can be rotated about the lateral axis of the interface corresponding with, or approximating the rotation of the connection opening 132.

The aforementioned configuration can advantageously reduce a bending moment exerted on the interface 100 by hose pull. In at least one configuration, the variable recess distance 406 provides a connection opening 132 that is angled rearward or upward about the lateral axis of the interface 100. In such a configuration, the variable recess distance 406 is greater towards the upper portion or top of the connection opening 132 than towards the lower portion or bottom. The aforementioned configuration can advantageously improve the comfort of the interface 100. In at least one configuration, the variable recess distance 406 is the same, or substantially similar at or near a lower portion or bottom of the connection opening 132 and an upper portion or top of the connection opening 132. In at least one configuration, the variable recess distance 406 at or near lateral sides of the connection opening 132 is the same, or substantially similar.

In at least one configuration, the recessed surface 400 spans only a portion of the perimeter of the support wall 163. In at least one configuration, the recess distance 406 is a variable recess distance 406. For example, in such a configuration the recess distance 406 can vary from a maximum at or near an upper portion of the connection opening 132 to a minimum away from the upper portion of the connection opening 132, such as at or near each lateral side and/or the lower portion of the connection opening 132. In such a configuration, a lower portion of the interface 100 can be offset from an upper portion of the interface 100. The upper portion of the interface 100 can extend further forward than the lower portion. This can allow sufficient room for a user's nose within the upper portion, while reducing the volume of the breathing chamber because of the offset lower portion, thereby reducing the dead space of the interface 100.

The elastomeric friction member 142 is carried by or coupled to the cushion module 120. In particular, the friction member 142 is coupled to the support wall 163. At least a portion of the support wall 163 is embedded within the friction member 142 to form a mechanical anchor to secure the friction member 142 to the housing 122. In the illustrated arrangement, only a portion of the support wall 163) is embedded within the friction member 142 and the support wall 163 passes through a peripheral surface 146 of the friction member 142. A portion or an entirety of a perimeter of the support wall 163 defines a first portion that is embedded within the friction member 142. A portion or an entirety of a perimeter of the support wall 163 defines a second portion that is exposed or not embedded within the friction member 142. In the illustrated arrangement, the second portion is annular or extends along an entirety of a perimeter of the support wall 163 such that the peripheral surface 146 of the friction member 142 is spaced from the connecting portion 402 or an adjacent portion of the housing 122 in a radial direction. The second portion is located radially outward of the first portion. The support wall 163 can also provide hoop strength to the friction member 142 or limit outward expansion of the friction member 142 to enhance the friction fit with the collar 152 of the frame 110.

The friction member 142 can be permanently coupled to the support wall 163, such as by an over-molding process. The support wall 163 can include one or more apertures 166 through which the material of the friction member 142 extends. This can be a result of the over-molding process. Such an arrangement provides a mechanical interlock between the friction member 142 and the support wall 163 of the housing 122. In the illustrated arrangement, the support wall 163 includes the plurality of apertures 166, each of which extends axially through the support wall 163. With such an arrangement, the apertures 166 can be aligned with a pull direction of the tooling used to form the housing 122. The apertures 166 can be evenly or unevenly distributed around the perimeter of the support wall 163. The apertures 166 preferably are sized such that the portions of the friction member 142 passing therethrough are strong enough to resist breaking in response to normal or expected forces acting on the friction member 142.

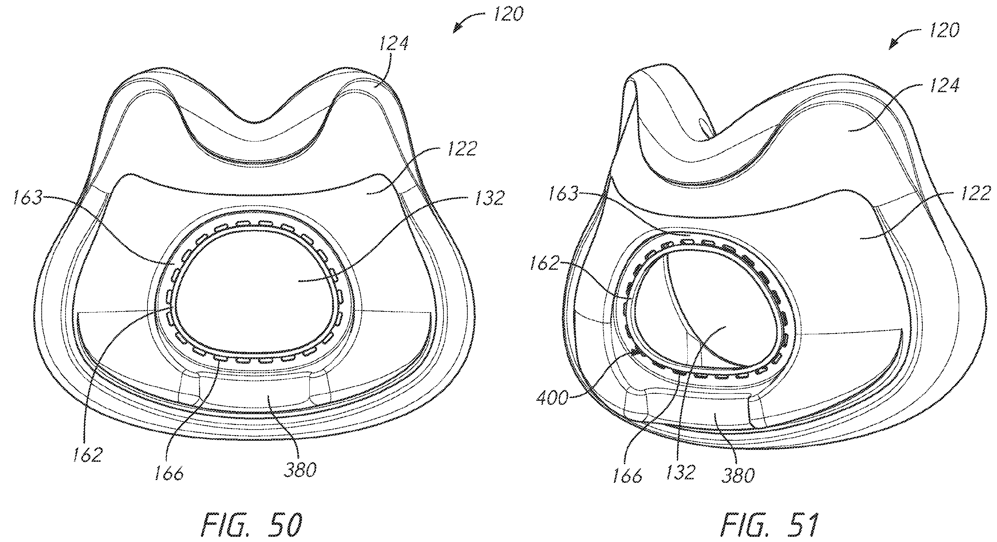

With reference to Figures 50-52, 57 and 58, a radially-inward edge of the support wall 163 is connected to a support flange 162. The support wall 163 defines a wall thickness and the support flange 162 defines a length in generally the same direction that is greater than the wall thickness of the support wall 163. The support flange 162 can increase the strength and reduce the deformation of the support wall 163 in comparison to an arrangement without a support flange 162. The support flange 162 can also assist with the mechanical interlock of the friction member 142 to the cushion module 120 and/or can provide support to the friction member 142 such that the friction coupling 140 can exhibit desirable characteristics related to the assembly and disassembly of the cushion module 120, as described above with respect to Figures 1-12, for example. In some configurations, the apertures 166 are located on the support wall 163 adjacent the support flange 162.

The support flange 162 can be sized and shaped to provide a sufficient or desirable level of reinforcement to the support wall 163 and/or mechanical interlocking with the friction member 142. In the illustrated arrangement, the support flange 162 extends in both axial directions relative to the support wall 163. In other words, the support flange 162 projects in both forward and rearward directions from the support wall 163. In the illustrated arrangement, the support flange 162 projects a greater distance in a forward direction from the support wall 163 relative to the distance of the rearward projection. However, in other arrangements this could be reversed or the portions on each side of the support flange 162 could have equal axial dimensions. The ends of the support flange 162 can have rounded corners or edges to reduce stress concentrations in the support wall 163 and the boundaries between the support wall 163 and the friction member 142 in comparison to an arrangement with sharp corners or edges.

As illustrated in Figure 52, the support flange 162 defines a central, longitudinal axis 410 that is offset with respect to, or defines an angle relative to, the axis of the friction member 142, which in the illustrated arrangement coincides with the assembly axis 104. Such an arrangement allows the pull direction of the tooling for the housing 122 and the tooling for the friction member 142 to be different from one another. In some configurations, the pull direction of the tooling is improved or optimized for each of the housing 122 and the friction member 142. In the illustrated arrangement, the axis 410 of the support flange 162 is closer to horizontal than the assembly axis 104 when the cushion module 120 is vertically-oriented, as shown in Figure 52. A forward portion of the assembly axis 104 is tilted downward relative to the axis 410 and a rearward portion of the assembly axis 104 is tilted upward relative to the axis 410. In other respects, the support flange 162 can be the same as or similar to the support flange 162 of the interface 100 of Figures 21-45.

As described, at least a portion of the support wall 163 is embedded within the friction member 142. The friction member 142 extends axially away from the support wall 163. A portion of the friction member 142 is located on a forward side (distal the user) of the support wall 163 or extends axially away from the support wall 163 in a forward direction. A portion of the friction member 142 is located a rearward side (proximal the user) of the support wall 163 or extends axially away from the support wall 163 in a rearward direction. In the illustrated arrangement, the portion of the friction member 142 located on the forward side of the support wall 163 is larger than the portion of the friction member 142 located on a rearward side of the support wall 163 in at least one dimension. For example, the portion of the friction member 142 located on the forward side of the support wall 163 can have a larger axial dimension or is longer than the portion of the friction member 142 located on a rearward side of the support wall 163, which has a smaller axial dimension or is shorter than the portion on the forward side.

In at least one configuration, the portion of the elastomeric friction member 142 that is located on the rearward side of the support wall 163 is larger than the portion of the elastomeric friction member 142 that is located on the forward side of the support wall. That is, the elastomeric friction member 142 can be considered to extend rearwardly. In at least one configuration, the portion of the elastomeric friction member 142 that is located on the rearward side of the support wall 163 is equal in length to the portion of the elastomeric friction member 142 that is located on the forward side of the support wall 163.

In the illustrated arrangement, the connection opening 132 is positioned below the nasal region of the cushion module 120 and can be within an oral region of the cushion module 120 and/or approximately aligned with a mouth of the user in a vertical direction. Accordingly, the recessed support wall 163 in combination with the primarily forward projection of the illustrated friction member 142 takes advantage of the available space within the oral region of the breathing chamber 102 located below the user's nose. Such an arrangement allows at least the conduit connector portion 158 of the frame 110 to be located closer to the user's face, which reduces the effect of hose pull forces on the interface 100. Hose pull considerations can have greater importance in under-nose interfaces or other interfaces having a forward-leaning orientation, such as when the nasal/nose portion projects further forward than the oral/mouth portion, and/or in interfaces lacking a forehead support or other similar support arrangement (e.g., overhead strap). Such interfaces generally are less stable and more prone to displacement as a result of hose pull forces. Thus, the recessed support wall 163 of the illustrated interface 100 can improve stability as a result of allowing the conduit connector portion 158 to be located closer to the user's face.

The portion of the friction member 142 located on a forward side of the support flange 162 (or the support wall 163 if there is no support flange 162 or if the support flange 162 does not extend forward of the support wall 163) defines an unsupported portion 310 of the friction member 142, which can define an unsupported length dimension in the axial direction. In other words, the unsupported portion 310 is not directly supported in a radial direction by the support flange 162 (or the support wall 163). A portion of the friction member 142 radially inward from the support flange 162 (or the support wall 163) defines a supported portion 312 of the friction member 142, which can define a supported length in the axial direction. The supported portion 312 is directly supported in a radial direction by the support wall 163 and/or the support flange 162. Thus, the unsupported portion 310 can deform radially outward or increase in diameter or circumference with less force than the supported portion 312. In some configurations, as described above, the unsupported portion 310 applies less radial force to the collar 152 than the supported portion 312.

In the illustrated arrangement, the peripheral surface 146 of the friction member 142 has portions in front of and behind the support wall 163 that lie along a continuous path. That is, the portion of the peripheral surface 146 behind the support wall 163 lies on the same trajectory as the portion in front of the support wall 163. This is a substantially linear trajectory. In an alternate configuration, rather than the same trajectory, the portion of the peripheral surface 146 behind the support wall 163 can lie along a continuous trajectory, for example that defined by a curve or spline with respect to the portion in front of the support wall 163. In the illustrated arrangement, the peripheral surface 146 is oriented at a slightly oblique angle relative to the support wall 163. In other arrangements, the peripheral surface 146 can be perpendicular to the support wall 163. In either case, the portions of the peripheral surface 146 immediately adjacent the front and rear surfaces of the support wall 163 oppose one another or are substantially aligned or aligned with one another. That is, preferably there is no significant offset between the portions of the peripheral surface 146 immediately adjacent the front and rear surfaces of the support wall 163. As described above, preferably the friction member 142 covers only a portion of the support wall 163 such that a portion of the support wall 163 is exposed, which provides space for and/or can be contacted by the shut-off portion of the molding tool for the friction member 142. With such an arrangement, the shut-off portions of the molding tool for the friction member 142 are substantially aligned or aligned with one another, which results in a more reliable molding process that is less prone to flashing than when the shut-off portions of the molding tool are not aligned, as described above with reference to Figures 13 and 14.

As described above, the recess distance 406 can be selected to position the friction member 142 desirably close to the user's mouth in use to position the frame 110, or at least the conduit connector portion 158 of the frame 110, close to the user's face to reduce the instability induced by hose pull forces. The friction member 142 can define a total friction member length 180 (Figure 58) between a forward end and a rearward end selected to provide a desirable level of retention or other attributes of the friction coupling 140, as described above. Furthermore, a forward length 220 (Figure 58) of the unsupported portion 310 can also be selected to provide a desirable level of retention or other attributes of the friction coupling 140. In the illustrated arrangement, the recess distance 406, the total friction member length 180 and the forward length 220 of the unsupported portion 310 are selected such that at least a portion of the friction member 142 projects forwardly from the recess 400 when the cushion module 120 is viewed from the side, as illustrated in Figure 49, for example.

In some configurations, the recess distance 406 is less than the total friction member length 180 and/or the forward length 220 of the unsupported portion 310 to balance the positioning of the friction member 142 close to the user's mouth while also providing a desirable amount of clearance. However, in other configurations, the recess distance 406 can be equal to or greater than one or both of the total friction member length 180 and the forward length 220 of the unsupported portion 310. Such an arrangement can occur by making the total friction member length 180 relatively small to reduce the retention force or material of the friction member 142 or by making the recess distance 406 relatively large to position the friction member 142 close to the user. For example, a cushion module 120 intended for user's with smaller faces (e.g., a size small or relatively smaller size) could have a greater recess distance 406 than a cushion module 120 intended for user's with larger faces (e.g., a size large or relatively larger size) assuming the remainder of the housing 122 of the cushion module 120 is the same or similar in size and/or shape.

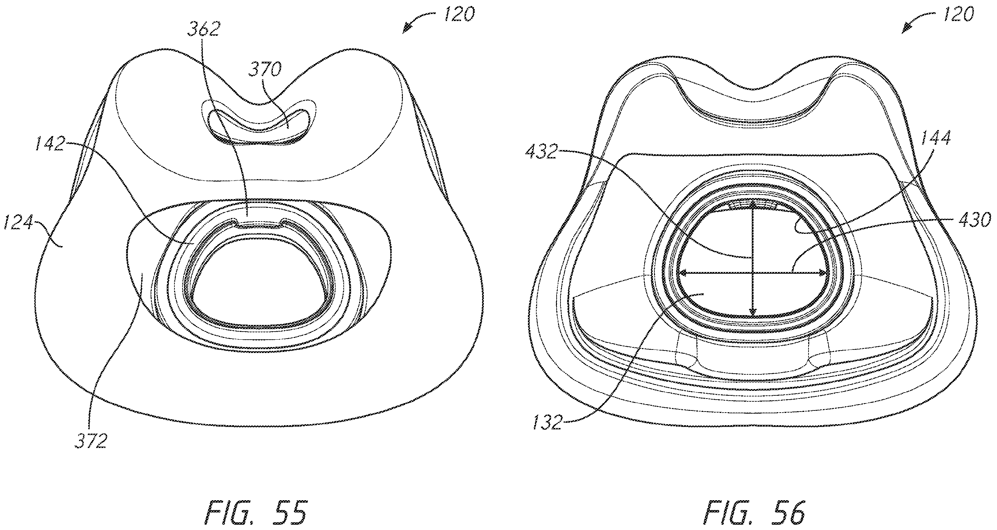

The illustrated friction member 142 has a somewhat rounded 'D' shape or a rounded trapezoidal shape when viewed from the front or rear of the cushion module 120 or along the assembly axis 104. The shape of the friction member 142 corresponds with or is complementary to the shape of the collar 152. With reference to Figure 56, the connection opening 132 of the friction member 142 defines a major axis 430 and a minor axis 432. The major axis 430 has a relatively larger dimension compared to the minor axis 432. The minor axis 432 has a relatively smaller dimension compared to the major axis 430. In the illustrated arrangement, the minor axis 432 extends between the top and the bottom of an engagement surface 144 of the friction member 142 and lies within a vertical, central plane of the cushion module 120 in the anteroposterior direction. This plane can correspond to or be parallel with a sagittal plane of the user of the patient interface. This plane can be perpendicular to a transverse plane of the user of the patient interface. The major axis 430 is perpendicular to the minor axis 430 and extends between the widest points of the left and right lateral sides of the engagement surface 144. However, this arrangement could be reversed.

The dimensions of the major axis 430 and the minor axis 432 can be selected, with consideration to the shape of the connection opening 132, to provide a suitable area through which a desirable flow rate of breathing gases into the cushion module 120 can be accommodated. The dimensions of the major axis 430 and the minor axis 432 can be selected to provide a desirable retention force or other desirable attributes of the friction coupling 140. A ratio of the major axis 430 dimension to the minor axis 432 dimension can be about 1.2-1.4:1, about 1.3:1 or about 1.27:1. The major axis 430 can have a dimension of about 25-45 mm, about 30-40 mm, or about 33 mm or 35 mm. The minor axis 432 can have a dimension of about 15-35 mm, about 20-30 mm, or about 25 mm or 26 mm.

The major axis 430 dimension can change along the friction member 142. The major axis 430 dimension can decrease along the length of the friction member 142 from the front of the friction member 142 (the distal end of the friction member with respect to the user) to the rear of the friction member 142 (the proximal end of the friction member 142 with respect to the user). In at least one configuration, the major axis 430 dimension can increase along the length of the friction member 142 from the front of the friction member 142 (the distal end of the friction member with respect to the user) to the rear of the friction member 142 (the proximal end of the friction member 142 with respect to the user).

The minor axis 432 dimension can change along the friction member 142. The minor axis 432 dimension can decrease along the length of the friction member 142 from the front of the friction member 142 (the distal end of the friction member with respect to the user) to the rear of the friction member 142 (the proximal end of the friction member 142 with respect to the user). In at least one configuration, the minor axis 432 dimension can increase along the length of the friction member 142 from the front of the friction member 142 (the distal end of the friction member with respect to the user) to the rear of the friction member 142 (the proximal end of the friction member 142 with respect to the user).

With reference to Figure 58, the friction member 142 can define a wall thickness 340 between the engagement surface 144 and the peripheral surface 146. Because one or both of the engagement surface 144 and the peripheral surface 146 can be angled relative to the longitudinal axis 104 of the friction member 142, the wall thickness 340 can vary along the length of the friction member 142. The wall thickness 340 can generally correspond to the first thickness or total thickness 190 described with respect to prior embodiments.

The embedded portion of the combination of the support wall 163 and support flange 162 (if present) defines a radial dimension or supported thickness 342. A portion of the friction member 142 radially inward of the support wall 163 (e.g., the supported portion 312) defines a radial dimension or an inner thickness 344. The inner thickness 344 generally corresponds to the fourth thickness 196 described with respect to prior embodiments. A ratio of the supported thickness 342 to the inner thickness 344 or to the wall thickness 340 can be selected to provide or contribute to desirable attributes of the friction coupling 140, as described herein. For example, the supported thickness 342 can be selected to provide for a desirable radial dimension of the apertures 166 (e.g., 1 mm) and/or a desirable radial dimension of the support flange 162 (e.g., 1 mm). The supported thickness 342 can also be selected to provide for a desirable amount of support for the supported portion 312.

The inner thickness 344 can be selected to provide for or facilitate a desirable interference fit with the frame 110. For example, the inner thickness 344 can be selected such that the compression of the portion of the friction member 142 defining the inner thickness 344 after assembly of the cushion module 120 to the frame 110 provides a desirable retention force or a desirable contribution to the overall retention force, as described above.

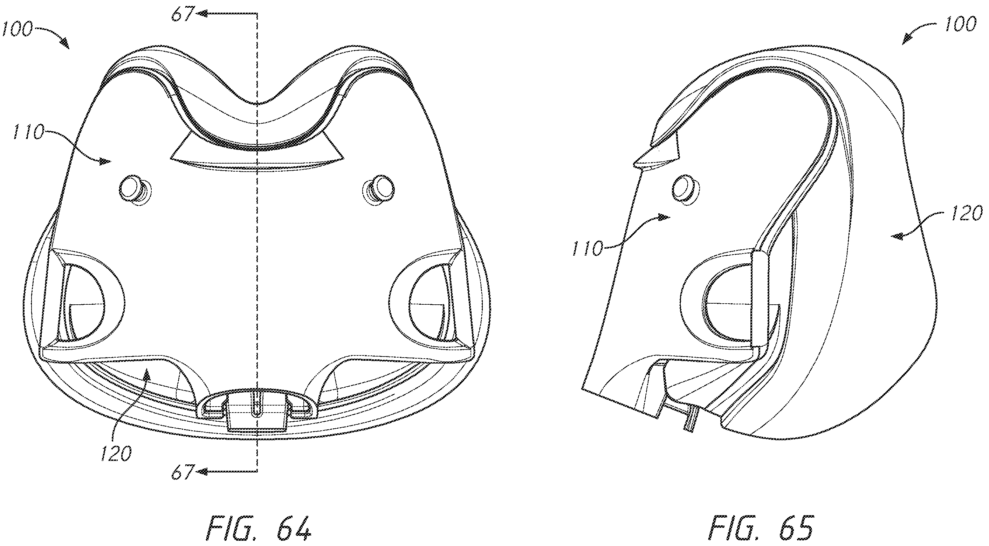

Figures 64-69 illustrate the friction member 142 of the cushion module 120 engaged with the collar 152 of the frame 110. As described, when the cushion module 120 is fully assembled to the frame 110, the interior or engagement surface 144 of the friction member 142 engages an exterior or engagement surface 154 of the collar 152. The engagement surface 144 can engage the exterior surface 154 with an interference (e.g., friction) fit along at least a portion of the friction member 142 or the collar 152. As a result of the interference or friction fit, one or both of the friction member 142 and the collar 152 is deformed when the cushion module 120 is assembled to the frame 110. In the illustrated arrangement, the peripheral surface 146 of the friction member 142 is exposed. That is, the peripheral surface 146 is not engaged by an outer wall or other similar structure. In other configurations, the exterior surface of the friction member 142 can engage the interior surface of the collar 152.

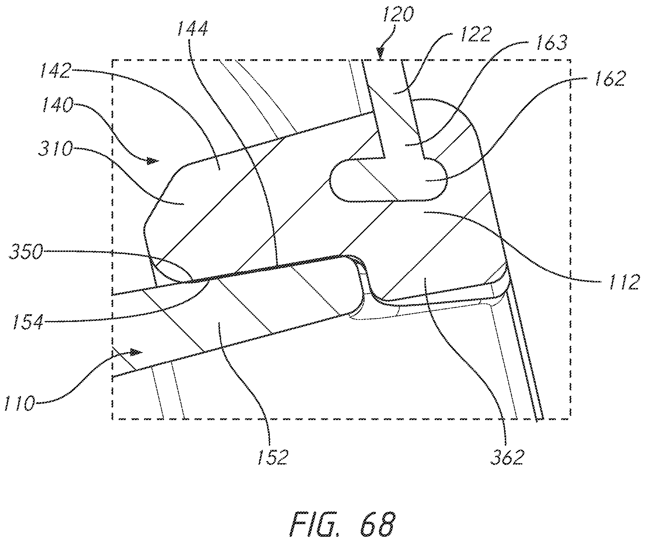

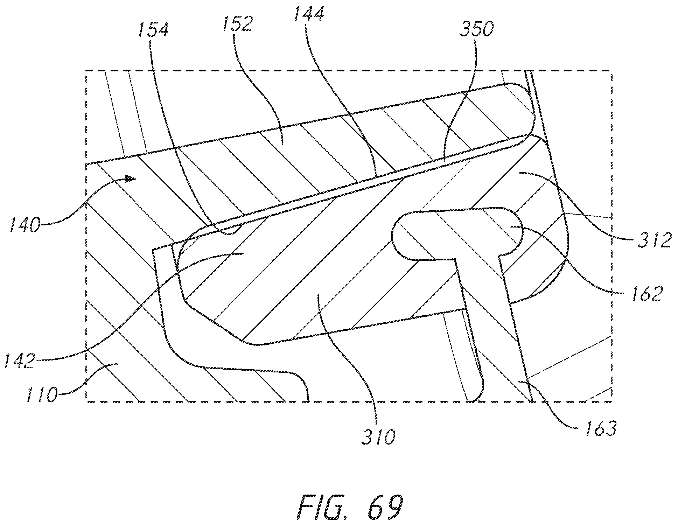

In some configurations, a retention force generated by the interference fit varies along the length of the friction coupling 140 because of, for example, variation in the wall thickness 340 along the length of the friction member 142 and/or the level of support provided to the friction member 142 (e.g., between the unsupported portion 310 and the supported portion 312). In the illustrated arrangement, an entirety or a substantial entirety of the length of the friction member 142 provides for an interference fit. For example, as illustrated in Figures 68 and 69, an interference fit 350 is created along an entirety or a substantial entirety of the engagement surface 144 or a length of the friction member 142 (excluding cut-outs or chamfered edges). However, in other configurations, an interference fit could be provided along only a portion of the length of the friction coupling 140.

Figures 68 and 69 illustrate the collar 152 of the frame 110 and the friction member 142 of the cushion module 120 in an as-formed state to illustrate the positive interference fit 350 between the friction member 142 and the collar 152 when the cushion module 120 is assembled to the frame 110. The interference fit 350 represents the total amount of deformation required by the friction member 142 and/or the collar 152 for the cushion module 120 to fit onto the frame 110. In reality, one or both of the friction member 142 and the collar 152 would deform so that the engagement surface 144 and a corresponding portion of the exterior surface 154 of the collar 152 are coincident. In the illustrated arrangement, the amount or the radial dimension of the interference fit 350 is constant along the length of the friction member 142 or friction coupling 140. However, in other arrangements, the amount or radial dimension of the interference fit 350 can vary along the length of the friction member 142 or friction coupling 140, such as due to a tapered shape or angle of the engagement surface 144 and/or exterior surface 154, as described with respect to other embodiments herein.

The cushion module 120 and the frame 110 can incorporate structures to indicate correct assembly orientation, to inhibit or prevent incorrect assembly and/or to guide the cushion module 120 and the frame 110 towards the correct assembly orientation. In some configurations, such structures can inhibit or prevent relative movement (e.g., rotational movement) after the cushion module 120 is correctly and fully assembled to the frame 110. In some configurations, the cushion module 120 and the frame 110 can incorporate one or more sets of cooperating protrusions and recesses that provide any or all of the above-described functions.

In the illustrated arrangement, the collar 152 of the frame 110 includes a recess 360 extending in a forward direction from a rearward end of the collar 152. In the illustrated arrangement, the recess 360 is located in an upper portion (e.g., upper half) of the collar 152 and aligned with a central, vertical plane passing in a forward-rearward direction through the frame 110. The friction member 142 of the cushion module 120 includes a protrusion 362 configured to be received by the recess 360 when the cushion module 120 is assembled to the frame 110. The protrusion 362 protrudes radially inwards into the connection opening 132. In at least one configuration, the protrusion can protrude away from the support wall 163.

The recess 360 and the protrusion 362 can have suitable corresponding or complementary shapes. In some configurations, the protrusion 362 occupies an entirety of the recess 360. Such an arrangement can substantially inhibit or prevent relative rotational movement of the cushion module 120 and the frame 110 about an axis of the friction coupling 140, the friction member 142 and/or the collar 152 (e.g., the z-axis) when circular shapes are employed. In some embodiments, the recess 360 has a generally trapezoidal shape oriented such that a width at rearward edge of the collar 152 is greater than a width at a position closer to the forward end of the collar 152. In other configurations, the protrusion 362 can be configured to occupy only a portion of the recess 360. Such arrangements can provide an indication or guidance as to the correct assembly orientation and can inhibit or prevent incorrect assembly (e.g., the cushion module 120 being oriented upside-down relative to the frame 110).

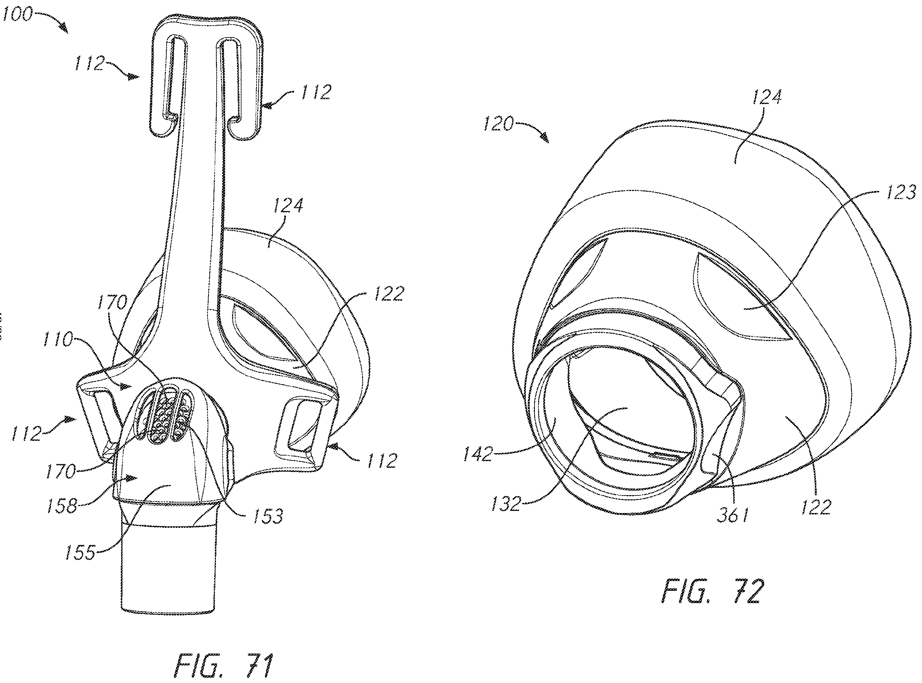

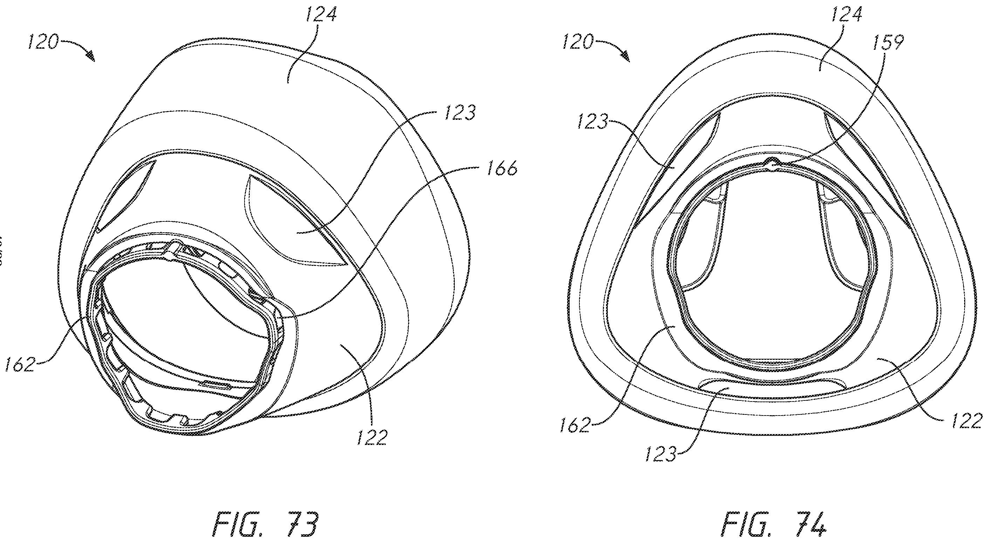

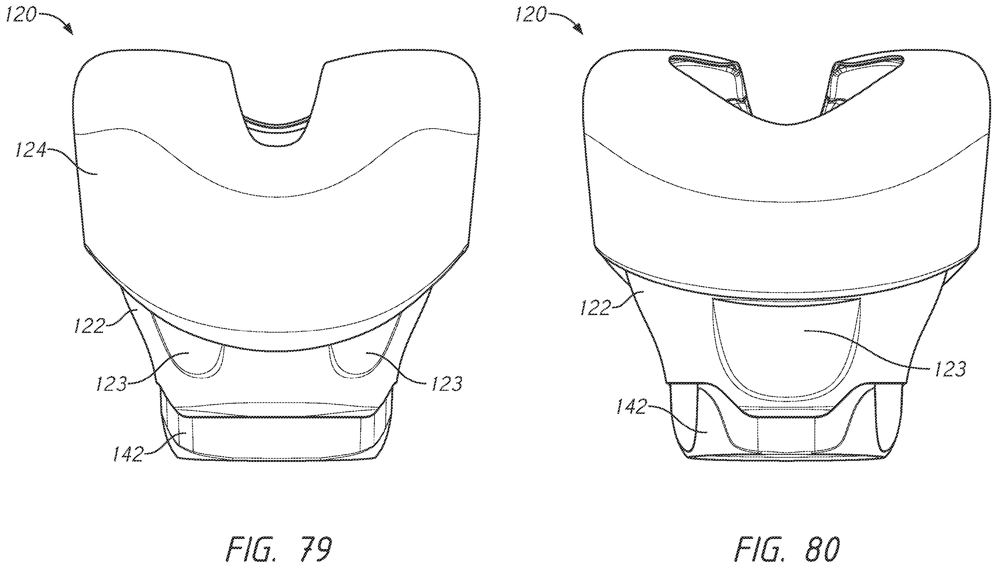

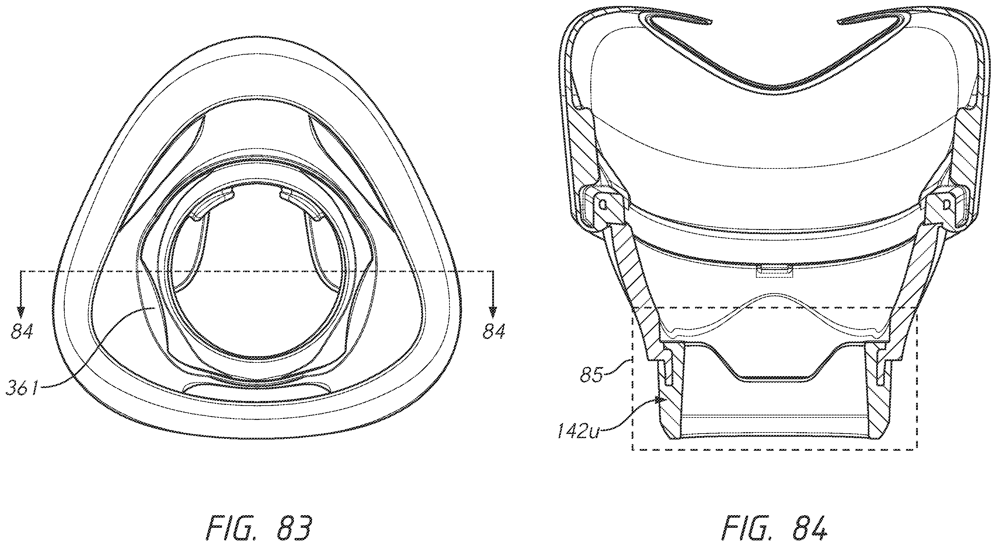

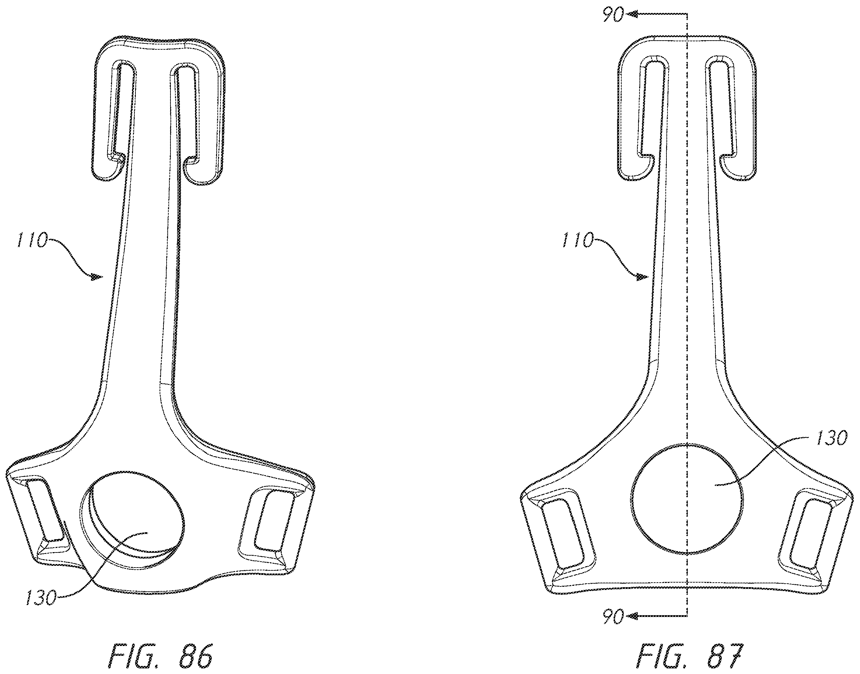

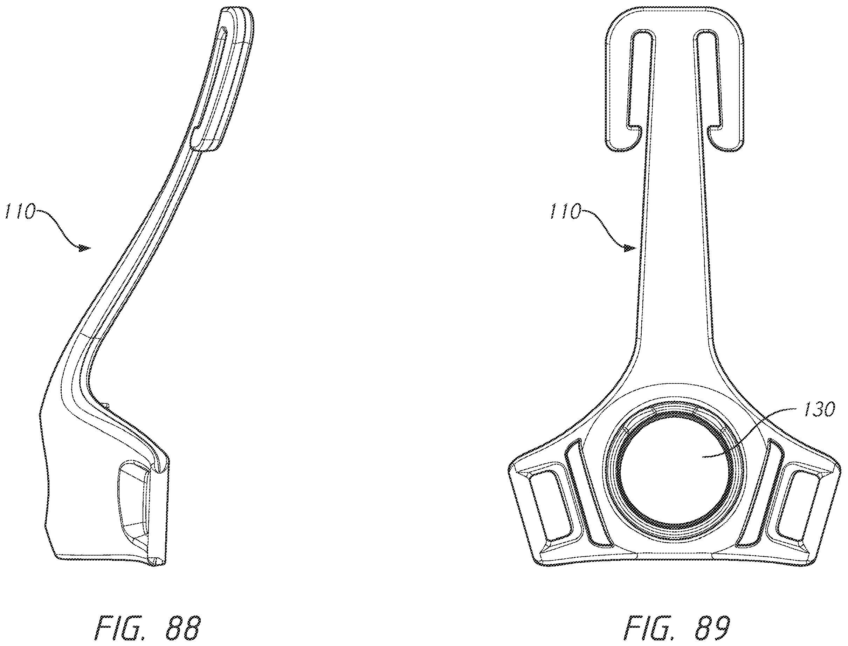

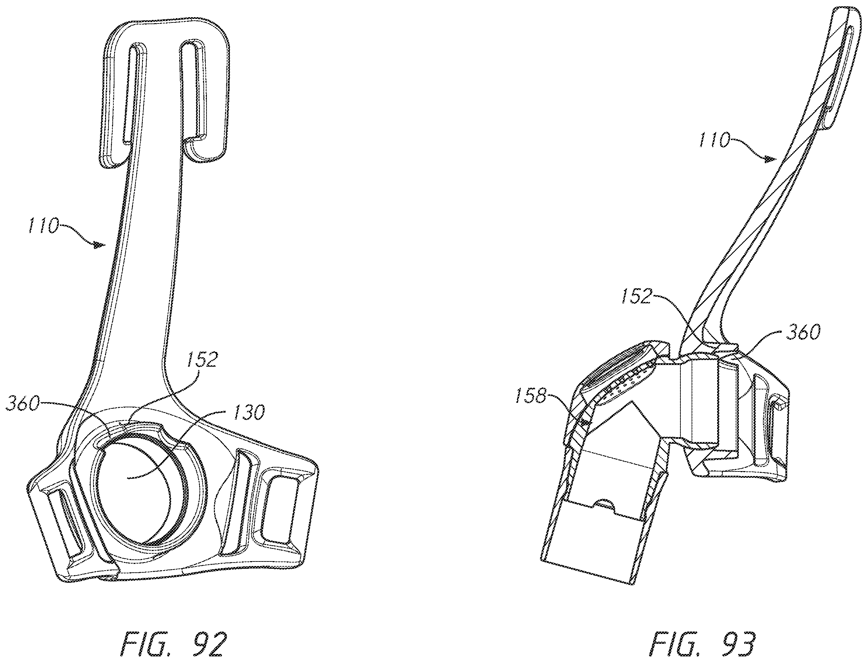

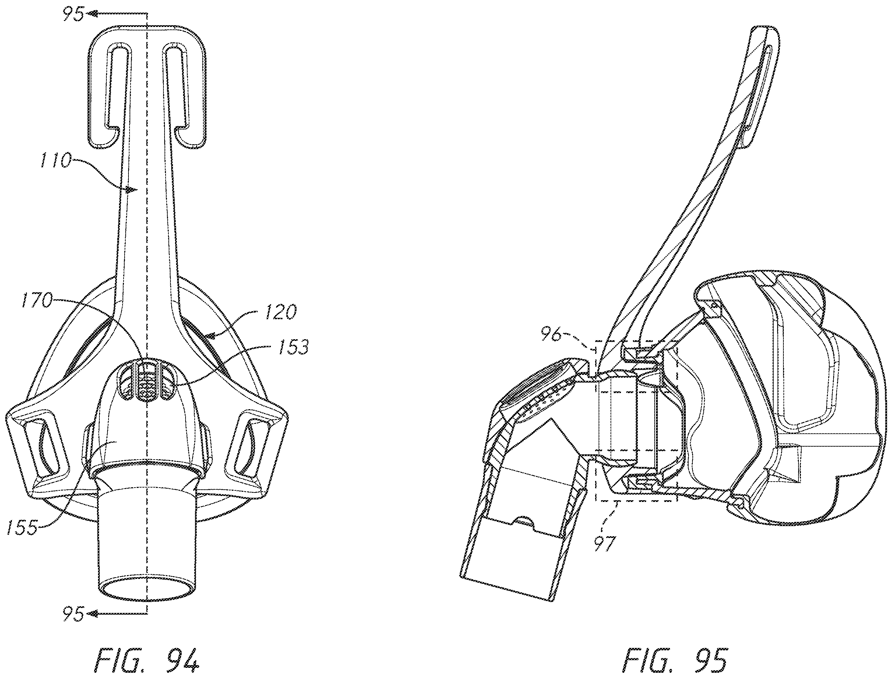

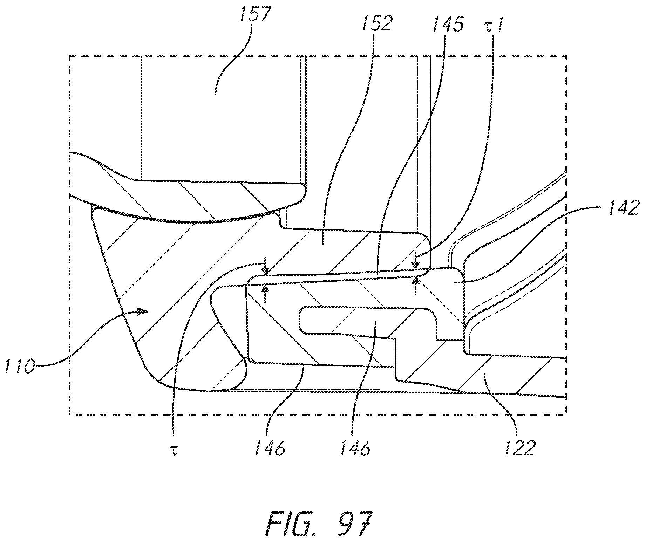

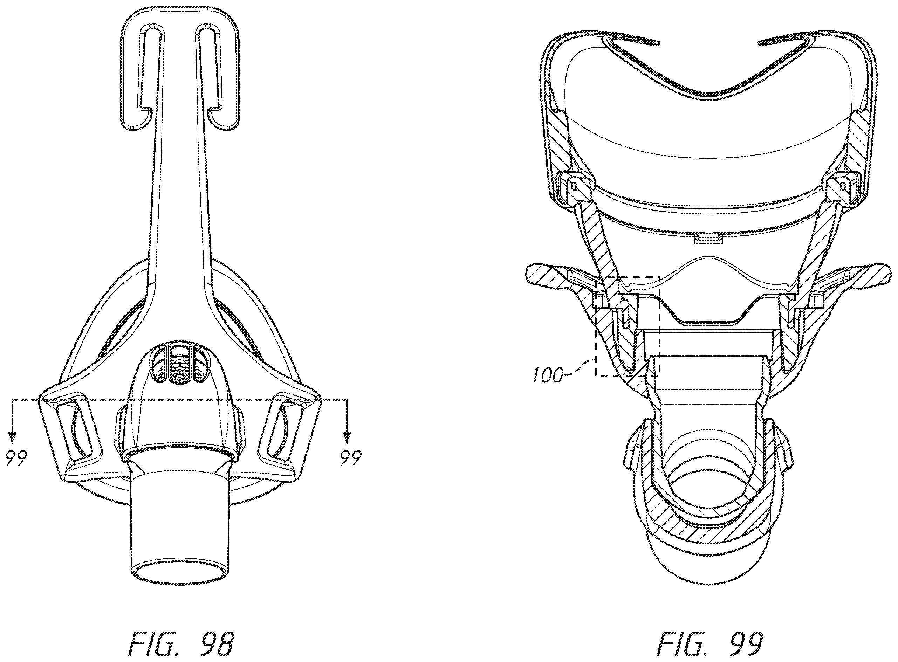

Figures 71-100 illustrate another patient interface 100 that is similar to the interface 1 00 of Figures 1-7 in some respects. Accordingly, the same reference numbers are used to refer to the same or similar features or components between the two interfaces 100. Any features or components of the patient interface 100 of Figures 71-100 that are not described in detail can be the same as or similar to the corresponding feature or component of the patient interface 100 of Figures 1-7, or can be of another suitable arrangement. The illustrated patient interface 100 includes a frame 110 and a cushion module 120 that includes a cushion or seal 124 coupled to a housing 122. In some configurations, for example as shown in Figure 72 and 74, the housing 122 can include one or more grips 123 that can provide the user a place to better or more easily grip the cushion module 120, for example, during assembly and/or disassembly with the frame 110. In the illustrated arrangement, the grips 123 are defined by recessed portions of the housing 122. In other arrangements, the grips 123 could be defined by protruding portions or by other arrangements that increase a user's grip on the cushion module 120 relative to a version lacking the grips 123. The connection opening 132 can be generally circular to accommodate a generally circular inlet collar 152 of the frame 110. A friction coupling 140 couples the cushion module 120 and the frame 110.

As described herein, the friction coupling 140 is or comprises an elastomeric friction member 142 coupled to, e.g., overmolded to, a support flange 162. The support flange 162 can be relatively more rigid than the elastomeric friction member 142. The elastomeric friction member 142 and support flange 162 together can be considered or called a cushion module flange 147. The cushion module flange 147 can define the connection opening 132. In the illustrated configuration, the elastomeric friction member 142 encloses the support flange 162. Therefore, the support flange 162 can be considered an internal flange. In some configurations, the cushion module flange 147 is designed to be backwards compatible such that the cushion module 120 can be coupled to other or prior models of the frame 110. The housing 122 comprises one or more apertures 166 through which the material of the elastomeric friction member 142 extends as a result of the over-molding process. In the illustrated arrangement, the apertures 166 are located in the support flange 162. At least a portion of the perimeter of the apertures 166 can be curved, which can help reduce stress concentration locations at the border(s) between the elastomeric friction member 142 and the support flange 162. The size of the apertures 166 can be selected based at least in part on the strength needed for the elastomeric friction member 142. The support flange 162 can include a region 159 of increased thickness. In the illustrated embodiment, the region 159 of increased thickness is disposed at or along the top of the support flange 162. The region 159 of increased thickness can serve as a gate used during injection molding of the housing 122.

As described above, the elastomeric friction member 142 can have a greater hardness than the seal 124. However, in other configurations the elastomeric friction member 142 can have the same hardness as the seal 124. In at least one configuration, the elastomeric friction member 142 can be constructed from the same material as the seal 124. For example, in at least one embodiment, the elastomeric friction member 142 and the seal 124 can each be formed from a silicone material and, thus, can be formed from the same material. In at least one configuration, the elastomeric friction member 140 can have a lower hardness than the seal 124. At least a surface of the friction member 142 that engages the frame 110 can have a textured surface finish, which as described previously herein can provide a desirable balance between retention and allowing sliding movement for assembly and disassembly.

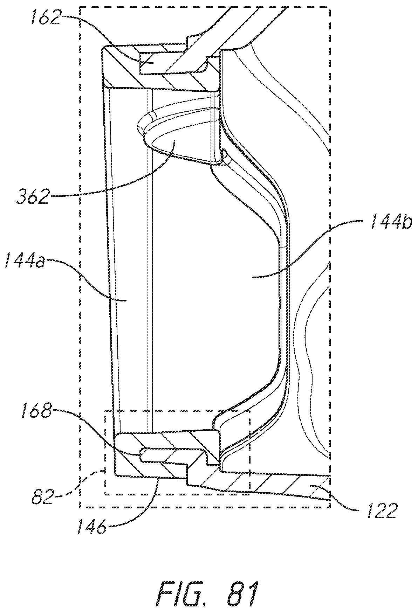

As shown in, for example, Figure 81, at least a portion of a front edge or perimeter of the support flange 162 can be a curved edge 168. The curved edge 168 can help reduce stress concentration locations at the border(s) between the elastomeric friction member 142 and the support flange 162. Reducing stress concentrations can help increase the lifespan and/or decrease wear of the elastomeric friction member 142 and/or the support flange 162.

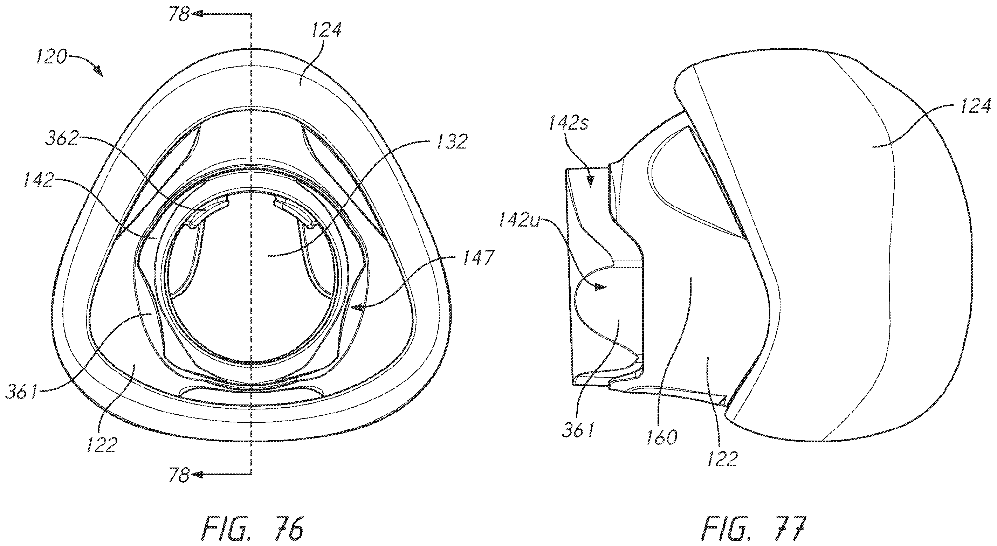

In the illustrated configuration, a rearward-most extent of the elastomeric friction member 142 is at or forward of a portion of the main wall 160 (shown in Figure 77) surrounding and/or adjacent to the connection opening 132. In other words, the support flange 162 and/or friction member 142 extend only outward from the main wall 160. Such an arrangement avoids the elastomeric friction member 142 extending into the breathing chamber 102 thereby providing more room and/or avoiding interference with the user's nose, for example in a nasal mask interface 100. As the user's nose typically fills a large portion of space within the breathing chamber 102 of a nasal mask, the housing 122 would need to be made larger to accommodate an inwardly projecting cushion module flange, which in some cases may not be desirable. An outwardly projecting cushion module flange 147 can help reduce hose-pull.

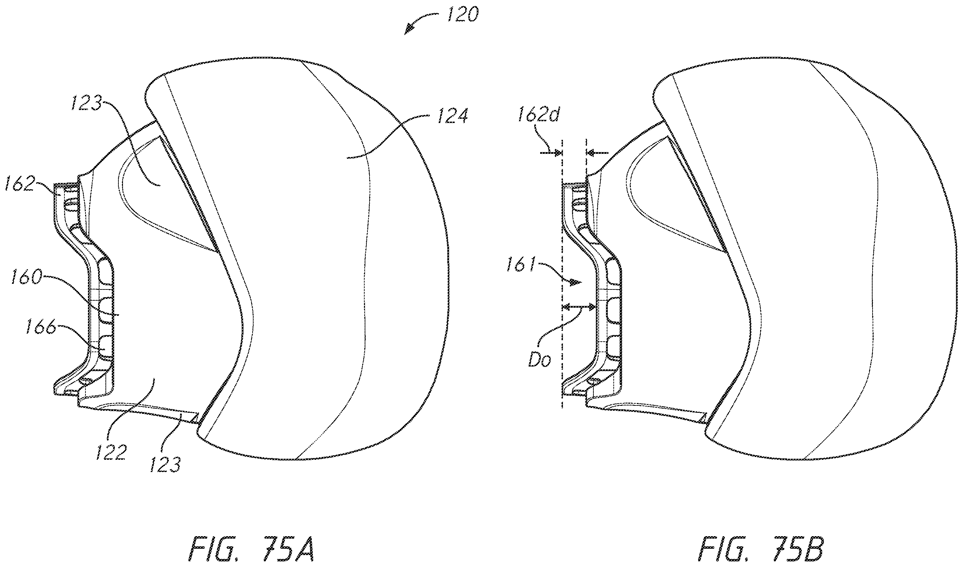

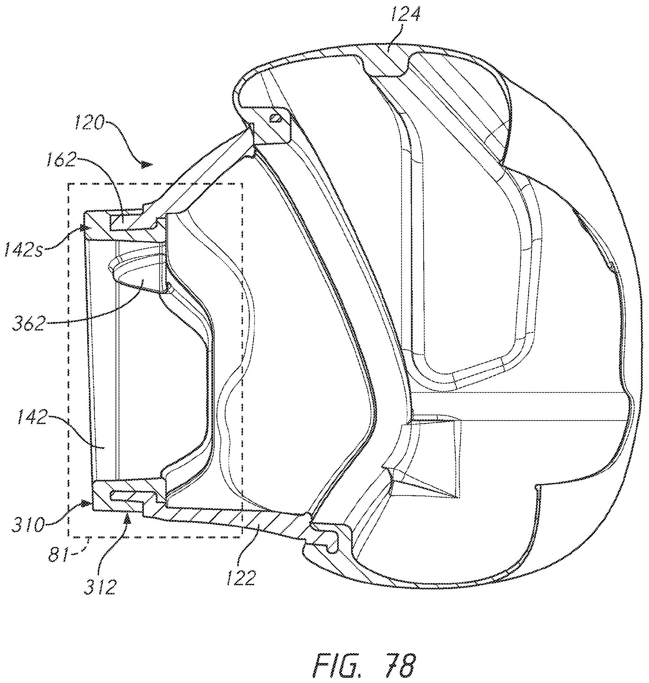

As shown in Figures 75A-75B, lateral sides or portions (e.g., arcs) of the support flange 162 are offset from upper and lower portions (e.g., arcs) in a direction of a longitudinal axis of the support flange 162 by an offset distance Do. The offset forms recessed regions 161 along the lateral sides of the support flange 162. When the elastomeric friction member 142 is coupled (e.g., overmolded) to the support flange 162, portions of the elastomeric friction member 142 span the recessed regions 161. A portion of the elastomeric friction member 142 that is radially inward and/or outward (with respect to the connection opening 132) from the support wall 163 and/or the support flange 162 defines a supported portion 312 (shown in, e.g., Figure 78) of the elastomeric friction member 142. The supported portion 312 is directly supported in a radial direction by the support wall 163 and/or the support flange 162. A portion of the elastomeric friction member 142 that does not extend radially outward or inward with respect to the support flange 162 and/or the support wall 163 defines an unsupported portion 310 (shown in, e.g., Figure 78). In other words, the unsupported portion 310 is not directly supported in a radial direction by the support flange 162 (or the support wall 163). Thus, the unsupported portion 310 can deform radially outward or increase in diameter or circumference with less force than the supported portion 312. In some configurations, as described further herein, the unsupported portion 310 applies less radial force to the collar 152 than the supported portion 312.

The elastomeric friction member 142 can therefore comprise supported regions 142s. In at least one form, the supported regions 142s can be the regions of the elastomeric friction member 142 where the axial length of the supported portion 312 is greater than the axial length of the unsupported portion 310. In other words, the supported regions 142s can be regions where the second length 182 of the elastomeric friction member 142 (as described with reference to at least Figure 3) is greater than the third length 184.

The elastomeric friction member 142 can also comprise unsupported regions 142u. In at least one form, the unsupported regions 142u can be the regions of the elastomeric friction member 142 where the axial length of the supported portion 312 is less than the axial length of the unsupported portion 310. In other words, the unsupported regions 142u can be regions where the second length 182 (as described with reference to at least Figure 3) of the elastomeric friction member 142 is less than the third length 184.

The elastomeric friction member 142 therefore has supported regions 142s (that mostly overlie or surround the support flange 162) along the upper and lower portions of the elastomeric friction member 142, and unsupported regions 142u (that mostly span the recessed regions 161) along the lateral portions, as shown in Figure 77. In some configurations, a dimension 162d (e.g., an axial length) of the support flange 162 is constant along an entirety of the support flange 162. In some configurations, the dimension 162d of the support flange 162 (shown in Figure 75B) is about 3.4mm. In some such configurations, the offset distance Do is about 5mm. In such configurations, a ratio of the offset distance Do to the support flange dimension 162d is therefore 5:3.4 or 1.47:1.

In some configurations, one or more second flange portion(s) of the support flange 162 (which may be the one or more lateral sides or portions of the support flange 162 as in the configuration shown in, e.g., Figures 75A-75B) are offset from one or more first flange portion(s) of the support flange 162 (which may be the upper and/or lower portions of the support flange 162 as in the configuration shown in, e.g., Figures 75A-75B. The one or more first flange portion(s) surround a first perimeter portion of the connection opening 132. The one or more second flange portion(s) surround a second perimeter portion of the connection opening 132. The one or more first flange portion/s can be offset from the one or more second flange portion/s along the assembly axis of the patient interface 100. The one or more first flange portion/s can be offset from the one or more second flange portion/s by a distance approximately equal to the dimension 162 of the support flange 162. The first flange portion(s) have or define a first portion of a front edge or perimeter of the support flange 162. The second flange portion(s) have or define a second portion of the front edge or perimeter of the support flange 162. The second portion of the front edge of the support flange 162 can be offset from the first portion of the front edge of the support flange 162. In some configurations, the second portion of the front edge of the support flange 162 is rearwardly (along a longitudinal axis of the connection opening 132 or along a direction of assembly of the cushion module 120 to the frame 110) offset from the first portion of the front edge of the support flange 162.

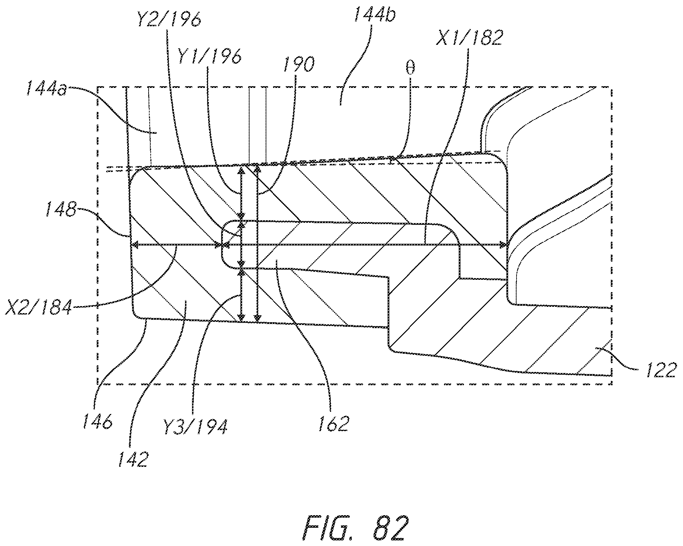

In the supported regions 142s of the elastomeric friction member 142, the support flange 162 extends for a significant length of the total length of the elastomeric friction member 142, thereby supporting the elastomeric friction member 142 along a significant portion of its length. Therefore, a supported length x1, shown in Figure 82, of the elastomeric friction member 142 (where the supported length x1 can be defined as an axial length from the base or rear edge of the elastomeric friction member 142 to the forward, free end of the support flange 162 as shown) is a significant portion of the total length of the elastomeric friction member 142. Both the supported regions 142s and unsupported regions 142u can include unsupported lengths, i.e., the length of the elastomeric friction member 142 that extends beyond the support flange 162, indicated by x2 in Figure 82. However, in the supported regions 142s, the unsupported lengths are small relative to the total length of the elastomeric friction member 142. Therefore, if x1 ≥ x2 for a given region of the elastomeric friction member 142, as shown in Figure 82, that region is a supported region 142s. If x1 < x2, that region of the elastomeric friction member 142 is an unsupported region 142u. The unsupported regions 142u can advantageously reduce the influence of manufacturing variations on the removal force between the cushion module 120 and frame 110. The unsupported regions 142u can flex, for example like cantilevers, along their length, thereby allowing for more compensation for manufacturing variations compared to a cushion module 120 with no unsupported regions.

As shown in Figure 82, the elastomeric friction member 142 defines a first thickness 190 that extends in or substantially in a radial direction or perpendicular to the direction of assembly 104. The support flange 162 defines a second thickness 192 that extends in or substantially in a radial direction or perpendicular to the direction of assembly 104. A portion of the elastomeric friction member 142 located radially outside of the support flange 162 defines a third thickness 194 that extends in or substantially in a radial direction or perpendicular to the direction of assembly 104. A portion of the elastomeric friction member 142 located radially inside of the support flange 162 defines a fourth thickness 196 that extends in or substantially in a radial direction or perpendicular to the direction of assembly 104. In the illustrated configuration, the first thickness 190 equals the sum of the second thickness 192, the third thickness 194, and the fourth thickness 196.

In the illustrated configuration, the fourth thickness 196 ≥ the second thickness 192, and the fourth thickness 196 ≥ the third thickness 194. The third thickness 194 can result from the material that forms the elastomeric friction member 142 passing through the apertures 166 during overmolding to secure the elastomeric friction member 142 to the support flange 162. In some configurations, for example in which the elastomeric friction member 142 is secured to the support flange 162 via means other than overmolding, such as chemical bonding, the third thickness 194 can be zero or approach zero. In some configurations, the second thickness 192 is equal or approximately equal to the third thickness 194. In some configurations, the second thickness 192 and/or third thickness 194 is 1mm or approximately 1mm. The third thickness 194 can vary along the length of the elastomeric friction member 142 due to, for example, angled surfaces of the support flange 162 and/or expansion or shrinkage of the elastomeric friction member 142 after molding.

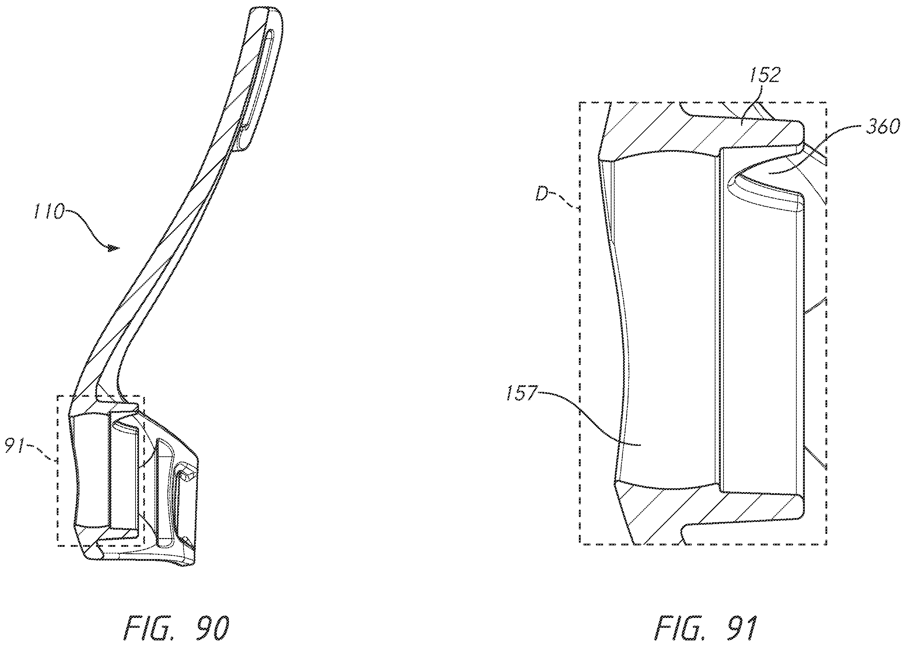

Similar to other configurations of patient interfaces 100 shown and described herein, the elastomeric friction member 142 includes a pair of protrusions 362, each configured to be received by one of a pair of recesses 360 (shown in Figure 91) of the collar 152 when the cushion module 120 is assembled to the frame 110. Cooperation between the protrusions 362 and recesses 360 can advantageously inhibit relative motion (e.g., rotation) between the frame 110 and cushion module 120. In the illustrated configuration, the protrusions 362 and recesses 360 are generally triangular. The elastomeric friction member 142 can include a pair of recesses or indents 361 in the peripheral surface 146. The recesses 361 can cooperate with a rear side of the frame 110 to help with alignment between the cushion module 120 and the frame 110.

In the illustrated arrangement, the elastomeric friction member 142 comprises an engagement surface 144 that engages an engagement surface 154 of the collar 152. A friction-fit between the collar 152, e.g., the engagement surface 154, and the elastomeric friction member 142 secures the frame 110 and cushion module 120 together. In at least one configuration, the engagement surface 144 of the elastomeric friction member 142 is an interior or radially inward-facing surface of the elastomeric friction member 142. In at least one configuration, the engagement surface 154 of the collar 152 is an exterior or radially outward-facing surface of the collar 152. However, in other arrangements, the elastomeric friction member 142 could engage an interior surface of the collar 152. Preferably, with either arrangement the frame 110 defines at least a portion of the breathing chamber 102. For example, in the illustrated configuration, an interior space defined by the collar 152 defines a portion of the breathing chamber 102.

As described above, in the illustrated arrangement, the peripheral surface 146 is exposed or is not directly covered when the cushion module 120 is assembled to the frame 110. With such an arrangement, the elastomeric friction member 142 is compressed from a single surface (e.g., the engagement surface 144), or from the engagement surface 144 and a front surface of the elastomeric friction member 142 (that may abut a rear surface of the frame 110). Accordingly, dimensional variations as a result of the manufacturing process will not impact the assembly and removal force to as great an extent as a design in which both the interior and exterior surfaces of the elastomeric friction member 142 engage a surface of a cooperating structure. Moreover, having the peripheral surface 146 of the elastomeric friction member 142 exposed to atmosphere advantageously allows the cushion module 120 and/or frame 110 to be easier to clean than an arrangement having a channel, which can be difficult to access by the user.

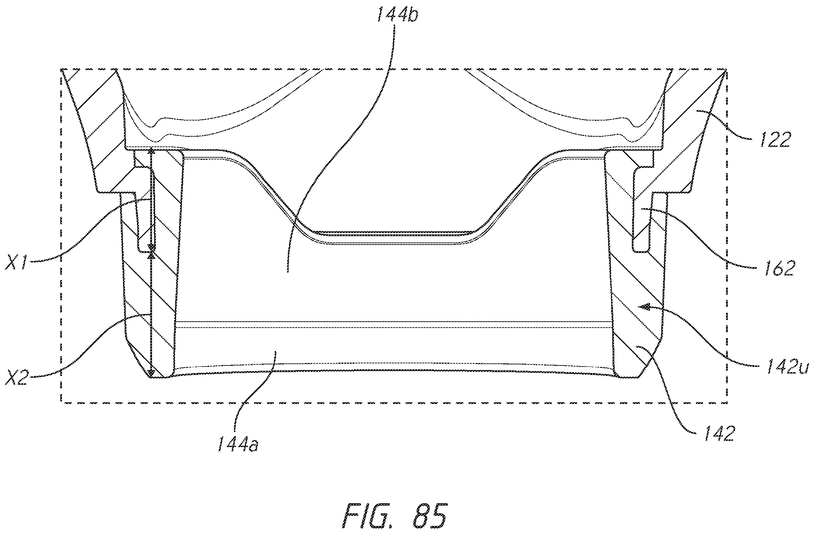

The engagement surface 144 can have a first engaging surface 144a and a second engaging surface 144b, as shown in Figures 81-82. As shown in Figure 82, the second engaging surface 144b can extend at an angle θ relative to the first engaging surface 144a. The angle θ can be selected based at least in part on the grade of silicone used for the elastomeric friction member 142 and/or the forces desired to be required to connect and/or disconnect the frame 110 and cushion module 120. For example, in some configurations in which the elastomeric friction member 142 is made of or includes LSR 4070 silicone, a θ of 3° may be selected. In some configurations, the entire first engaging surface 144a is part of the unsupported length x2 of the elastomeric friction member 142, as shown in Figure 85 (which shows a section view of the unsupported regions 142u, as indicated by x1 < x2). In the supported regions 142s, a portion of the first engaging surface 144a may be part of the supported length x1 of the elastomeric friction member 142.

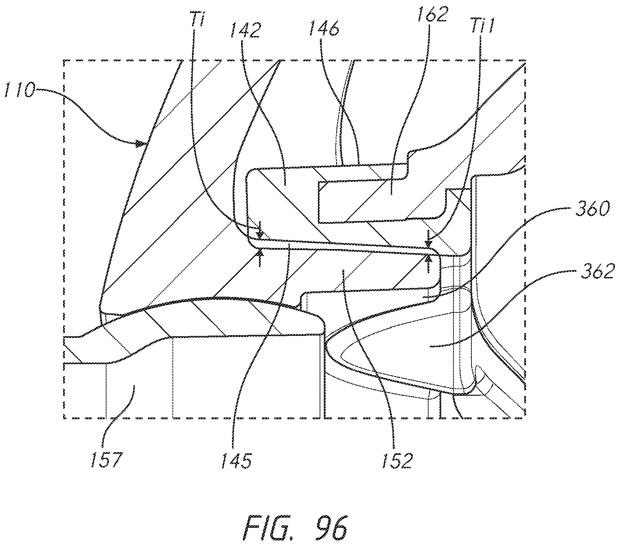

As the frame 110 and cushion module 120 are coupled, an interference region 145 of the elastomeric friction member 142 compresses and flexes to accommodate the inlet collar 152 and create the friction fit between the frame 110 and cushion module 120. As shown in Figure 96, the amount the elastomeric friction member 14-2 compresses can be considered a thickness Ti of the interference region 145 between the elastomeric friction member 142 and the inlet collar 152. A greater amount of compression or interference region thickness Ti results in a greater degree of or stronger friction or interference between the two components. The inlet collar 152 and cushion module flange 147 can be sized and/or otherwise designed so that the interference between the two is such that the force required to separate the frame 110 and cushion module 120 once connected is between about 10N and 50N during the intended lifespan of the patient interface 100.

In some configurations, the interference region thickness Ti decreases between the forward end of the cushion module flange 147 and rear end of the cushion module flange 147. In the illustrated configuration, the interference region thickness Ti is greatest or at a maximum at or near the forward end of the cushion module flange 147 (e.g., where Ti is labeled in Figure 96) and decreases toward the base or rear end of the cushion module flange 147 to a small interference region thickness labeled Ti1 in Figure 96. The interference between the frame cushion module flange 147 and inlet collar 152 is therefore biased (or greater) toward the front of the cushion module flange 147. Having a greater interference closer to the body and more vertical part of the frame 110 and farther away from the rear, free end of the inlet collar 152 can advantageously cause the inlet collar 152 to flex less and/or shifts more of the friction forces between the frame 110 and the cushion module 120 toward the base or forward end of the inlet collar 152. This can lessen the effects of rotation of a conduit connector coupled to the frame 110 as described herein. The variation in interference region thickness Ti can be at least partially the result of an angle of the inlet collar 152 relative to the cushion module flange 147 and/or the angle θ of the second engaging surface 144b relative to the first engaging surface 144a.

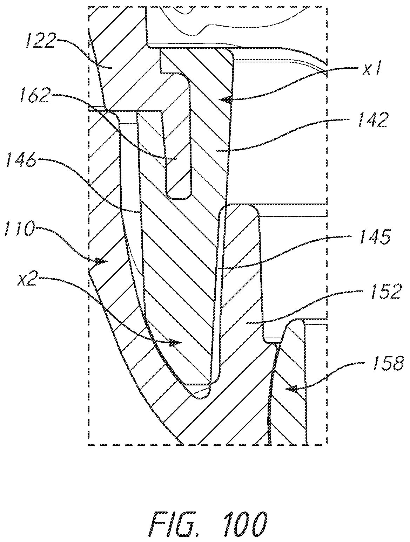

In some configurations, for example as shown in Figure 100, in the unsupported regions 142u, the interference region 145 is entirely disposed in the unsupported length x2 of the elastomeric friction member 142. In some configurations or portions of the cushion module flange 147, e.g., in supported regions 142s, the interference region 145 can at least partially be in or overlap with the supported length x1 of the elastomeric friction member 142. In portions of the interference region 145 in the supported length x1, the elastomeric friction member 142 will compress (and may flex around the edges of the support flange 162) to accommodate the inlet collar 152. In portions of the interference region 145 in the unsupported length x2, the elastomeric friction member 142 can flex outward, for example like a cantilever, instead of or in addition to compressing. A larger thickness of the elastomeric friction member 142 can flex in the unsupported lengths x2 compared to a smaller thickness of the elastomeric friction member 142 that compresses in the supported lengths x1. The unsupported lengths x2, and therefore particularly the unsupported regions 142u in which the unsupported length x2 is greater than the supported length x1, can advantageously reduce the influence of manufacturing variations on the removal force between the cushion module 120 and frame 110. A given manufacturing tolerance (e.g., 0.1mm) is a greater percentage of the interference region thickness Ti compared to the thickness of the unsupported length of the elastomeric friction member 142, which can flex.

In the illustrated arrangement, the frame 110 defines a socket 157 configured to receive a conduit connector, such as an elbow 158, configured to connect a breathing circuit to the patient interface 100. In the illustrated arrangement, the collar 152 defines at least a portion of the elbow socket 157. In some configurations, the conduit connector or elbow 158 is capable of pivoting or rotating about one or more axes relative to the frame 110. The conduit connector or elbow 158 may have one rotational degree of freedom. For example, the conduit connector or elbow 158 can have a swivel connection with the frame 110. Alternatively, the conduit connector or elbow 158 can have more than one rotational degree of freedom. For example, the conduit connector or elbow 158 may have more than one rotational degree of freedom. The elbow 158 can be removably coupled to the frame 110.

The vent 170 can be located in the elbow 158. In particular, the vent 170 can be located in a portion of an upper, outer, or patient-distal curve of the elbow 158, as shown in Figures 71 and 93-95. In the illustrated configuration, an elbow vent piece 155 is fitted over a portion of the elbow 158 including the vent 170. The elbow vent piece 155 can be removably coupled to the elbow 158. The elbow vent piece 155 includes one or more vent apertures 153 extending through the cover 155. In use, gases pass through the vent 170 and then through the one or more vent apertures 153 to atmosphere. In some configurations, a diffusing element is disposed between the vent 170 and the vent apertures 153. In some configurations, a diffusing element is disposed within the vent apertures 153.

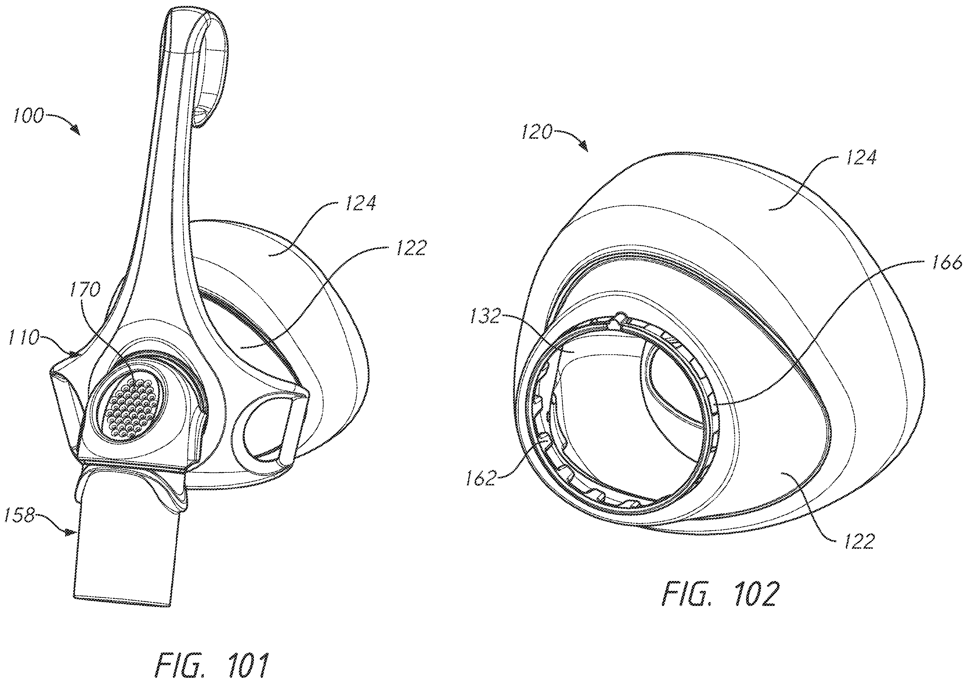

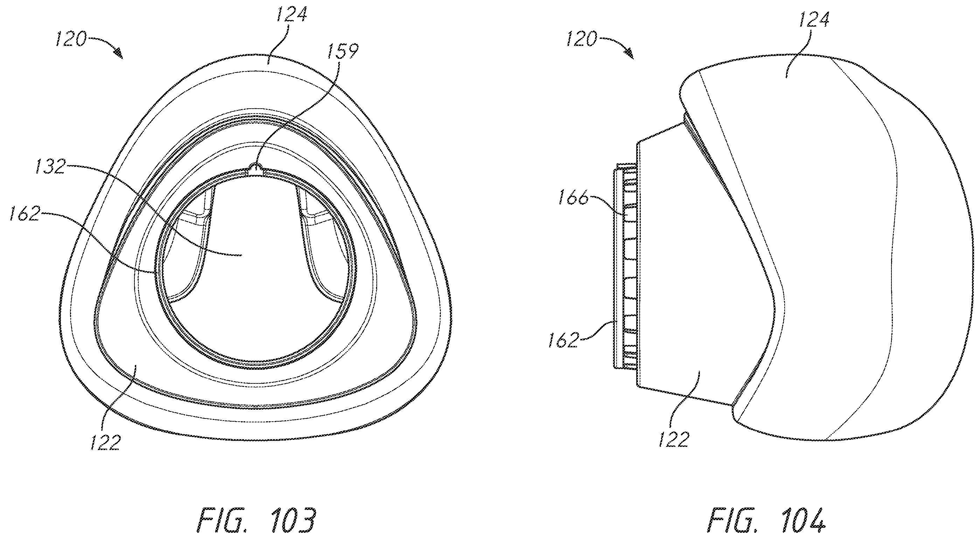

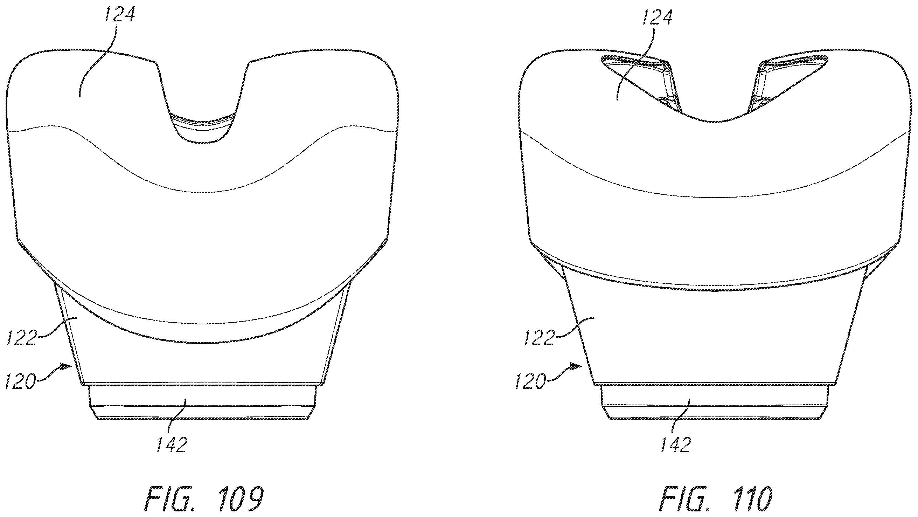

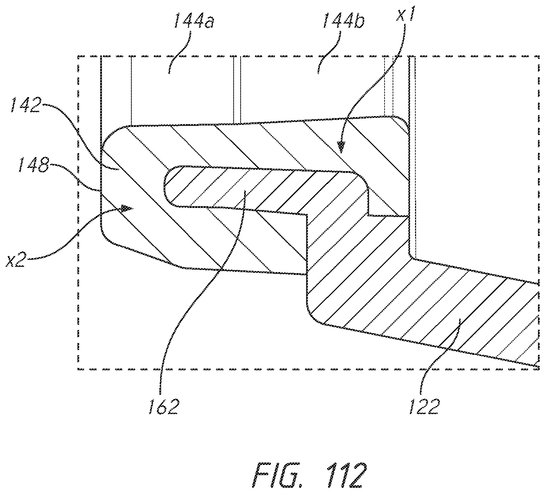

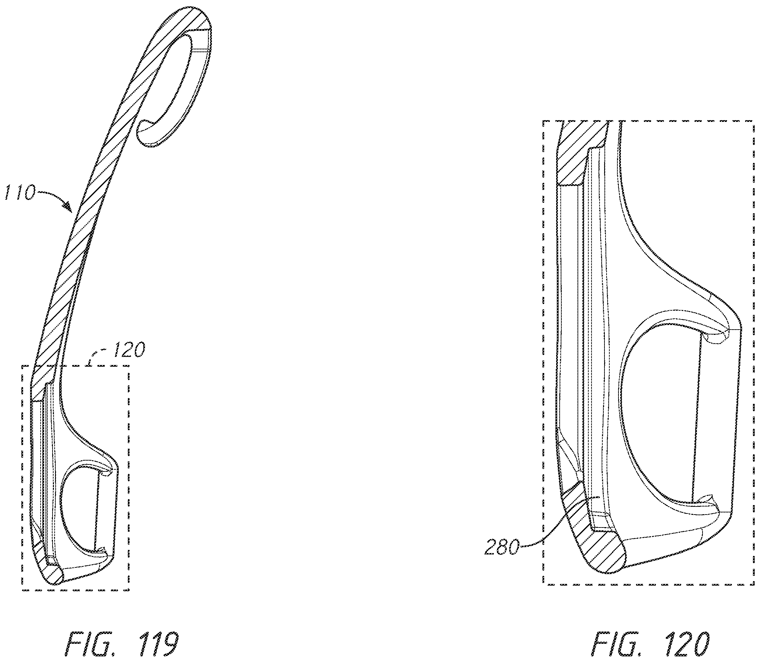

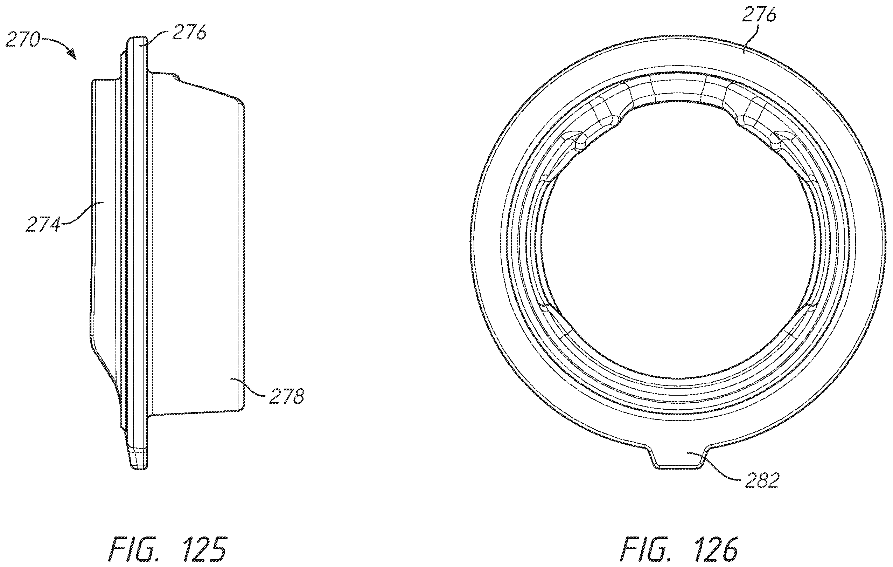

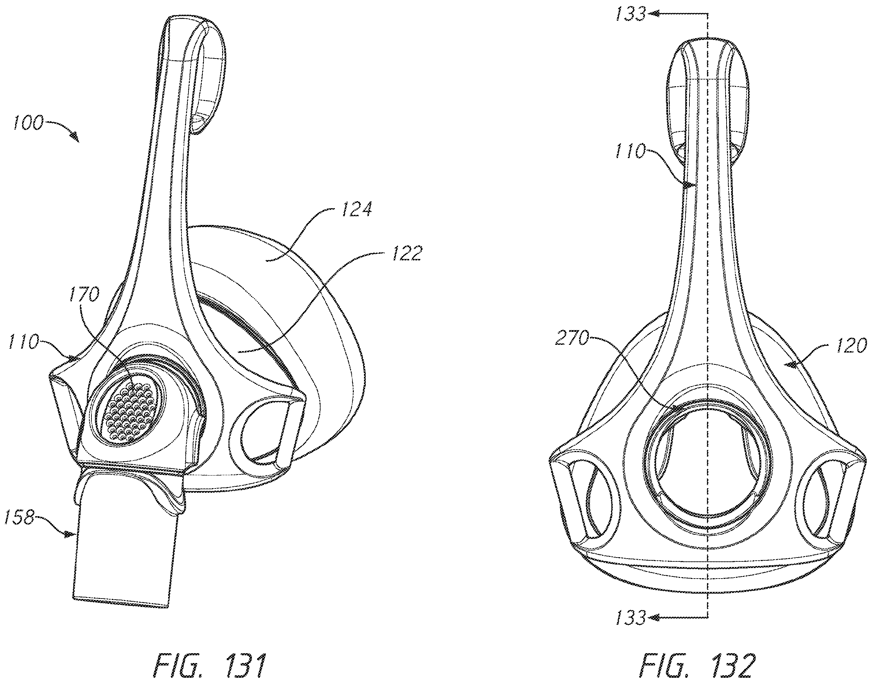

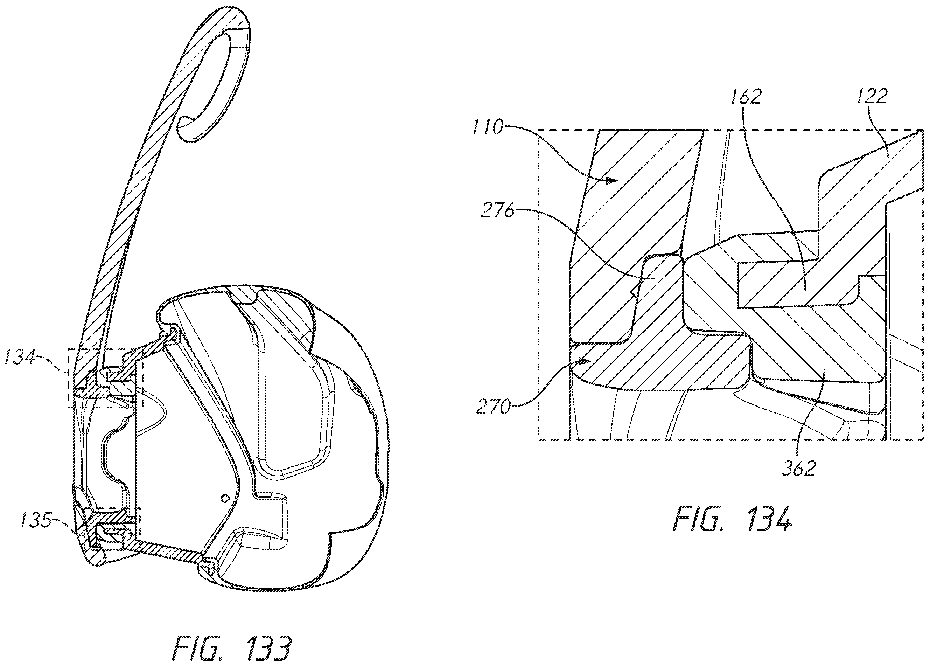

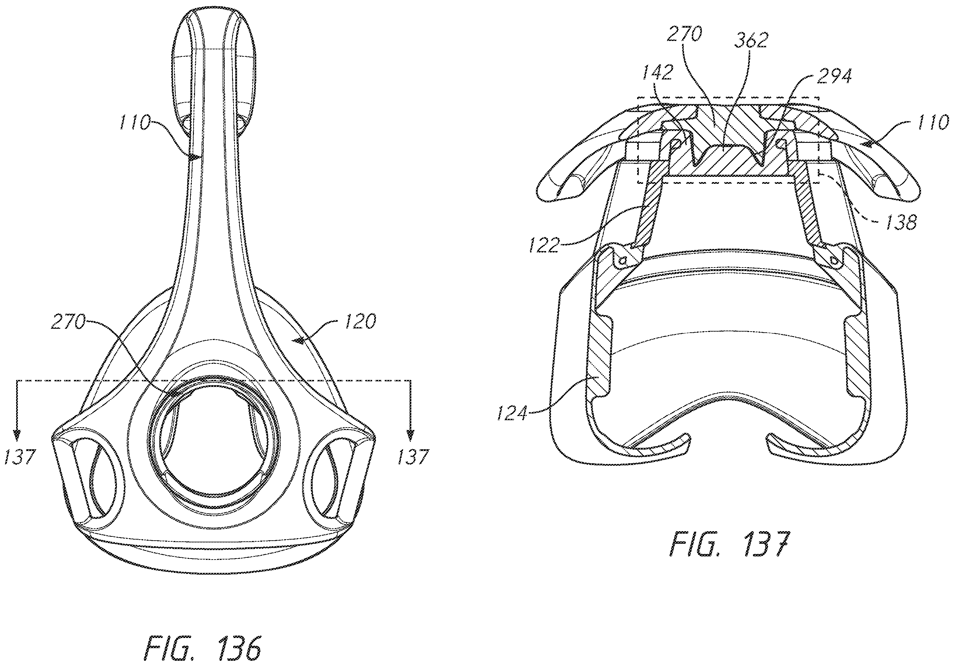

Figures 101-138 illustrate another patient interface 100 that is similar to the patient interface 100 of Figures 71-100 in some respects. Accordingly, the same reference numbers are used to refer to the same or similar features or components between the two interfaces 100. Any features or components of the patient interface 100 of Figures 101-138 that are not described in detail can be the same as or similar to the corresponding feature or component of the patient interface 100 of Figures 71-100, or can be of another suitable arrangement. The illustrated patient interface 100 includes a frame 110 and a cushion module 120 that includes a cushion or seal 124 coupled to a housing 122. The connection opening 132 can be generally circular to accommodate a generally circular portion (e.g., insert collar 278 as described herein) of the frame 110. A friction coupling 140 couples the cushion module 120 and the frame 110.

As described herein, the friction coupling 140 is or comprises an elastomeric friction member 142 coupled to, e.g., overmolded to, a support flange 162. The support flange 162 can be relatively more rigid than the elastomeric friction member 142. The elastomeric friction member 142 and support flange 162 together can be considered or called a cushion module flange 147. The cushion module flange 147 can define the connection opening 132. In the illustrated configuration, the elastomeric friction member 142 encloses the support flange 162. Therefore, the support flange 162 can be considered an internal flange. The support flange 162 can include a region 159 of increased thickness. In the illustrated embodiment, the region 159 of increased thickness is disposed at or along the top of the support flange 162. The region 159 of increased thickness can serve as a gate used during injection molding of the housing 122.

The housing 122 comprises one or more apertures 166 through which the material of the elastomeric friction member 142 extends as a result of the over-molding process. In the illustrated arrangement, the apertures 166 are located in the support flange 162. The apertures 166 can be rectangular or trapezoidal. At least a portion of the perimeter of the apertures 166 can be curved, which can help reduce stress concentration locations at the border(s) between the elastomeric friction member 142 and the support flange 162. The size of the apertures 166 can be selected based at least in part on the strength needed for the elastomeric friction member 142. In some configurations, each aperture 166 can have a major dimension of about 3mm. Each aperture 166 can have a minor dimension of about 1.8mm. The apertures 166 can be separated from each other by a distance of about 3mm along the perimeter of the support flange 162. In at least one configuration, an outer edge (defining the profile of the aperture on a radially outer surface of the support flange 162) of at least one of the apertures 166 can be rounded. In at least one configuration, an inner edge (defining the profile of the aperture on a radially inner surface of the support flange 162) of at least one of the apertures 166 can be rounded. In at least one configuration, both the outer edge and the inner edge of at least one of the apertures 166 can be rounded. Rounding the inner edge and/or the outer edge of one or more of the apertures 166 can help reduce stress concentrations in the elastomeric friction member 142 at or near the apertures 166, thereby increasing the life of the connection between the elastomeric friction member 142 and the housing 122.

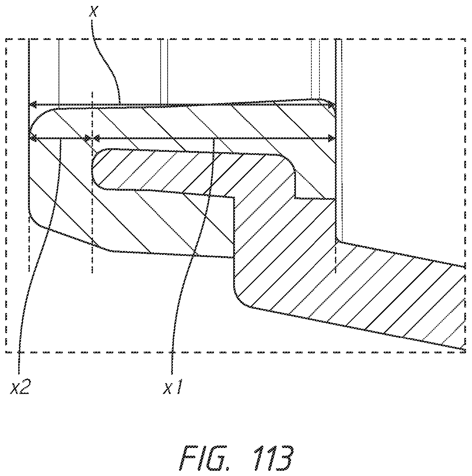

As previously described, a portion of the elastomeric friction member 142 that is radially inward and/or (with respect to the connection opening 132) from the support wall 163 and/or the support flange 162 defines a supported portion 312 of the elastomeric friction member 142. A portion of the elastomeric friction member 142 that does not extend radially outward and/or inward with respect to the support flange 162 and/or the support wall 163 defines an unsupported portion 310. In the illustrated configuration, a supported length x1 of the elastomeric friction member 142 is greater than an unsupported length x2 of the elastomeric friction member 142 (as shown in Figure 113) about the entire perimeter of the elastomeric friction member 142. The supported length x1 can correspond to the second length 182 as described with reference to Figure 3. The unsupported length x2 can correspond to the third length 184 described with reference to Figure 3. The supported length x1 of the elastomeric friction member 142 or cushion module flange 147 can be about 6.7 mm. The unsupported length x2 of the elastomeric friction member 142 or cushion module flange 147 can be about 1.8 mm. A total length x of the elastomeric friction member 142 or cushion module flange 147 can be about 8.5 mm. As shown, the unsupported length x2 can be less than the supported length x1. In some configurations, a ratio of the unsupported length x2 to the supported length x1 can be about 1:3.7. In use, for example, when the cushion module 120 is coupled or being coupled to the frame 110, the unsupported length x2 can elastically deform, compress, and/or flex outwardly, for example, like a cantilever, relatively more than the supported length x1.

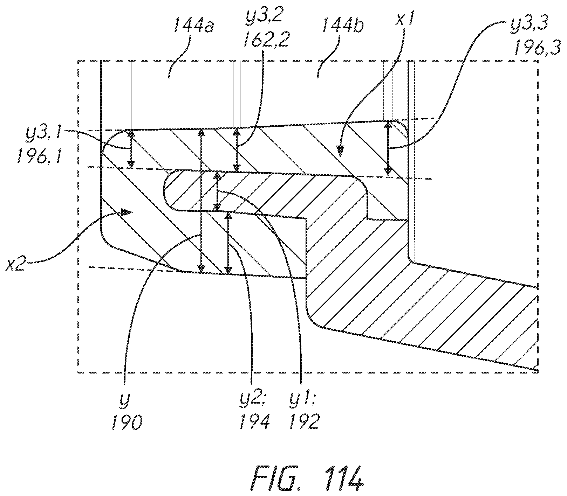

As shown in Figure 114, the elastomeric friction member 142 defines a first thickness 190 that extends in or substantially in a radial direction or perpendicular to the direction of assembly 104. The support flange 162 defines a second thickness 192 that extends in or substantially in a radial direction or perpendicular to the direction of assembly 104. A portion of the elastomeric friction member 142 located radially outside of the support flange 162 defines a third thickness 194. that extends in or substantially in a radial direction or perpendicular to the direction of assembly 104. A portion of the elastomeric friction member 142 located radially inside of the support flange 162 defines a fourth thickness 196 that extends in or substantially in a radial direction or perpendicular to the direction of assembly 104.

In the illustrated configuration, the first thickness 190 equals the sum of the second thickness 192, the third thickness 194, and the fourth thickness 196. As shown, the first thickness 190, or total thickness of the elastomeric friction member 142, can vary along its length. The second thickness 192, third thickness 194, and/or fourth thickness 196 can vary along their length(s). In the embodiment of Figure 114, the fourth thickness has a distal thickness 196, 1, a central thickness 196, 2, and a proximal thickness 196, 3. The first thickness 190 can be about 3.5 mm. The second thickness 192 can be about 1 mm. The third thickness 194 can be about 1.5 mm. The distal thickness 196, 1 can be about 0.9 mm. The central thickness 196, 2 can be about 1.1 mm. The proximal thickness 196, 3 can be about 1.4 mm. In some cases, the various thickness of the elastomeric friction member 142 can vary due to, for example, angled surfaces of the support flange 162 and/or expansion or shrinkage of the elastomeric friction member 142 during or after molding.

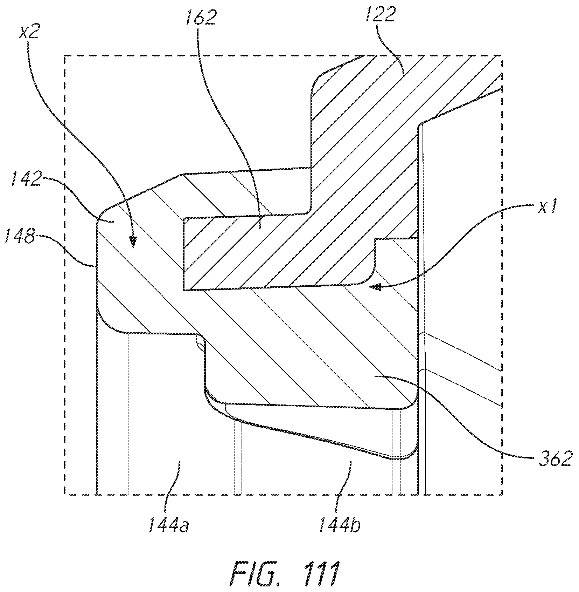

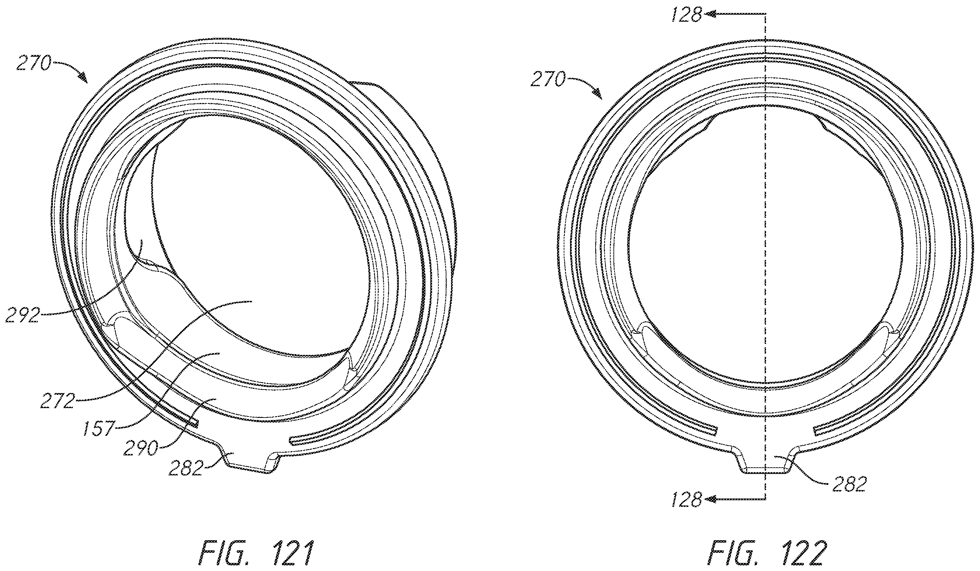

As shown in, e.g., Figures 121-138, the frame 110 can include a frame insert 270. In the illustrated configuration, the frame insert 270 includes an abutment collar 274, an abutment flange 276, and an insert collar 278. The abutment flange 276 has an enlarged dimension, e.g., diameter, circumference, or perimeter, compared to the abutment collar 274 and insert collar 278. The abutment collar 274 extends forward (or away from the user in use) from the abutment flange 276. The insert collar 278 extends rearward (or toward the user in use) from the abutment flange 276. The abutment flange 276 can include an aligning feature 282. In the illustrated configuration, the aligning feature 282 is a projection or portion or the abutment flange 276 protruding radially outward from the remainder of the abutment flange 276. In the illustrated configuration, the aligning feature 282 is located at or along a bottom of the abutment flange 276. The insert collar 278 can include an insert collar recess 360 recessed from a free rear edge of the insert collar 278. In the illustrated configuration, the insert collar recess 360 is disposed in or along a top of the insert collar 278. The frame insert 270 has an insert opening 272 defining a gas flow path through the frame insert 270. As described herein, the cushion module 120 can be coupled to the frame 110 by sliding the cushion module flange 147 onto the insert collar 278. When the cushion module 120 and frame 110 are fully coupled, a leading surface or abutment surface 148 (shown in Figures 111-112) of the elastomeric friction member 142 abuts a rear surface of the abutment flange 276 of the frame insert 170.

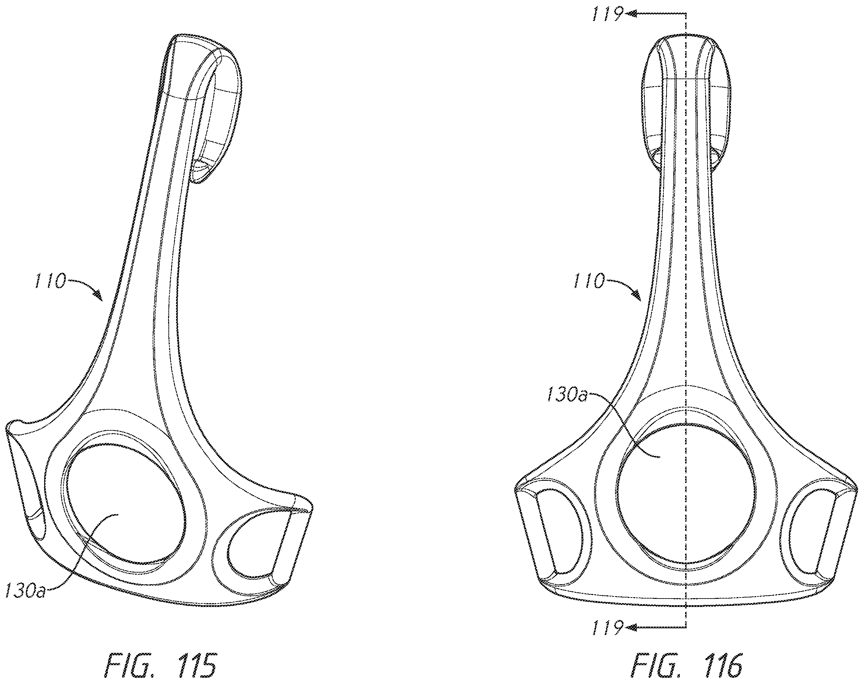

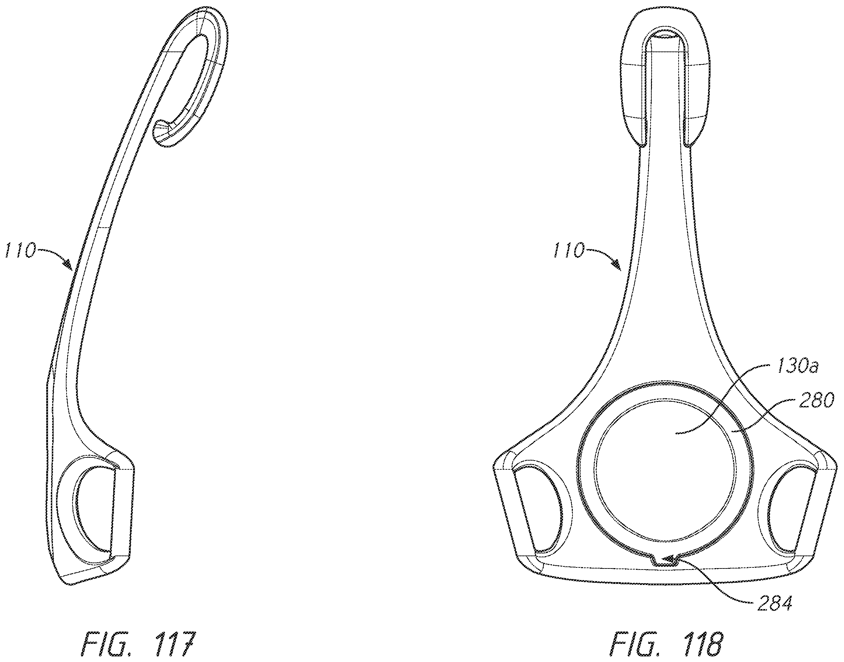

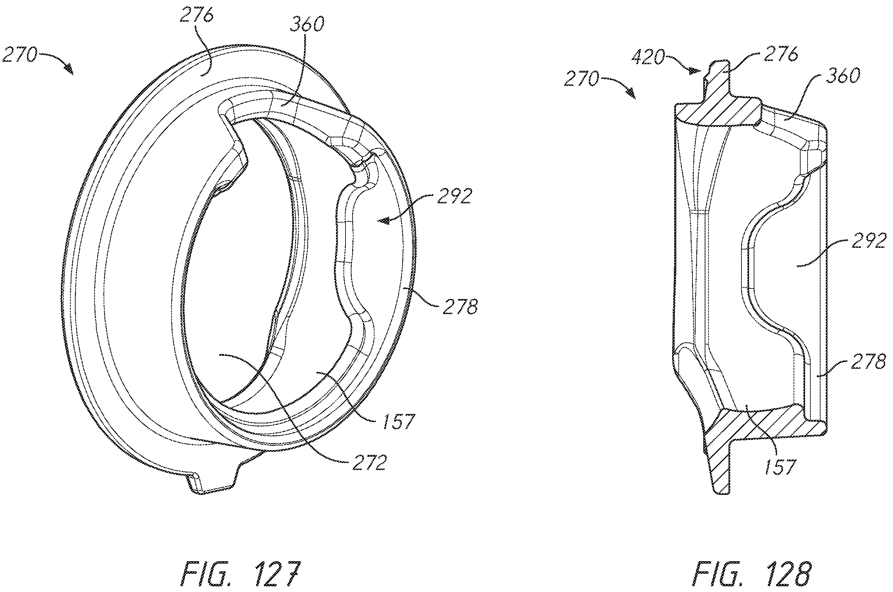

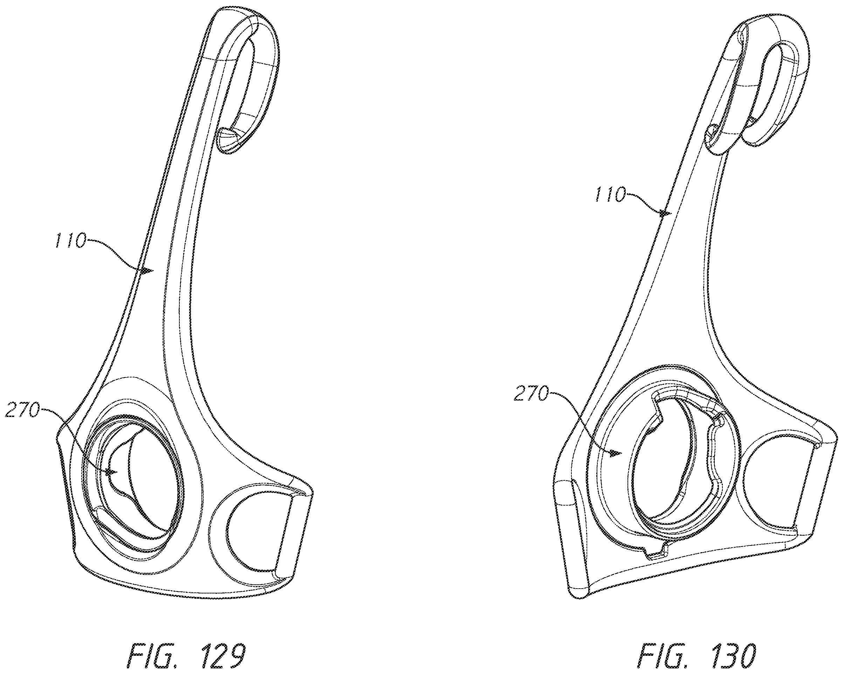

A main body of the frame 110, shown in Figures 115-120, includes a frame inlet opening 130a. that surrounds the gas inlet 130. When the frame insert 270 is assembled with the main body of the frame 110, the abutment collar 274 of the frame insert 270 extends into the frame inlet opening 130a, and at least a portion of the insert opening 272 defines the gas inlet 130, as shown in Figures 129-134. The main body of the frame 110 includes a rear peripheral recess or channel 280 in a rear or back surface of the frame 110 surrounding the perimeter of the frame inlet opening 130a. When the frame insert 270 is assembled with the main body of the frame 110, the abutment flange 276 of the frame insert 270 at least partially rests in the rear peripheral recess 280 of the main body of the frame 1 10. The rear peripheral recess 280 can include an extension 284 (shown in Figure 118) that receives the aligning feature 282 of the abutment flange 276 when the frame insert 270 is coupled to the main body of the frame 110. The aligning feature 282 and extension 284 can help properly align the frame insert 270 relative to the main body of the frame 110 during assembly. In the illustrated configuration, the extension 284 if positioned at or along a bottom of the rear peripheral recess 280 to accommodate an aligning feature 282 positioned at or along the bottom of the abutment flange 266, as illustrated in Figures 121-128. A front surface of the abutment flange 276 can contact a surface of the rear peripheral recess 280 when the frame insert 270 and main body of the frame 110 are coupled. The frame insert 270 can be secured to the main body of the frame 110, for example, via welding (e.g., ultrasonic welding), adhesives, and/or other means along the abutting surfaces of the frame insert 270 (e.g., the front surface of the abutment flange 276) and the main body of the frame 110 (e.g., the rear surface of the rear peripheral recess 280). An energy concentrator 420 can be used to concentrate energy during an ultrasonic welding process to ensure a complete weld between the abutment flange 276 and rear peripheral recess 280. As shown in Figure 128, the energy concentrator 420 can be located on the abutment flange 276. In the illustrated configuration, the energy concentrator 420 protrudes from a front surface of the abutment flange 276. The energy concentrator 420 can be generally triangular as shown.

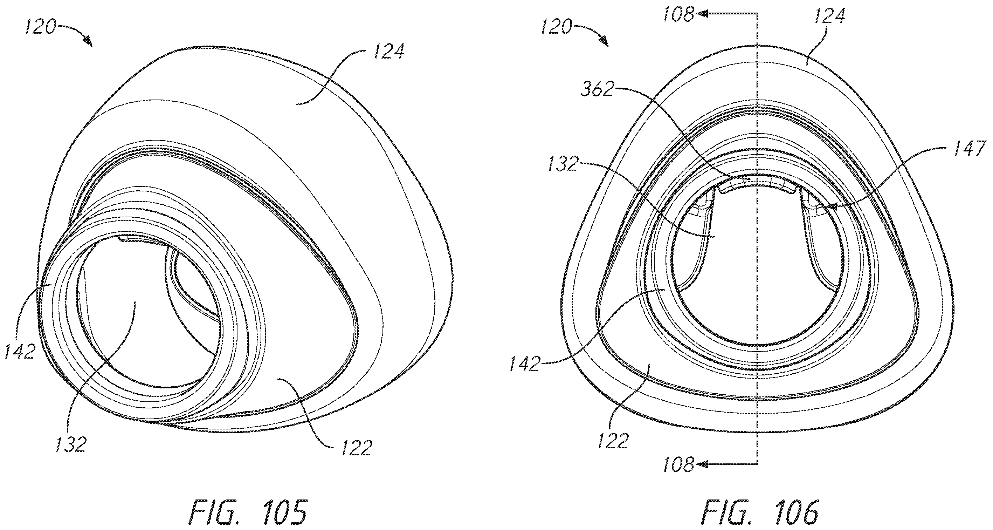

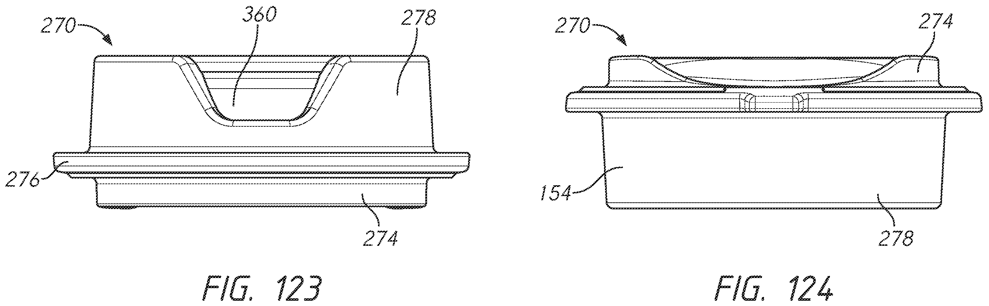

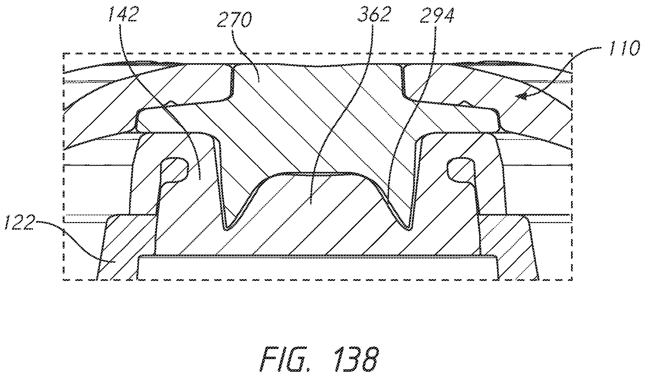

The elastomeric friction member 142 can include a protrusion or projection 362 (shown in, e.g., Figure 106) configured to be received by the insert collar recess 360 of the frame insert 270 (shown in, e.g., Figures 123 and 127), when the cushion module 120 is assembled to the frame 110. Cooperation between the protrusion 362 and recess 360 can advantageously inhibit relative motion (e.g., rotation) between the frame 110 and cushion module 120. The protrusion 362 can have tapered lateral edges such that a leading edge of the protrusion 362 as the cushion module 120 is coupled to the frame 110, e.g., the frame insert 270, is narrower than an opposite base of the protrusion 362. The tapered lateral edges can act as a lead in feature for the user when coupling the cushion module 120 to the frame 110 and/or can help the cushion module 120 and frame 110 self-align or correct slightly improper alignment during assembly. During assembly of the cushion module 120 and the frame 110, there is interference (indicated by region 294 in Figure 138) between the lateral edges of the protrusion 362 and lateral edges of the corresponding recess 360 of the frame insert 270. The interference can provide tactile feedback, for example, via a resistance force, to the user that the cushion module 120 and frame 110 are properly coupled.

In the illustrated arrangement, the elastomeric friction member 142 comprises an engagement surface 144 that engages an engagement surface 154 of the insert collar 278. A friction-fit between the collar 278, e.g., the engagement surface 154, and the elastomeric friction member 142 secures the frame 110 and cushion module 120 together. In at least one configuration, the engagement surface 144 is an interior or radially inward-facing surface of the elastomeric friction member 142. In at least one configuration, the engagement surface 154 is an exterior or radially outward-facing surface of the collar 278. However, in other arrangements, the elastomeric friction member 142 could engage an interior surface of the collar 278. Preferably, with either arrangement the frame 110 defines at least a portion of the breathing chamber 102. For example, in the illustrated configuration, an interior space defined by the collar 278 defines a portion of the breathing chamber 102.

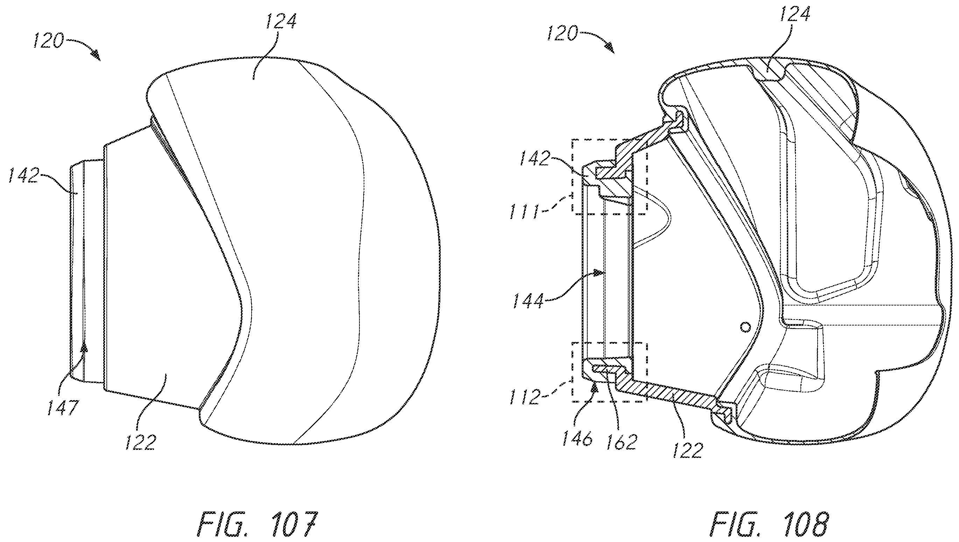

As described above, in the illustrated arrangement, the peripheral surface 146 (shown in, e.g., Figure 108) is exposed or is not directly covered when the cushion module 120 is assembled to the frame 110. With such an arrangement, the elastomeric friction member 142 is compressed from a single surface (e.g., the engagement surface 144). Accordingly, dimensional variations as a result of the manufacturing process will not impact the assembly and removal force to as great an extent as a design in which both the interior and exterior surfaces of the elastomeric friction member 142 engage a surface of a cooperating structure. Moreover, having the peripheral surface 146 of the elastomeric friction member 142 exposed to atmosphere advantageously allows the cushion module 120 and/or frame 110 to be easier to clean than an arrangement having a channel, which can be difficult to access by the user.

The engagement surface 144 can have a first engaging surface 144a. and a second engaging surface 144b, as shown in Figures 111-112. The second engaging surface 144b can extend at an angle relative to the first engaging surface 144a. The angle can be selected based at least in part on the grade of silicone used for the elastomeric friction member 142 and/or the forces desired to be required to connect and/or disconnect the frame 110 and cushion module 120.

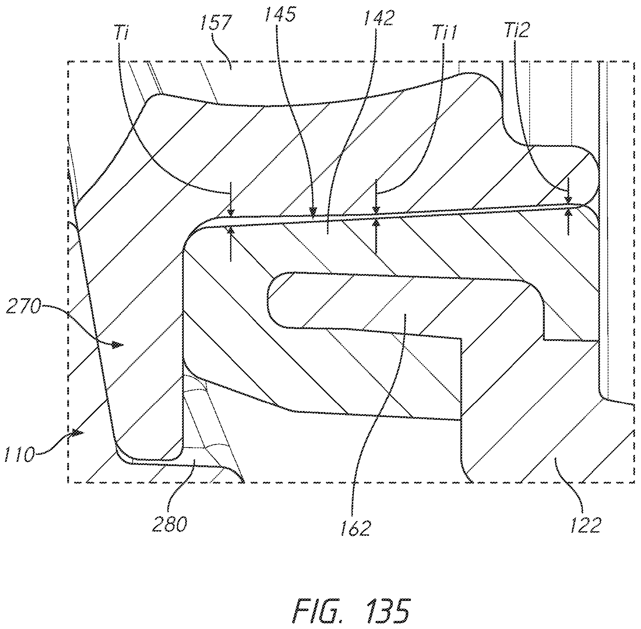

As the frame 110 and cushion module 120 are coupled, an interference region 145 (shown in Figure 135) of the elastomeric friction member 142 compresses and flexes to accommodate the inlet collar 152 and create the friction fit between the frame 110 and cushion module 120. As shown in Figure 135, the amount the elastomeric friction member 142 compresses can be considered a thickness Ti of the interference region 145 between the elastomeric friction member 142 and the inlet collar 152. A greater amount of compression or interference region thickness Ti results in a greater degree of or stronger friction or interference between the two components. The inlet collar 152 and cushion module flange 147 can be sized and/or otherwise designed so that the interference between the two is such that the force required to separate the frame 110 and cushion module 120 once connected is between about 10N and 50N during the intended lifespan of the patient interface 100.

The angle between the first 144a and second 144b engaging surfaces causes there to be a different interference between the first engaging surface 144a and the insert collar 152 compared to the interference between the second engaging surface 144b and the insert collar 152. In some configurations, the interference region thickness Ti decreases between the forward end of the cushion module flange 147 and rear end of the cushion module flange 147 toward the rear end of the cushion module flange 147. In the illustrated configuration, the interference region thickness Ti is greatest or at a maximum at or near the forward end of the cushion module flange 147 (e.g., where Ti is labeled in Figure 135) and decreases toward the base or rear end of the cushion module flange 147 to a small interference region thickness labeled Ti2 in Figure 135. In other words, Ti > Ti2. The interference between the frame cushion module flange 147 and inlet collar 152 is therefore biased (or greater) toward the front of the cushion module flange 147. Having a greater interference closer to the body and more vertical part of the frame 110 and farther away from the rear, free end of the inlet collar 152 can advantageously cause the inlet collar 152 to flex less and/or shifts more of the friction forces between the frame 110 and the cushion module 120 toward the base or forward end of the inlet collar 152, which can lessen the effects of rotation of a conduit connector coupled to the frame 110 as described herein. The variation in interference region thickness Ti can be at least partially the result of an angle of the inlet collar 152 relative to the cushion module flange 147 and/or the angle of the second engaging surface 144b relative to the first engaging surface 144a. In some configurations, the thickness of a central portion or midpoint of the cushion module flange 147 (e.g., where Ti1 is labeled in Figure 135), which can be located at or near a transition between the first engaging surface 144a and the second engaging surface 144b, can be equal to the thickness Ti2. In other words, the interference region can have a varying thickness in the region of the first engaging surface 144a and a constant thickness in the region of the second engaging surface 144b. In other configurations, central thickness Ti1 can be greater or less than Ti2.

In the illustrated arrangement, the frame insert 270 defines a socket 157 configured to receive a conduit connector, such as an elbow 158, configured to connect a breathing circuit to the patient interface 100. In the illustrated arrangement, the insert collar 278 includes and/or defines at least a portion of the elbow socket 157. The elbow socket 157 can include a cut-out 290, as shown in Figure 121, which can assist with inserting and/or removing the elbow 158. The cut-out 290 can be located along an inner surface of a bottom portion of the abutment collar 274. The elbow socket 157 can include one or more recesses 292. The elbow socket recesses 292 can be regions of reduced thickness of the insert collar 278. The elbow socket recesses 292 can allow the insert collar 278 to flex, for example, when being removed from a mold tool during manufacturing and/or during assembly with other components, while reducing or minimizing deformation of the socket 157. In some configurations, the conduit connector or elbow 158 is capable of pivoting or rotating about one or more axes relative to the frame 110. For example, the conduit connector or elbow 158 can have a swivel connection with the frame 110. The elbow 158 can be removably coupled to the frame 110. The vent 170 can be located in the elbow 158. In particular, the vent 170 can be located in a portion of an upper, outer, or patient-distal curve of the elbow 158, as shown in Figure 101.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to". Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment.

The term "plurality" refers to two or more of an item. Recitations of quantities, dimensions, sizes, formulations, parameters, shapes and other characteristics should be construed as if the term "about" or "approximately" precedes the quantity, dimension, size, formulation, parameter, shape or other characteristic. The terms "about" or "approximately" mean that quantities, dimensions, sizes, formulations, parameters, shapes and other characteristics need not be exact, but may be approximated and/or larger or smaller, as desired, reflecting acceptable tolerances, conversion factors, rounding off, measurement error and the like and other factors known to those of skill in the art. Recitations of quantities, dimensions, sizes, formulations, parameters, shapes and other characteristics should also be construed as if the term "substantially" precedes the quantity, dimension, size, formulation, parameter, shape or other characteristic. Unless otherwise defined, the term "substantially" means that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

Numerical data may be expressed or presented herein in a range format. It is to be understood that such a range format is used merely for convenience and brevity and thus should be interpreted flexibly to include not only the numerical values explicitly recited as the limits of the range, but also interpreted to include all of the individual numerical values or sub-ranges encompassed within that range as if each numerical value and sub-range is explicitly recited. As an illustration, a numerical range of "1 to 5" should be interpreted to include not only the explicitly recited values of about 1 to about 5, but should also be interpreted to also include individual values and sub-ranges within the indicated range. Thus, included in this numerical range are individual values such as 2, 3 and 4 and sub-ranges such as "1 to 3," "2 to 4" and "3 to 5," etc. This same principle applies to ranges reciting only one numerical value (e.g., "greater than 1") and should apply regardless of the breadth of the range or the characteristics being described.

A plurality of items may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. Furthermore, where the terms "and" and "or" are used in conjunction with a list of items, they are to be interpreted broadly, in that any one or more of the listed items may be used alone or in combination with other listed items. The term "alternatively" refers to selection of one of two or more alternatives, and is not intended to limit the selection to only those listed alternatives or to only one of the listed alternatives at a time, unless the context clearly indicates otherwise.

Reference to any prior art in this specification is not, and should not be taken as, an acknowledgement or any form of suggestion that that prior art forms part of the common general knowledge in the field of endeavour in any country in the world.

It should be noted that various changes and modifications to the presently preferred embodiments described herein will be apparent to those skilled in the art. Such changes and modifications may be made without departing from the scope of the invention and without diminishing its attendant advantages.

## Claims

1. A respiratory mask comprising a cushion module (120) and a frame (110), the cushion module (120) configured for attachment to the frame (110), the frame (110) having a front wall (150) and a collar (152) extending from the front wall (150), the cushion module (120) comprising:
a housing (122);
a cushion (124); and
an elastomeric friction member (142);
wherein the cushion (124) defines a face-contacting surface and the housing (122) defines a connection opening (132) and supports the elastomeric friction member (142), and
wherein the housing (122) comprises a wall portion, at least a portion of which is external to the elastomeric friction member (142) and extends between the elastomeric friction member (142) and the cushion (124),
wherein the elastomeric friction member (142) is configured to engage the collar (152) when the cushion module (120) is coupled to the frame (110);
wherein the housing (122) of the cushion module (120) further comprises a support flange (162), wherein the support flange (162) at least partially defines the connection opening (132) and extends within the elastomeric friction member (142) substantially in a direction of assembly of the cushion module (120) to the frame (110).

2. The respiratory mask of Claim 1, wherein the support flange (162) is embedded within the elastomeric friction member (142) to couple the elastomeric friction member (142) to the housing (122).

3. The respiratory mask of either Claim 1 or 2, wherein the support flange (162) comprises a plurality of apertures (166) and wherein the elastomeric friction member (142) extends through each of the plurality of apertures (166).

4. The respiratory mask of either Claim 1 or 2, wherein the housing (122) comprises a plurality of apertures (166) at a junction between the support flange (162) and an adjacent portion of the housing (122) and wherein the elastomeric friction member (142) extends through each of the apertures (166).

5. The respiratory mask of any one of Claims 1-2, wherein the wall portion of the housing (122) comprises a plurality of apertures (166) and wherein the elastomeric friction member (142) extends through each of the apertures (166).

6. The respiratory mask of any one of Claims 1-5, wherein the elastomeric friction member (142) defines an abutment surface (148) configured to contact the front wall (150) of the frame (110) when the cushion module (120) is coupled to the frame (110).

7. The respiratory mask of any one of Claims 1-6, wherein the elastomeric friction member (142) and the cushion (124) are constructed of the same material.

8. The respiratory mask of any one of Claims 1-6, wherein the elastomeric friction member (142) is more rigid than the cushion (124).

## Patentansprüche

1. Beatmungsmaske, umfassend ein Polsterungsmodul (120) und einen Rahmen (110), wobei das Polsterungsmodul (120) zur Anbringung an dem Rahmen (110) gestaltet ist, wobei der Rahmen (110) eine Vorderwand (150) und eine Manschette (152) aufweist, die sich von der Vorderwand (150) aus erstreckt, wobei das Polsterungsmodul (120) Folgendes umfasst:
ein Gehäuse (122),
ein Polster (124) und
ein elastomeres Reibungselement (142),
wobei das Polster (124) eine gesichtskontaktierende Oberfläche definiert und das Gehäuse (122) eine Verbindungsöffnung (132) definiert und das elastomere Reibungselement (142) trägt, und
wobei das Gehäuse (122) einen Wandabschnitt umfasst, von dem mindestens ein Abschnitt außerhalb des elastomeren Reibungselements (142) liegt und sich zwischen dem elastomeren Reibungselement (142) und dem Polster (124) erstreckt,
wobei das elastomere Reibungselement (142) dafür gestaltet ist, mit der Manschette (152) in Eingriff zu stehen, wenn das Polsterungsmodul (120) mit dem Rahmen (110) gekoppelt ist,
wobei das Gehäuse (122) des Polsterungsmoduls (120) ferner einen Trägerflansch (162) umfasst, wobei der Trägerflansch (162) zumindest teilweise die Verbindungsöffnung (132) definiert und sich in dem elastomeren Reibungselement (142) im Wesentlichen in eine Richtung des Zusammenfügens des Polsterungsmoduls (120) mit dem Rahmen (110) erstreckt.

2. Beatmungsmaske nach Anspruch 1, wobei der Trägerflansch (162) in das elastomere Reibungselement (142) eingebettet ist, um das elastomere Reibungselement (142) mit dem Gehäuse (122) zu koppeln.

3. Beatmungsmaske nach entweder Anspruch 1 oder 2, wobei der Trägerflansch (162) mehrere Öffnungen (166) umfasst und wobei sich das elastomere Reibungselement (142) durch jede der mehreren Öffnungen (166) erstreckt.

4. Beatmungsmaske nach entweder Anspruch 1 oder 2, wobei das Gehäuse (122) mehrere Öffnungen (166) an einem Kreuzungspunkt zwischen dem Trägerflansch (162) und einem benachbarten Abschnitt des Gehäuses (122) umfasst und wobei sich das elastomere Reibungselement (142) durch jede der Öffnungen (166) erstreckt.

5. Beatmungsmaske nach einem der Ansprüche 1 bis 2, wobei der Wandabschnitt des Gehäuses (122) mehrere Öffnungen (166) umfasst und wobei sich das elastomere Reibungselement (142) durch jede der Öffnungen (166) erstreckt.

6. Beatmungsmaske nach einem der Ansprüche 1 bis 5, wobei das elastomere Reibungselement (142) eine Auflagefläche (148) definiert, die dafür gestaltet ist, mit der Vorderwand (150) des Rahmens (110) in Kontakt zu stehen, wenn das Polsterungsmodul (120) mit dem Rahmen (110) gekoppelt ist.

7. Beatmungsmaske nach einem der Ansprüche 1 bis 6, wobei das elastomere Reibungselement (142) und das Polster (124) aus dem gleichen Material gefertigt sind.

8. Beatmungsmaske nach einem der Ansprüche 1 bis 6, wobei das elastomere Reibungselement (142) starrer als das Polster (124) ist.

## Revendications

1. Masque respiratoire comprenant un module de coussin (120) et un cadre (110), le module de coussin (120) étant configuré pour être attaché au cadre (110), le cadre (110) ayant une paroi avant (150) et un collier (152) s'étendant depuis la paroi avant (150), le module de coussin (120) comprenant :
un logement (122) ;
un coussin (124) ; et
un élément de frottement élastomérique (142) ;
dans lequel le coussin (124) définit une surface de contact avec le visage et le logement (122) définit une ouverture de connexion (132) et supporte l'élément de frottement élastomérique (142), et
dans lequel le logement (122) comprend une partie de paroi, dont au moins une partie est extérieure à l'élément de frottement élastomérique (142) et s'étend entre l'élément de frottement élastomérique (142) et le coussin (124),
dans lequel l'élément de frottement élastomérique (142) est configuré pour entrer en prise avec le collier (152) lorsque le module de coussin (120) est couplé au cadre (110) ;
dans lequel le logement (122) du module de coussin (120) comprend en outre une bride de support (162), dans lequel la bride de support (162) définit au moins partiellement l'ouverture de connexion (132) et s'étend au sein de l'élément de frottement élastomérique (142) sensiblement dans une direction d'assemblage du module de coussin (120) avec le cadre (110).

2. Masque respiratoire selon la revendication 1, dans lequel la bride de support (162) est encastrée au sein de l'élément de frottement élastomérique (142) pour coupler l'élément de frottement élastomérique (142) au logement (122).

3. Masque respiratoire selon la revendication 1 ou 2, dans lequel la bride de support (162) comprend une pluralité d'ouvertures (166) et dans lequel l'élément de frottement élastomérique (142) s'étend à travers chacune de la pluralité d'ouvertures (166).

4. Masque respiratoire selon la revendication 1 ou 2, dans lequel le logement (122) comprend une pluralité d'ouvertures (166) au niveau d'une jonction entre la bride de support (162) et une partie adjacente du logement (122) et dans lequel l'élément de frottement élastomérique (142) s'étend à travers chacune des ouvertures (166).

5. Masque respiratoire selon l'une quelconque des revendications 1 à 2, dans lequel la partie de paroi du logement (122) comprend une pluralité d'ouvertures (166) et dans lequel l'élément de frottement élastomérique (142) s'étend à travers chacune des ouvertures (166).

6. Masque respiratoire selon l'une quelconque des revendications 1 à 5, dans lequel l'élément de frottement élastomérique (142) définit une surface de butée (148) configurée pour entrer en contact avec la paroi avant (150) du cadre (110) lorsque le module de coussin (120) est couplé au cadre (110).

7. Masque respiratoire selon l'une quelconque des revendications 1 à 6, dans lequel l'élément de frottement élastomérique (142) et le coussin (124) sont fabriqués dans le même matériau.

8. Masque respiratoire selon l'une quelconque des revendications 1 à 6, dans lequel l'élément de frottement élastomérique (142) est plus rigide que le coussin (124).
